# EUROPEAN PATENT SPECIFICATION

(11) **EP 3 065 767 B1**
(45) Date of publication and mention of the grant of the patent: **30.12.2020**
(21) Application number: 14802336.9
(22) Date of filing: 06.11.2014
(51) Int. Cl.: A61K 38/26

(54) **GIP-GLP-1 DUAL AGONIST COMPOUNDS AND METHODS**
ZWEIFACH-GIP-GLP-1-AGONISTENVERBINDUNG UND VERFAHREN
COMPOSÉS AGONISTES DOUBLES GIP-GLP-1 ET PROCÉDÉS

(30) Priority: 06.11.2013 EP 13191843; 14.07.2014 EP 14176878
(43) Date of publication of application: 14.09.2016
(73) Proprietor: Zealand Pharma A/S, 2860 Søborg (DK)
(72) Inventor: SHELTON, Anne Pernille Tofteng, DK-2600 Glostrup (DK); NØRREGAARD, Pia, DK-2600 Glostrup (DK); FOG, Jacob Ulrik, DK-2600 Glostrup (DK); KNUDSEN, Carsten Boye, DK-2600 Glostrup (DK)
(74) Representative: Mewburn Ellis LLP
(86) International application number: PCT/EP2014/073970
(87) International publication number: WO 2015/067715

(56) References cited:
- WO-A1-2009/030738
- WO-A1-2010/070251
- WO-A1-2011/006497
- WO-A1-2011/088837
- WO-A1-2011/119657
- WO-A1-2013/164483
- WO-A1-2014/041195
- WO-A2-2010/011439
- KJELD MADSEN ET AL: "Structure-Activity and Protraction Relationship of Long-Acting Glucagon-like Peptide-1 Derivatives: Importance of Fatty Acid Length, Polarity, and Bulkiness", JOURNAL OF MEDICINAL CHEMISTRY, vol. 50, no. 24, 1 November 2007 (2007-11-01), pages 6126-6132, XP055131268, ISSN: 0022-2623, DOI: 10.1021/jm070861j

## Description

### Field of the Invention

The invention relates to compounds having agonist activity at both the GIP and GLP-receptors, and to their use in the treatment of metabolic disorders.

### Background of the Invention

Diabetes and obesity are increasing health problems globally and are associated with various other diseases, particularly cardiovascular diseases (CVD), obstructive sleep apnea, stroke, peripheral artery disease, microvascular complications and osteoarthritis. There are 246 million people worldwide with diabetes, and by 2025 it is estimated that 380 million will have diabetes. Many have additional cardiovascular risk factors including high/aberrant LDL and triglycerides and low HDL. Cardiovascular diseases account for about 50% of the mortality in people with diabetes, and the morbidity and mortality rates relating to obesity and diabetes underscore the medical need for efficacious treatment options.

Incretins are gastrointestinal hormones that regulate blood glucose by enhancing glucose-stimulated insulin secretion (Drucker, DJ and Nauck, MA, Lancet 368: 1696-705 (2006)). To date there are two known incretins: glucagon-like peptide-1 (GLP-1), and glucose-dependent insulinotropic polypeptide (GIP). The incretin GLP-1 is derived from the pre-proglucagon gene. Pre-proglucagon is a 158-amino acid precursor polypeptide that is processed in different tissues to form a number of different proglucagon-derived peptides, including glucagon, GLP-1, glucagon-like peptide-2 (GLP-2) and oxyntomodulin (OXM). Glucagon is a 29-amino acid peptide that corresponds to amino acids 33 through 61 of pre-proglucagon, while GLP-1 is produced as a 37-amino acid peptide that corresponds to amino acids 72 through 108 of pre-proglucagon. GIP is a 42-amino acid peptide derived by proteolytic processing from a 133-amino acid precursor, pre-pro-GIP. All the peptides are involved in a wide variety of physiological functions, including glucose homeostasis, insulin secretion, gastric emptying, and intestinal growth, as well as the regulation of food intake.

The discovery of the incretins has led to the development of two new classes of drugs for the treatment of diabetes mellitus. Thus, injectable GLP-1 receptor agonists, and small molecule compounds (oral DPP-4 inhibitors) that inhibit enzymatic inactivation of both endogenous GLP-1 and GIP, are now on the market (GLP-1 receptor agonists: Byetta™, Bydureon™ Lixisenatide™ and Victoza™; and DPP-4 inhibitors: Januvia™, Galvus™, Onglyza™ and Trajenta™). Apart from the acute effects of GLP-1 and GIP on insulin secretion, the incretins have some long-term effects. Evidence from several laboratories shows that GLP-1 receptor agonists protect pancreatic β-cehs by inhibiting apoptosis and enhancing proliferation. For instance, a study by Farilla *et al.* showed that GLP-1 has anti-apoptotic effects in human islets (Farilla, L, Endocrinology 144: 5149-58 (2003)). Such effects have not been reported for GIP until recently. Weidenmaier *et al.* reported that a DPP-4 resistant GIP analogue had anti-apoptotic effects (Weidenmaier, SD, PLOS One 5(3): e9590 (2010)). Interestingly, in a mouse model of diabetes and obesity, the combination of the GLP-1 receptor agonist Liraglutide and an acylated GIP analogue showed superior glucose-lowering and insulinotropic effects compared to treatment with Liraglutide and GIP analogue alone (Gault, VA, Clinical Science 121: 107-117 (2011)).

Chronic treatment with GLP-1 receptor agonists causes significant weight loss in diabetic humans. Interestingly, extended use of DPP-4 inhibitors in similar patients does not consistently change body weight. Evidence suggests (Matthias Tschöp oral presentation at ADA (American Diabetes Association), 2011) that body weight loss associated with GLP-1 agonist treatment is enhanced when GLP-1 and GIP are co-administered. In rodents, coadministration of GLP-1 and GIP results in greater body weight loss than GLP-1 treatment alone (Finan, Sci Transl Med. 2013; 5(209):209ra151. Irwin N et al, 2009, Regul Pept; 153: 70-76. Gault et al, 2011, Clin Sci; 121:107-117).. Thus, in addition to improving blood glucose control, GIP may also enhance GLP-1-mediated body weight loss.

Recently, different peptides have been shown to bind and activate both the GIP and the GLP-1 receptor and to suppress body weight gain and reduce food intake (see, for example WO2012/088116, WO2010/148089, WO2012/167744, WO 2013/164483, WO 2014/096145, WO 2014/096150 and WO 2014/096149). However, most of these peptides have short terminal elimination half-life (T½).

WO 2011/119657 describes compounds with activity at the GIP and GLP-1 receptors, based on the full length GIP sequence.

### Summary of the Invention

Broadly, the present invention concerns acylated and truncated GIP analogues which comprise one or more substitutions as compared to wild-type GIP and which may have the property of an altered, preferably increased GLP-1 activity, e.g., as assessed in *in vitro* efficacy assays and an altered, preferably increased terminal elimination half-life (T½), as assessed in *in vivo* studies in mice.

It has been found in animals that GIP-GLP1 dual acting receptor agonists are superior to existing and marketed GLP-1 analogues because the dual agonists offer improved glycemic control, and enhanced body weight loss. The GIP-GLP1 dual agonists disclosed herein (also referred to as GIP analogues) may thus be used as therapeutics for metabolic disorders including, but not limited to, type 2 diabetes mellitus, obesity and related disorders.

The invention provides a GIP analogue having the general Formula I: 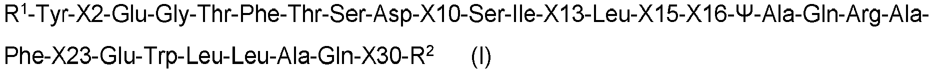 wherein
R¹ is H, C₁₋₄ alkyl, acetyl, formyl, benzoyl, trifluoroacetyl or pGlu;
X2 is selected from Aib and D-Ala;
X10 is selected from Tyr and Leu;
X13 is selected from Ala, Tyr and Aib;
X15 is selected from Asp and Glu;
X16 is selected from Glu and Lys;
X23 is selected from Val and Ile;
X30 is selected from Lys, Gly and Y1, or is absent;
and positions 1 to 29 of the GIP analogue have a maximum of 4 changes compared to the sequence Y-Aib-EGTFTSDYSIYLDKKAQRAFVEWLLAQ;
Y1 (when present) is selected from Gly-Pro-Ser-Ser-Gly-Ala-Pro-Pro-Pro-Ser, Lys-Pro-Ser-Ser-Gly-Ala-Pro-Pro-Pro-Ser, Gly-Pro-Ser-Ser-Gly-Ala-Pro-Pro-Ser, Pro-Ser-Ser-Gly-Ala-Pro-Pro-Pro-Ser and Pro-Ser-Ser-Gly-Ala-Pro-Pro-Ser;
Ψ is a residue of Lys, Arg, Orn or Cys in which the side chain is conjugated to a substituent having the formula or -Z²-Z¹; where:
-Z¹ is a fatty chain having a polar group at one end of the chain and a connection to Ψ or Z², -X- at the end of the chain distal from the polar group,
wherein the polar group comprises a carboxylic acid or a carboxylic acid bioisostere, a phosphonic acid, or a sulfonic acid group;
and -X- is a bond, -CO-, -SO-, or -SO₂-;
-Z²- (if present) is a spacer of formula: 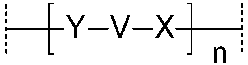
connecting Z¹ to Ψ;
wherein:
each Y is independently -NH, -NR, -S or -O, where R is alkyl, a protecting group or forms a linkage to another part of the spacer Z²;
each X is independently a bond, CO-, SO-, or SO₂-;
with the proviso that when Y is -S, the X to which it is bound is a bond;
each V is independently a bivalent organic moiety linking Y and X;
and n is 1-10;

and
R² is -NH₂ or -OH;
or a pharmaceutically acceptable salt or solvate thereof;
wherein said GIP analogue has agonist activity at the GIP and GLP-1 receptors.

For the avoidance of doubt, those positions which are not expressly stated to permit variability are fixed and may only include the stated residue.

In some embodiments, the GIP analogue further comprises the residue Glu15.

Combinations of residues which may be present at some of the variable positions from 1 to 29 include:
Aib2, Tyr10, Tyr13, Asp15, Lys16, Val23;
D-Ala2, Tyr10, Tyr13, Asp15, Lys16, Val23;
Aib2, Tyr10, Aib13, Asp15, Lys16, Val23;
Aib2, Tyr10, Ala13, Glu15, Lys16, Val23;
Aib2, Leu10, Ala13, Glu15, Lys16, Val23;
Aib2, Tyr10, Tyr13, Glu15, Lys16, Val23;
D-Ala2, Tyr10, Ala13, Asp15, Lys16, Val23,;
Aib2, Tyr10, Ala13, Asp15, Lys16, Val23;
Aib2, Tyr10, Tyr13, Asp15, Lys16, Ile23;
D-Ala2, Tyr10, Tyr13, Asp15, Lys16, Ile23;
Aib2, Tyr10, Aib13, Asp15, Lys16, Ile23;
Aib2, Tyr10, Ala13, Glu15, Lys16, Ile23; and
Aib2, Leu10, Ala13, Glu15, Lys16, Ile23.

Residues 1-29 of Formula I may have the sequence:
Y-Aib-EGTFTSDYSIYLDKΨAQRAFVEWLLAQ;
Y-DAIa-EGTFTSDYSIYLDKΨAQRAFVEWLLAQ;
Y-Aib-EGTFTSDYSI-Aib-LDKΨAQRAFVEWLLAQ;
Y-Aib-EGTFTSDYSIALEKΨAQRAFVEWLLAQ;
Y-Aib-EGTFTSDLSIALEKΨAQRAFVEWLLAQ;
Y-Aib-EGTFTSDYSIALEKΨAQRAFVEWLLAQ;
Y-DAIa-EGTFTSDYSIALEKΨAQRAFVEWLLAQ;
Y-Aib-EGTFTSDLSIALEKΨAQRAFVEWLLAQ;
Y-Aib-EGTFTSDYSIALDKΨAQRAFVEWLLAQ;
Y-DAla-EGTFTSDYSIALDKΨAQRAFVEWLLAQ;
Y-Aib-EGTFTSDYSIALDEΨAQRAFVEWLLAQ;
Y-Aib-EGTFTSDYSIALDKΨAQRAFIEWLLAQ-; or
Y-Aib-EGTFTSDYSIALDKΨAQRAFVEWLLAQ.

Residues 1-29 of Formula I may have the sequence:
Y-Aib-EGTFTSDYSIYLDK-K([17-carboxy-heptadecanoyl]-isoGlu-Peg3-Peg3)-AQRAFVEWLLAQ;
Y-Aib-EGTFTSDYSIYLDK-K([19-Carboxy-nonadecanoyl]-isoGlu-Peg3-Peg3)-AQRAFVEWLLAQ;
Y-Aib-EGTFTSDYSIYLDK-K([17-carboxy-heptadecanoyl]-isoGlu)-AQRAFVEWLLAQ;
H-Y-Aib-EGTFTSDYSIYLDK-K([17-carboxy-heptadecanoyl]-isoGlu-GSGSGG)-AQRAFVEWLLAQ;
Y-Aib-EGTFTSDYSIYLDK-K([17-carboxy-heptadecanoyl]-Peg3-Peg3)-AQRAFVEWLLAQ;
Y-Aib-EGTFTSDYSIYLDK-K([17-carboxy-heptadecanoyl]-isoGlu-Peg3-Peg3-Peg3)-AQRAFVEWLLAQ;
Y-Aib-EGTFTSDYSIYLDK-K([17-carboxy-heptadecanoyl]-Dapa-Peg3-Peg3)-AQRAFVEWLLAQ;
Y-Aib-EGTFTSDYSIYLDK-K([19-Carboxy-nonadecanoyl]-isoGlu-Peg3-Peg3-Peg3)-AQRAFVEWLLAQ;
Y-DAIa-EGTFTSDYSIYLDK-K([17-carboxy-heptadecanoyl]-isoGlu-Peg3-Peg3)-AQRAFVEWLLAQ;
Y-Aib-EGTFTSDYSI-Aib-LDK-K([17-carboxy-heptadecanoyl]-isoGlu-Peg3-Peg3)-AQRAFVEWLLAQ;
Y-Aib-EGTFTSDYSIALEK-K([17-carboxy-heptadecanoyl]-isoGlu-Peg3-Peg3)-AQRAFVEWLLAQ;
Y-Aib-EGTFTSDLSIALEK-K([17-carboxy-heptadecanoyl]-isoGlu-Peg3-Peg3)-AQRAFVEWLLAQ;
Y-Aib-EGTFTSDYSIALEK-K[19-carboxy-nonadecanoyl]-isoGlu-Peg3-Peg3)-AQRAFVEWLLAQ;
Y-Aib-EGTFTSDLSIALEK-K([19-carboxy-nonadecanoyl]-isoGlu-Peg3-Peg3)-AQRAFVEWLLAQ;
Y-DAIa-EGTFTSDYSIYLDK-K([19-carboxy-nonadecanoyl]-isoGlu-Peg3-Peg3)-AQRAFVEWLLAQ;
Y-Aib-EGTFTSDYSIALEK-K([19-carboxy-nonadecanoyl]-isoGlu-Peg3-Peg3)-AQRAFVEWLLAQ;
Y-DAIa-EGTFTSDYSIALEK-K([19-carboxy-nonadecanoyl]-isoGlu-Peg3-Peg3)-AQRAFVEWLLAQ;
Y-Aib-EGTFTSDLSIALEK-K([19-carboxy-nonadecanoyl]-isoGlu-Peg3-Peg3)-AQRAFVEWLLAQ;
Y-Aib-EGTFTSDYSIYLDK-K([19-carboxy-nonadecanoyl]-Peg3-Peg3)-AQRAFVEWLLAQ;
Y-Aib-EGTFTSDYSIYLDK-K([19-carboxy-nonadecanoyl]-lsoGlu)-AQRAFVEWLLAQ;
Y-Aib-EGTFTSDYSIYLDK-K([19-carboxy-nonadecanoyl]-Dapa-Peg3-Peg3)-AQRAFVEWLLAQ;
Y-Aib-EGTFTSDYSIALDK-K([19-carboxy-nonadecanoyl]-isoGlu-Peg3-Peg3)-AQRAFVEWLLAQ;
Y-DAIa-EGTFTSDYSIALDK-K([19-carboxy-nonadecanoyl]-isoGlu-Peg3-Peg3)-AQRAFVEWLLAQ;
Y-Aib-EGTFTSDYSIALDE-K([19-carboxy-nonadecanoyl]-isoGlu-Peg3-Peg3)-AQRAFVEWLLAQ;
Y-Aib-EGTFTSDYSIALDK-K([19-carboxy-nonadecanoyl]-isoGlu-Peg3-Peg3)-AQRAFIEWLLAQ;
Y-Aib-EGTFTSDYSIALDK-K([17-carboxy-heptadecanoyl]-isoGlu-Peg3-Peg3)-AQRAFVEWLLAQ; or
Y-Aib-EGTFTSDYSIALDK-K((19-Carboxy-nonadecanoyl)-[(Piperazine-1-yl)-acetyl]-Peg3-Peg3)-AQRAFVEWLLAQ.

The peptide backbone of Formula I may have the sequence:
Y-Aib-EGTFTSDYSIYLDKΨAQRAFVEWLLAQGPSSGAPPPS;
Y-DAIa-EGTFTSDYSIYLDKΨAQRAFVEWLLAQGPSSGAPPPS;
Y-Aib-EGTFTSDYSI-Aib-LDKΨAQRAFVEWLLAQGPSSGAPPPS;
Y-Aib-EGTFTSDYSIALEKΨAQRAFVEWLLAQ K;
Y-Aib-EGTFTSDLSIALEKΨAQRAFVEWLLAQK;
Y-Aib-EGTFTSDYSIALEKΨAQRAFVEWLLAQGPSSGAPPPS;
Y-DAIa-EGTFTSDYSIALEKΨAQRAFVEWLLAQGPSSGAPPPS;
Y-Aib-EGTFTSDLSIALEKΨAQRAFVEWLLAQKPSSGAPPPS;
Y-Aib-EGTFTSDYSIALDKΨAQRAFVEWLLAQKPSSGAPPPS;
Y-DAIa-EGTFTSDYSIALDKΨAQRAFVEWLLAQKPSSGAPPPS;
Y-Aib-EGTFTSDYSIALDKΨAQRAFVEWLLAQGPSSGAPPPS;
Y-Aib-EGTFTSDYSIALDEΨAQRAFVEWLLAQGPSSGAPPPS;
Y-Aib-EGTFTSDYSIALDKΨAQRAFIEWLLAQGPSSGAPPPS-;
Y-Aib-EGTFTSDYSIALDKΨAQRAFVEWLLAQGPSSGAPPP;
Y-Aib-EGTFTSDYSIALDKΨAQRAFVEWLLAQGPSSGAPPPS; or
Y-Aib-EGTFTSDLSIALEKΨAQRAFVEWLLAQGPSSGAPPPS.

The peptide backbone of Formula I may have the sequence:
Y-Aib-EGTFTSDYSIYLDK-K([17-carboxy-heptadecanoyl]-isoGlu-Peg3-Peg3)-AQRAFVEWLLAQGPSSGAPPPS;
Y-Aib-EGTFTSDYSIYLDK-K([19-Carboxy-nonadecanoyl]-isoGlu-Peg3-Peg3)-AQRAFVEWLLAQGPSSGAPPPS;
Y-Aib-EGTFTSDYSIYLDK-K([17-carboxy-heptadecanoyl]-isoGlu)-AQRAFVEWLLAQGPSSGAPPPS;
H-Y-Aib-EGTFTSDYSIYLDK-K([17-carboxy-heptadecanoyl]-isoGlu-GSGSGG)-AQRAFVEWLLAQGPSSGAPPPS;
Y-Aib-EGTFTSDYSIYLDK-K([17-carboxy-heptadecanoyl]-Peg3-Peg3)-AQRAFVEWLLAQGPSSGAPPPS;
Y-Aib-EGTFTSDYSIYLDK-K([17-carboxy-heptadecanoyl]-isoGlu-Peg3-Peg3-Peg3)-AQRAFVEWLLAQGPSSGAPPPS;
Y-Aib-EGTFTSDYSIYLDK-K([17-carboxy-heptadecanoyl]-Dapa-Peg3-Peg3)-AQRAFVEWLLAQGPSSGAPPPS;
Y-Aib-EGTFTSDYSIYLDK-K([19-Carboxy-nonadecanoyl]-isoGlu-Peg3-Peg3-Peg3)-AQRAFVEWLLAQGPSSGAPPPS;
Y-DAIa-EGTFTSDYSIYLDK-K([17-carboxy-heptadecanoyl]-isoGlu-Peg3-Peg3)-AQRAFVEWLLAQGPSSGAPPPS;
Y-Aib-EGTFTSDYSI-Aib-LDK-K([17-carboxy-heptadecanoyl]-isoGlu-Peg3-Peg3)-AQRAFVEWLLAQGPSSGAPPPS;
Y-Aib-EGTFTSDYSIALEK-K([17-carboxy-heptadecanoyl]-isoGlu-Peg3-Peg3)-AQRAFVEWLLAQK;
Y-Aib-EGTFTSDLSIALEK-K([17-carboxy-heptadecanoyl]-isoGlu-Peg3-Peg3)-AQRAFVEWLLAQK;
Y-Aib-EGTFTSDYSIALEK-K[19-carboxy-nonadecanoyl]-isoGlu-Peg3-Peg3)-AQRAFVEWLLAQK;
Y-Aib-EGTFTSDLSIALEK-K([19-carboxy-nonadecanoyl]-isoGlu-Peg3-Peg3)-AQRAFVEWLLAQK;
Y-DAIa-EGTFTSDYSIYLDK-K([19-carboxy-nonadecanoyl]-isoGlu-Peg3-Peg3)-AQRAFVEWLLAQGPSSGAPPPS;
Y-Aib-EGTFTSDYSIALEK-K([19-carboxy-nonadecanoyl]-isoGlu-Peg3-Peg3)-AQRAFVEWLLAQGPSSGAPPPS;
Y-DAIa-EGTFTSDYSIALEK-K([19-carboxy-nonadecanoyl]-isoGlu-Peg3-Peg3)-AQRAFVEWLLAQGPSSGAPPPS;
Y-Aib-EGTFTSDLSIALEK-K([19-carboxy-nonadecanoyl]-isoGlu-Peg3-Peg3)-AQRAFVEWLLAQKPSSGAPPPS;
Y-Aib-EGTFTSDYSIYLDK-K([19-carboxy-nonadecanoyl]-Peg3-Peg3)-AQRAFVEWLLAQGPSSGAPPPS;
Y-Aib-EGTFTSDYSIYLDK-K([19-carboxy-nonadecanoyl]-lsoGlu)-AQRAFVEWLLAQGPSSGAPPPS;
Y-Aib-EGTFTSDYSIYLDK-K([19-carboxy-nonadecanoyl]-Dapa-Peg3-Peg3)-AQRAFVEWLLAQGPSSGAPPPS;
Y-Aib-EGTFTSDLSIALEK-K([19-carboxy-nonadecanoyl]-isoGlu-Peg3-Peg3)-AQRAFVEWLLAQGPSSGAPPPS
Y-Aib-EGTFTSDYSIALDK-K([19-carboxy-nonadecanoyl]-isoGlu-Peg3-Peg3)-AQRAFVEWLLAQKPSSGAPPPS;
Y-DAIa-EGTFTSDYSIALDK-K([19-carboxy-nonadecanoyl]-isoGlu-Peg3-Peg3)-AQRAFVEWLLAQKPSSGAPPPS;
Y-Aib-EGTFTSDYSIALDK-K([19-carboxy-nonadecanoyl]-isoGlu-Peg3-Peg3)-AQRAFVEWLLAQGPSSGAPPPS;
Y-Aib-EGTFTSDYSIALDE-K([19-carboxy-nonadecanoyl]-isoGlu-Peg3-Peg3)-AQRAFVEWLLAQGPSSGAPPPS;
Y-Aib-EGTFTSDYSIALDK-K([19-carboxy-nonadecanoyl]-isoGlu-Peg3-Peg3)-AQRAFIEWLLAQGPSSGAPPPS;
Y-Aib-EGTFTSDYSIALDK-K([17-carboxy-heptadecanoyl]-isoGlu-Peg3-Peg3)-AQRAFVEWLLAQGPSSGAPPPS; or
Y-Aib-EGTFTSDYSIALDK-K((19-Carboxy-nonadecanoyl)-[(Piperazine-1-yl)-acetyl]-Peg3-Peg3)-AQRAFVEWLLAQGPSSGAPPPS.

The GIP analogue may be:
H-Y-Aib-EGTFTSDYSIYLDK-K([17-carboxy-heptadecanoyl]-isoGlu-Peg3-Peg3)-AQRAFVEWLLAQGPSSGAPPPS-NH₂
H-Y-Aib-EGTFTSDYSIYLDK-K([19-Carboxy-nonadecanoyl]-isoGlu-Peg3-Peg3)-AQRAFVEWLLAQGPSSGAPPPS-NH₂
H-Y-Aib-EGTFTSDYSIYLDK-K([17-carboxy-heptadecanoyl]-isoGlu)-AQRAFVEWLLAQGPSSGAPPPS-NH₂
H-Y-Aib-EGTFTSDYSIYLDK-K([17-carboxy-heptadecanoyl]-isoGlu-GSGSGG)-AQRAFVEWLLAQGPSSGAPPPS-NH₂
H-Y-Aib-EGTFTSDYSIYLDK-K([17-carboxy-heptadecanoyl]-Peg3-Peg3)-AQRAFVEWLLAQGPSSGAPPPS-NH₂
H-Y-Aib-EGTFTSDYSIYLDK-K([17-carboxy-heptadecanoyl]-isoGlu-Peg3-Peg3-Peg3)-AQRAFVEWLLAQGPSSGAPPPS-NH₂
H-Y-Aib-EGTFTSDYSIYLDK-K([17-carboxy-heptadecanoyl]-Dapa-Peg3-Peg3)-AQRAFVEWLLAQGPSSGAPPPS-NH₂
H-Y-Aib-EGTFTSDYSIYLDK-K([19-Carboxy-nonadecanoyl]-isoGlu-Peg3-Peg3-Peg3)-AQRAFVEWLLAQGPSSGAPPPS-NH₂
H-Y-DAIa-EGTFTSDYSIYLDK-K([17-carboxy-heptadecanoyl]-isoGlu-Peg3-Peg3)-AQRAFVEWLLAQGPSSGAPPPS-NH₂
H-Y-Aib-EGTFTSDYSI-Aib-LDK-K([17-carboxy-heptadecanoyl]-isoGlu-Peg3-Peg3)-AQRAFVEWLLAQGPSSGAPPPS-NH₂
H-Y-Aib-EGTFTSDYSIALEK-K([17-carboxy-heptadecanoyl]-isoGlu-Peg3-Peg3)-AQRAFVEWLLAQK-NH₂
H-Y-Aib-EGTFTSDLSIALEK-K([17-carboxy-heptadecanoyl]-isoGlu-Peg3-Peg3)-AQRAFVEWLLAQK-NH₂
H-Y-Aib-EGTFTSDYSIALEK-K[19-carboxy-nonadecanoyl]-isoGlu-Peg3-Peg3)-AQRAFVEWLLAQK-NH₂
H-Y-Aib-EGTFTSDLSIALEK-K([19-carboxy-nonadecanoyl]-isoGlu-Peg3-Peg3)-AQRAFVEWLLAQK-NH₂
H-Y-DAIa-EGTFTSDYSIYLDK-K([19-carboxy-nonadecanoyl]-isoGlu-Peg3-Peg3)-AQRAFVEWLLAQGPSSGAPPPS-NH₂
H-Y-Aib-EGTFTSDYSIALEK-K([19-carboxy-nonadecanoyl]-isoGlu-Peg3-Peg3)-AQRAFVEWLLAQGPSSGAPPPS-NH₂
H-Y-DAIa-EGTFTSDYSIALEK-K([19-carboxy-nonadecanoyl]-isoGlu-Peg3-Peg3)-AQRAFVEWLLAQGPSSGAPPPS-NH₂
H-Y-Aib-EGTFTSDLSIALEK-K([19-carboxy-nonadecanoyl]-isoGlu-Peg3-Peg3)-AQRAFVEWLLAQGPSSGAPPPS-NH₂
H-Y-Aib-EGTFTSDLSIALEK-K([19-carboxy-nonadecanoyl]-isoGlu-Peg3-Peg3)-AQRAFVEWLLAQKPSSGAPPPS-NH₂
H-Y-Aib-EGTFTSDYSIYLDK-K([19-carboxy-nonadecanoyl]-Peg3-Peg3)-AQRAFVEWLLAQGPSSGAPPPS-NH₂
H-Y-Aib-EGTFTSDYSIYLDK-K([19-carboxy-nonadecanoyl]-lsoGlu)-AQRAFVEWLLAQGPSSGAPPPS-NH₂
H-Y-Aib-EGTFTSDYSIYLDK-K([19-carboxy-nonadecanoyl]-Dapa-Peg3-Peg3)-AQRAFVEWLLAQGPSSGAPPPS-NH₂
H-Y-Aib-EGTFTSDYSIALDK-K([19-carboxy-nonadecanoyl]-isoGlu-Peg3-Peg3)-AQRAFVEWLLAQKPSSGAPPPS-NH₂
H-Y-DAIa-EGTFTSDYSIALDK-K([19-carboxy-nonadecanoyl]-isoGlu-Peg3-Peg3)-AQRAFVEWLLAQKPSSGAPPPS-NH₂
H-Y-Aib-EGTFTSDYSIALDK-K([19-carboxy-nonadecanoyl]-isoGlu-Peg3-Peg3)-AQRAFVEWLLAQGPSSGAPPPS-NH₂
H-Y-Aib-EGTFTSDYSIALDE-K([19-carboxy-nonadecanoyl]-isoGlu-Peg3-Peg3)-AQRAFVEWLLAQGPSSGAPPPS-NH₂
H-Y-Aib-EGTFTSDYSIALDK-K([19-carboxy-nonadecanoyl]-isoGlu-Peg3-Peg3)-AQRAFIEWLLAQGPSSGAPPPS-N H₂
H-Y-Aib-EGTFTSDYSIALDK-K([17-carboxy-heptadecanoyl]-isoGlu-Peg3-Peg3)-AQRAFVEWLLAQGPSSGAPPPS-NH₂
H-Y-Aib-EGTFTSDYSIALDK-K((19-Carboxy-nonadecanoyl)-[(Piperazine-1-yl)-acetyl]-Peg3-Peg3)-AQRAFVEWLLAQGPSSGAPPPS-NH₂

Certain of the Y1 groups, when present, may provide increased stability in vivo, e.g. in serum, and so may contribute to the half life of the GIP analogue. Without wishing to be bound by theory, it is believed that these groups may help to stabilize the three dimensional conformation of the molecule and/or provide resistance to proteolytic degradation.

For example, the Y1 sequences Gly-Pro-Ser-Ser-Gly-Ala-Pro-Pro-Pro-Ser, Gly-Pro-Ser-Ser-Gly-Ala-Pro-Pro-Ser, Pro-Ser-Ser-Gly-Ala-Pro-Pro-Pro-Ser and Pro-Ser-Ser-Gly-Ala-Pro-Pro-Ser have homology with a C-terminal portion of the Exendin-4 molecule and appear to contribute to the stability of the molecule without concomitantly providing significant levels of GLP-1 agonist activity.

The invention further provides a pharmaceutical composition comprising a GIP analogue as described herein, or a pharmaceutically acceptable salt or solvate thereof, in admixture with a carrier, preferably a pharmaceutically acceptable carrier. The GIP analogue may,for example, be a pharmaceutically acceptable acid addition salt.

The pharmaceutical composition may be formulated as a liquid suitable for administration by injection or infusion, or which is formulated to cause slow release of said GIP analogue.

The invention further provides a therapeutic kit comprising a GIP analogue as descibed herein, and a device comprising a GIP analogue as described herein.

The invention further provides a GIP analogue as described herein, or a pharmaceutically acceptable salt or solvate thereof, for use in a method of medical treatment, e.g. for use in the treatment and/or prevention of a metabolic disorder.

The metabolic disorder may be diabetes or a diabetes related disorder, or obesity or an obesity related disorder. The link between obesity and diabetes is well known, so these conditions are not necessarily separate or mutually exclusive.

Diabetes related disorders include insulin resistance, glucose intolerance, increased fasting glucose, pre-diabetes, type 1 diabetes, type 2 diabetes, gestational diabetes hypertension, dyslipidemia, and combinations thereof.

Diabetes related disorders also include atherosclerosis, arteriosclerosis, coronary heart disease, peripheral artery disease and stroke; or conditions associated with atherogenic dyslipidemia, blood fat disorders, elevated blood pressure, hypertension, a prothrombotic state, bone related disorders and a proinflammatory state.

Bone related disorders include, but are not limited to, osteoporosis and increased risk of bone fracture.

The blood fat disorder may be selected from high triglycerides, low HDL cholesterol, high LDL cholesterol, and plaque buildup in artery walls, or a combination thereof.

The prothrombotic state may be selected from high fibrinogen levels in the blood and high plasminogen activator inhibitor-1 levels in the blood.

The proinflammatory state may be an elevated C-reactive protein level in the blood.

Obesity related disorders include obesity linked inflammation, obesity linked gallbladder disease and obesity induced sleep apnea, or may be associated with a condition selected from atherogenic dyslipidemia, blood fat disorders, elevated blood pressure, hypertension, a prothrombotic state, and a proinflammatory state, or a combination thereof.

### Brief Description of the Drawings

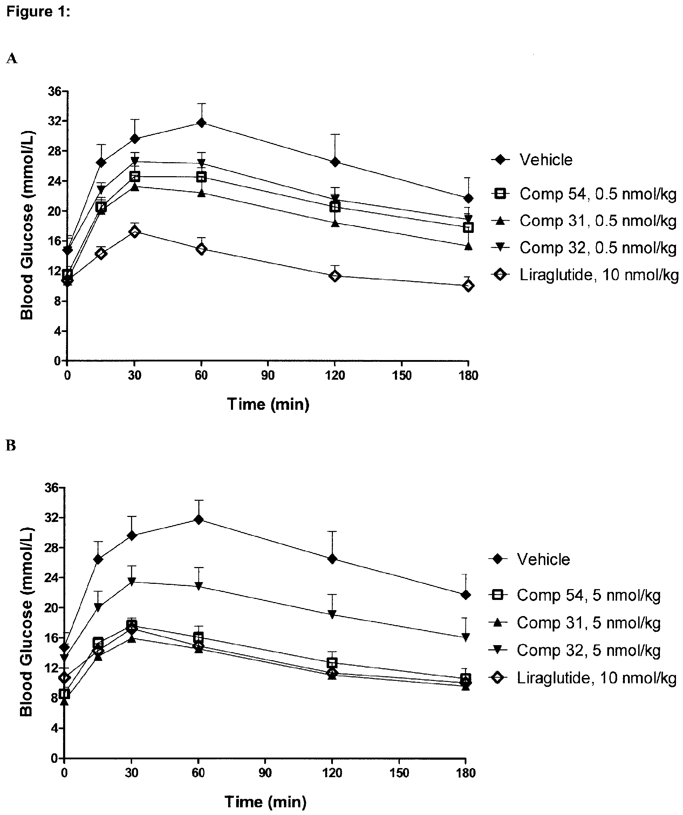

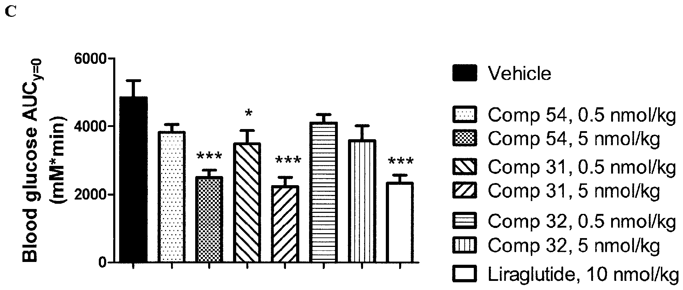

**Figure 1****:** Blood glucose levels **(A, B)** and area under the blood glucose curves (AUC) (C) in an IPGTT in 5-hour fasted diabetic *db*/*db* mice. The mice were injected s.c. with vehicle or GIP-GLP-1 dual acting receptor agonists (0.5 and 5 nmol/kg) 22 hours prior to the i.p. injection of glucose (t = 0). The GLP-1 analogue liraglutide (10 nmol/kg) was administered s.c. 4 hours before the i.p. injection of glucose. Data are means ± SEM; n = 8.

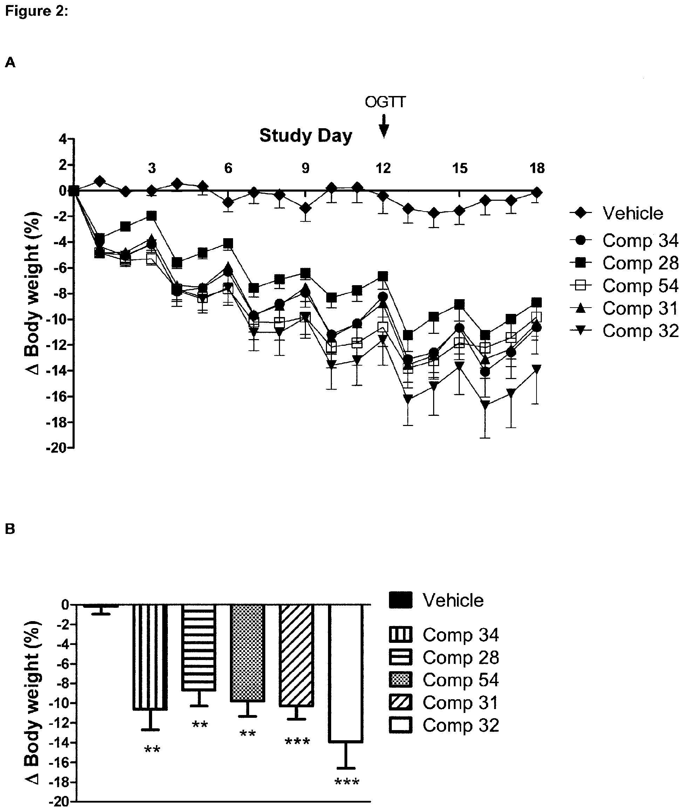

**Figure 2****:** Relative body weight changes (delta Δ = body weight at each study day - body weight at day 0) in DIO mice during the 18-days study period (A) and absolute body weight changes (delta Δ = body weight at day 18 - body weight at day 0) (B). Animals were treated once every third day with s.c. injections of vehicle or GIP-GLP-1 dual acting receptor agonists (3 nmol/kg). Data are means ± SEM; n = 7-9.

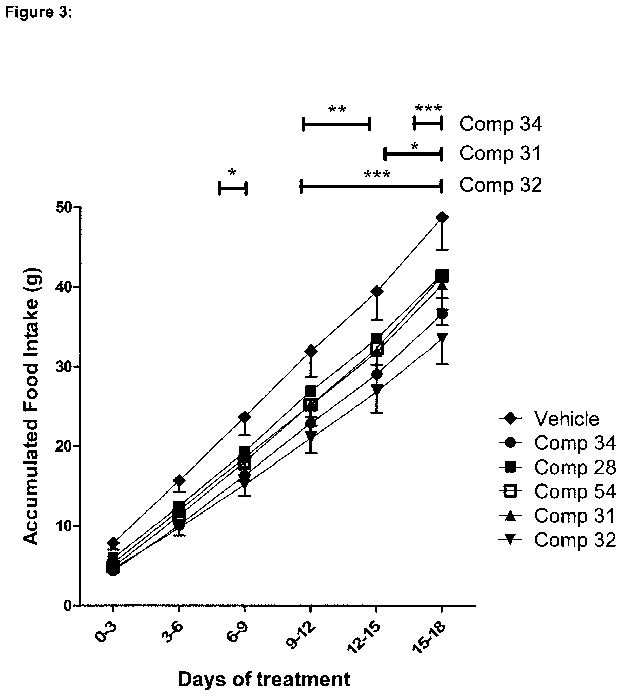

**Figure 3****:** Accumulated food intake in DIO mice. Animals were treated once every third day with s.c. injections of vehicle or GIP-GLP-1 dual acting receptor agonists (3 nmol/kg). Data are means ± SEM; n = 4-5.

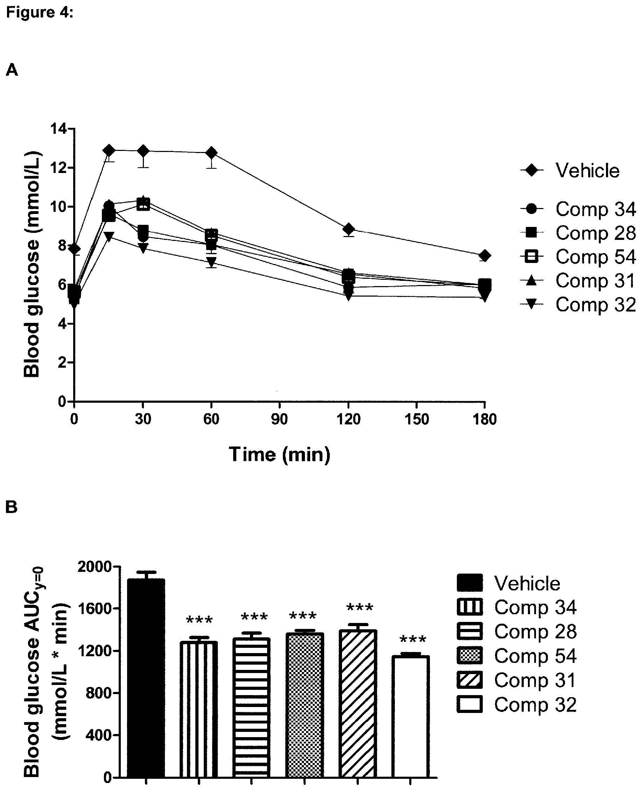

**Figure 4****:** Blood glucose levels during the OGTT **(A)** and area under the blood glucose curves (AUC) **(B)** on day 12 in 5-hour fasted DIO mice. The mice were injected with vehicle or test substances (3 nmol/kg) 5 hours prior to the oral gavage of glucose (t = 0). Data are means ± SEM; n = 7-9.

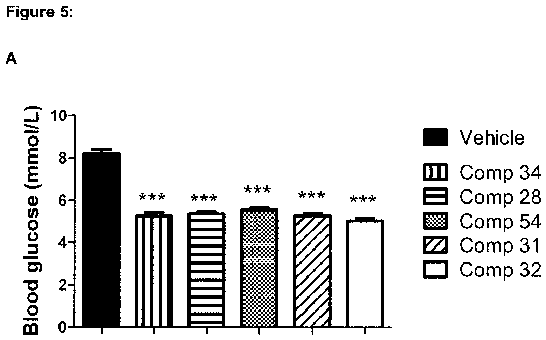

**Figure 5****:** Blood glucose on day 18 in 5-hour fasted DIO mice. The mice were injected with vehicle or GIP-GLP-1 dual acting receptor agonists (3 nmol/kg) 5 hours prior to the blood sampling. Data are means ± SEM; n = 7-9.

### Detailed Description of the Invention

Unless otherwise defined herein, scientific and technical terms used in this application shall have the meanings that are commonly understood by those of ordinary skill in the art. Generally, nomenclature used in connection with, and techniques of, chemistry, molecular biology, cell and cancer biology, immunology, microbiology, pharmacology, and protein and nucleic acid chemistry, described herein, are those well known and commonly used in the art.

### Definitions

Unless specified otherwise, the following definitions are provided for specific terms, which are used in the above written description.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise.

The term "including" is used to mean "including but not limited to." "Including" and "including but not limited to" are used interchangeably.

The terms "patient," "subject," and "individual" may be used interchangeably and refer to either a human or a non-human animal. These terms include mammals such as humans, primates, livestock animals (e.g., bovines, porcines), companion animals (e.g., canines, felines) and rodents (e.g., mice and rats).

The term "solvate" in the context of the present invention refers to a complex of defined stoichiometry formed between a solute (*in casu,* a peptide conjugate or pharmaceutically acceptable salt thereof according to the invention) and a solvent. The solvent in this connection may, for example, be water, ethanol or another pharmaceutically acceptable, typically small-molecular organic species, such as, but not limited to, acetic acid or lactic acid. When the solvent in question is water, such a solvate is normally referred to as a hydrate.

The term "agonist" as employed in the context of the invention refers to a substance (ligand) that activates the receptor type in question.

The term "GIP-GLP 1 dual receptor agonist" as employed in the context of the invention refers to a substance (ligand) that activates both the GIP receptor and the GLP-1 receptor.

Throughout the description and claims the conventional one-letter and three-letter codes for natural (or "proteinogenic") amino acids are used, as well as generally accepted three letter codes for other (non-natural or "non-proteinogenic") α-amino acids, such as Aib (α-aminoisobutyric acid), Orn (ornithine) and D-Ala (D-alanine). All amino acid residues in peptides of the invention are preferably of the L-configuration except where explicitly stated.

Among sequences disclosed herein are sequences incorporating an "H-" moiety at the amino terminus (N-terminus) of the sequence, and either an "-OH" moiety or an "-NH₂" moiety at the carboxy terminus (C-terminus) of the sequence. In such cases, and unless otherwise indicated, an "H-" moiety at the N-terminus of the sequence in question indicates a hydrogen atom (i.e. R¹ = H), corresponding to the presence of a free primary or secondary amino group at the N-terminus, while an "-OH" or an "-NH₂" moiety at the C-terminus of the sequence (i.e. R² = OH or NH₂) indicates a carboxy (COOH) group or an amido (CONH₂) group at the C-terminus, respectively.

Other R¹ groups are possible at the N-terminus, including pyroglutamic acid (pGlu; (S)-(-)-2-pyrrolidone-5-carboxylic acid), C₁₋₄ alkyl, acetyl, formyl, benzoyl and trifluoroacetyl.

### Receptor agonist activity

As mentioned above, the compounds described herein are GIP-GLP 1 dual receptor agonists. That is to say, they have agonist activity at both the GIP receptor and the GLP-1 receptor.

The term "agonist" as employed in the context of the invention refers to a substance (ligand) that is capable of binding to a particular receptor and activating signaling by that receptor. Thus a GIP receptor agonist is capable of binding to the GIP receptor (designated GIP-R) and activating signaling by that receptor, e.g. by generation of cAMP or inducing Ca²⁺ release. Agonist activity at the GIP receptor may therefore be measured by assessing GIP receptor signalling, which may may, for example, be measured via cAMP production or Ca²⁺ release.

The cDNA sequence encoding the human GIP receptor has GenBank accession no. BC101673.1 (Gl:75516688). The encoded amino acid sequence (including signal peptide) is: 
1 MTTSPILQLL LRLSLCGLLL QRAETGSKGQ TAGELYQRWE RYRRECQETL AAAEPPSGLA

61 CNGSFDMYVC WDYAAPNATA RASCPWYLPW HHHVAAGFVL RQCGSDGQWG LWRDHTQCEN

121 PEKNEAFLDQ RLILERLQVM YTVGYSLSLA TLLLALLILS LFRRLHCTRN YIHINLFTSF
 
181 MLRAAAILSR DRLLPRPGPY LGDQALALWN QALAACRTAQ IVTQYCVGAN YTWLLVEGVY

241 LHSLLVLVGG SEEGHFRYYL LLGWGAPALF VIPWVIVRYL YENTQCWERN EVKAIWWIIR

301 TPILMTILIN FLIFIRILGI LLSKLRTRQM RCRDYRLRLA RSTLTLVPLL GVHEVVFAPV

361 TEEQARGALR FAKLGFEIFL SSFQGFLVSV LYCFINKEVQ SEIRRGWHHC RLRRSLGEEQ

421 RQLPERAFRA LPSGSGPGEV PTSRGLSSGT LPGPGNEASR ELESYC
 (GenBank AAl01674.1 Gl:75516689). This may be employed in any assays to determine GIP signalling.

Similarly the compounds have agonist activity at the GLP-1 receptor (GLP-1-R), i.e. they are capable of binding to the GLP-1 receptor and activating signaling by that receptor, e.g. by generation of cAMP or inducing Ca²⁺ release. Agonist activity at the GLP-1 receptor may therefore be measured by assessing GLP-1 receptor signalling, which may may, for example, be measured via cAMP production or Ca²⁺ release.

The GLP-1 receptor may have the sequence of the human glucagon-like peptide 1 receptor (GLP-1R) having primary accession number P43220. The precursor protein (including signal peptide) has primary accession number NP_002053.3; Gl:166795283 and has sequence:

1 MAGAPGPLRL ALLLLGMVGR AGPRPQGATV SLWETVQKWR EYRRQCQRSL TEDPPPATDL
61 FCNRTFDEYA CWPDGEPGSF VNVSCPWYLP WASSVPQGHV YRFCTAEGLW LQKDNSSLPW
121 RDLSECEESK RGERSSPEEQ LLFLYIIYTV GYALSFSALV IASAILLGFR HLHCTRNYIH
181 LNLFASFILR ALSVFIKDAA LKWMYSTAAQ QHQWDGLLSY QDSLSCRLVF LLMQYCVAAN
241 YYWLLVEGVY LYTLLAFSVL SEQWIFRLYV SIGWGVPLLF VVPWGIVKYL YEDEGCWTRN
301 SNMNYWLIIR LPILFAIGVN FLIFVRVICI VVSKLKANLM CKTDIKCRLA KSTLTLIPLL
361 GTHEVIFAFV MDEHARGTLR FIKLFTELSF TSFQGLMVAI LYCFVNNEVQ LEFRKSWERW
421 RLEHLHIQRD SSMKPLKCPT SSLSSGATAG SSMYTATCQA SCS.

This may be employed in any assays to determine GIP signalling.

The compounds of the present invention have at least one GIP and one GLP-1 biological activity, in particular in treatment of metabolic diseases such as diabetes and obesity. This can be assessed, e.g., in *in vivo* assays, for example as described in the examples, in which the blood glucose level or another biological activity is determined after a test animal has been treated or exposed to a GIP analogue. In particular, compounds of the invention may be capable of improving glycaemic control when adminstered to a diabetic subject. Additionally or alternatively, they may be capable of reducing body weight when administered to an overweight or obese subject. In either case, the effect may be superior to that obtained with an equivalent quantity (by mass, or molar ratio) of wild type human GIP or GLP-1 in comparable subjects when given according to a comparable dosing regime.

Activity in *in vitro* assays may also be used as a measure of the compounds' activity. Typically the compounds have activity at both the GLP-1 and GIP receptors (designated GLP-1-R and GIP-R respectively). EC₅₀ values may be used as a numerical measure of agonist potency at a given receptor. An EC₅₀ value is a measure of the concentration of a compound required to achieve half of that compound's maximal activity in a particular assay. Thus, for example, a compound having EC₅₀ [GLP-1R] lower than the EC₅₀ [GLP-1R] of native GIP in a particular assay may be considered to have higher potency at the GLP-1R than GIP. In some embodiments of the present invention, the EC₅₀ GLP-1-R and/or EC₅₀ GIP-R is below 1.0 nM, below 0.9 nM, below 0.8 nM, below 0.7 nM, below 0.6 nM, below 0.5 nM, below 0.4 nM, below 0.3 nM, below 0.2 nM, below 0.1 nM, below 0.09 nM, below 0.08 nM, below 0.07 nM, below 0.06 nM, below 0.05 nM, below 0.04 nM, below 0.03 nM, below 0.02 nM, below 0.01 nM, below 0.009 nM, below 0.008 nM, below 0.007 nM, below 0.006 nM, below 0.005 nM, or below 0.004 nM, e.g. when assessed using the assay described in Example 2. In any given assay, the EC₅₀ value of a compound in a given assay may be assessed relative to the EC₅₀ of human GIP. Thus, the ratio of the EC₅₀ value of the test compound to the EC₅₀ value of wild type human GIP (EC₅₀[test compound] / EC₅₀[GIP]) at the human GIP receptor may be less than 10, less than 5, less than 1, less than 0.1, less than 0.05 or less than 0.01. The ratio of the EC₅₀ value of the test compound to the EC₅₀ value of wild type human GIP (EC₅₀[test compound] / EC₅₀[GIP]) at the GLP-1 receptor may be less than 10, less than 5, less than 1, less than 0.1, less than 0.05 or less than 0.01. It may also be desirable to compare the ratio of EC₅₀ values at the two receptors for the test compound and for human GIP. Preferably the test compound has an EC₅₀[GIP] / EC₅₀[GLP-1] which is greater than the equivalent ratio for GIP in the same assays.

### Lipophilic group

In all aspects, the compound of the invention comprises a residue Ψ, i.e. a residue selected from Lys, Arg, Orn and Cys in which the side chain is conjugated to a substituent -Z²-Z¹- as decribed in more detail below.

Without wishing to be bound by any particular theory, it is thought that the substituent binds plasma proteins (e.g. albumin) in the blood stream, thus shielding the compounds of the invention from enzymatic degradation and renal clearance and thereby enhancing the half-life of the compounds. It may also modulate the potency of the compound, e.g. with respect to the GIP receptor and/or the GLP-1 receptor.

The substituent is conjugated to the functional group at the distal end of the side chain from the alpha-carbon. The normal ability of the Lys, Arg, Orn or Cys side chain to participate in interactions mediated by that functional group (e.g. intra- and inter-molecular interactions) may therefore be reduced or completely eliminated by the presence of the substituent. Thus, the overall properties of the compound may be relatively insensitive to changes in the actual amino acid present as residue Ψ. Consequently, it is believed that any of the residues Lys, Arg, Orn and Cys may be present at any position where Ψ is permitted. However, in certain embodiments, it may be advantageous that the amino acid component of Ψ is Lys.

Ψ is a residue of Lys, Arg, Orn or Cys in which the side chain is conjugated to a substituent having the formula -Z¹ or -Z²-Z¹;
-Z¹ is a fatty chain having a polar group at one end of the chain and a connection to Ψ or Z², -X-, at the end of the chain distal from the polar group,
wherein the polar group comprises a carboxylic acid or a carboxylic acid bioisostere, a phosphonic acid, or a sulfonic acid group;
and -X- is a bond, -CO-, -SO-, or -SO₂-;
-Z²-, if present, is a spacer of formula: 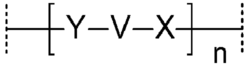 connecting Z¹ to Ψ;
wherein:
each Y is independently -NH, -NR, -S or -O, where R is alkyl, a protecting group or forms a linkage to another part of the spacer Z²;
each X is independently a bond, CO-, SO-, or SO₂-;
with the proviso that when Y is -S, the X to which it is bound is a bond;
each V is independently a bivalent organic moiety linking Y and X;
and n is 1-10;
*The group Z¹*
Z¹ is a fatty chain having a connection to Ψ or to Z², referred to herein as -X- and, at the end of the chain distal from the connection to Z², a polar group. -X- may be, for example, a bond, acyl (-CO-), sulfinyl (-SO-), or sulfonyl (-SO₂-), the connection being located at the ω-position with respect to the polar group, that is, at the end of the chain distal from the polar group. When Z¹ is bound directly to Ψ, that is, when Z² is not present, preferably -X- is acyl (-CO-), sulfinyl (-SO-), or sulfonyl (-SO₂-). Most preferably, -X- is acyl (-CO-).

Preferably, the polar group is an acidic or weakly acid group, for example a carboxylic acid or a carboxylic acid bioisostere, a phosphonate, or a sulfonate. The polar group may have a pKₐ of between -2 and 12 in water, more preferably between 1 and 7, more preferably between 3 and 6. Certain preferred polar groups have a pKₐ of between 4 and 5.

The polar group preferably comprises a carboxylic acid or carboxylic acid bioisostere. Suitable carboxylic acid bioisosteres are known in the art. Preferably the bioisostere has a proton having a pKₐ similar to the corresponding carboxylic acid. Examples of suitable bioisoteres may include, not by way of limitation, tetrazole, acylsulfomides, acylhydroxylamine, and squaric acid derivatives, as shown below (--- indicates the point of attachment): 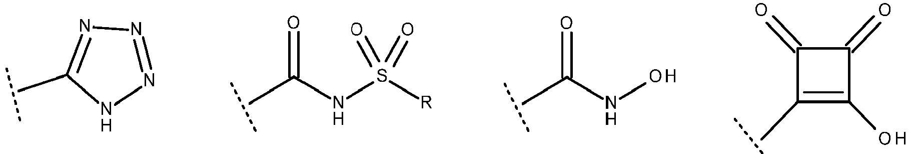 R is e.g. Me, CF₃

The polar group may be a group of formula A-B-, wherein A is a carboxylic acid (-COOH) or a carboxylic acid bioisostere, a phosphonic acid (-P(O)(OH)₂), or a sulfonic acid (-SO₂OH) group, and B is a bond or linker between A and the fatty chain. In some embodiments, the polar group is -COOH, that is, A is -COOH and B is a bond.

When B is a linker, it may be a cycloalkylene, heterocycloalkylene, C₆arylene, or C₅₋₆heteroarylene, or C₆arylene-O- or C₅₋₆heteroarylene-O-.

When B is phenylene it may, for example, be selected from 1,2-phenylene, 1,3-phenylene, 1,4-phenylene, preferably 1,4-phenylene (so that A-B- is a 4-benzoic acid substituent or 4-benzoic acid bioisostere). When B is phenylene-O-, it may, for example, be selected from 1,2-phenylene-O-, 1,3-phenylene-O-, 1,4-phenylene-O-, preferably 1,4-phenylene-O. Each phenylene of B may be optionally substituted with one or more substituents selected from fluoro, methyl, trifluoromethyl, amino, hydroxyl, and C₁₋₄alkoxy, preferably methoxy. It will be appreciated that substituent identity and position may be selected to subtly alter the pKₐ of the polar group. Suitable inductively or mesomerically electron-withdrawing or donating groups and their positional effects are known in the art. In some embodiments, B may be C₅₋₆heteroarylene, for example, pyridinylene or thiofuranylene, and may be optionally substituted as described.

For example, in some embodiments, A-B- may be selected from:

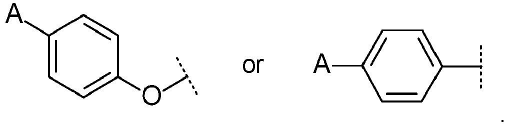

Preferably, A is -COOH. In some preferred polar groups, A is a carboxylic acid and B is C₆arylene-O-.

Fatty chain as used herein refers to a moiety comprising a chain of carbon atoms, the carbon atoms being predominantly substituted with hydrogen or hydrogen-like atoms, for example, a hydrocarbon chain. Such fatty chains are often referred to as lipophilic, although it will be appreciated that substitution may alter the lipophilic properties of the overall molecule.

The fatty chain may by aliphatic. It may be entirely saturated or may include one or more double or triple bonds. Each double bond, if present, may be in the E or Z configuration. The fatty chain may also have one or more cycloalkylene or heterocycloalkylene moieties in its length, and additionally or alternatively may have one or more arylene or heteroarylene moieties in its length. For example, the fatty chain may incorporate a phenylene or piperazinylene moiety in its length as, for example, shown below (wherein --- represents the points of attachment within the chain).

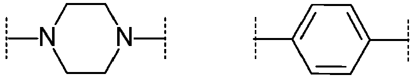

The fatty chain may be derived from a fatty acid, for example, it may be derived from a medium-chain fatty acid (MCFA) with an aliphatic tail of 6-12 carbon atoms, a long-chain fatty acid (LCFA) with an aliphatic tail of 13-21 carbon atoms, or a very long-chain fatty acid (LCFA) with an aliphatic tail of 22 carbon atoms or more. Examples of linear saturated fatty acids from which suitable fatty chains may be derived include tridecylic (tridecanoic) acid, myristic (tetradecanoic) acid, pentadecylic (pentadecanoic) acid, palmitic (hexadecanoic) acid, margaric (heptadecanoic) acid, and arachidic (eicosanoic) acid. Examples of linear unsaturated fatty acids from which suitable fatty chains may be derived include myristoleic acid, palmitoleic acid, sapienic acid and oleic acid.

The fatty chain may be connected to Ψ or to Z² by an amide linkage, a sulfinamide linkage, a sulfonamide linkage, or by an ester linkage, or by an ether, thioether or amine linkage. Accordingly, the fatty chain may have at the ω position, that is, the position distal to the polar group, a bond to Z² or an acyl (-CO-), sulfinyl (-SO-), or sulfonyl (-SO₂-) group. Preferably, the fatty chain has an acyl (-CO-) group at the position distal to the polar group and is connected to Z² by an amide or ester linkage.

In some embodiments, Z¹ is a group of formula:

A-B-Alk-X-

where A-B- is the polar group defined above, X is a bond, acyl (-CO-), sulfinyl (-SO-), or sulfonyl (-SO₂-), and Alk is a fatty chain that may be optionally substituted with one or more substituents. The fatty chain is preferably 16 to 28 carbon atoms in length (e.g. a C₁₆₋₂₈alkylene), more preferably, 16 to 26 carbons in length (e.g. a C₁₆₋₂₆alkylene), more preferably, 16 to 22 carbons in length (e.g. C₁₆₋alkylene), and may be saturated or unsaturated. Preferably, Alk is saturated, that is, preferably Alk is alkylene.

In some embodiments, Z¹ is an acyl group of formula:

A-B-Alk-(CO)-

or a sulfonyl group of formula:

A-B-Alk-(SO₂)-.

Optional substituents on the fatty chain may be independently selected from fluoro, C₁₋₄alkyl, preferably methyl; trifluoromethyl, hydroxymethyl, amino, hydroxyl, C₁₋₄alkoxy, preferably methoxy; oxo, and carboxyl, and may be independently located at any point along the chain. In some embodiments, each optional substituent is selected from fluoro, methyl, and hydroxyl. Where more than one substituent is present, substituents may be the same or different. Preferably, the number of substituents is 0 to 3; more preferably the fatty chain is unsubstituted.

Preferably, Z¹ is an acyl group of formula:

A-B-alkylene-(CO)-

where A and B are as defined above.

In some embodiments, Z¹ is:

4-carboxyphenoxynonanoyl HOOC-C₆H₄-O-(CH₂)₈-(CO)-.

Certain preferred Z¹ are derived from long-chain saturated α,ω-dicarboxyhc acids of formula HOOC-(CH₂)₁₂₋₂₂-COOH, preferably, long-chain saturated α,ω-dicarboxyhc acids having an even number of carbon atoms in the aliphatic chain. For example, and not by way of limitation, Z¹ may be:

17-carboxyheptadecanoyl HOOC-(CH₂)₁₆-(CO)-;

19-carboxynonadecanoyl HOOC-(CH₂)₁₈-(CO)-;

or

21-carboxyhenicosanoyl HOOC-(CH₂)₂₀-(CO)-.

The carboxylic acid group may be replaced by a bioisotere as detailed herein.

### The group Z²

Z² is an optional spacer that connects Z¹ to the side chain of the amino acid component of Ψ. At its most general, Z², if present, is a spacer bound at one terminus by Y, which may be a nitrogen, oxygen or sulfur atom, and at the other terminus by X, which may be a bond or an acyl (-CO-), sulfinyl (-SO-), sulfonyl (-SO₂-) or absent. Accordingly, Z² may be a spacer of formula (--- indicate points of attachment): 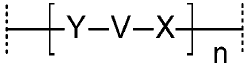 wherein:
Y may be -NH, -NR, -S or -O, where R may be alkyl, a protecting group or may form a linkage to another part of the spacer, with the remaining valency forming a linkage to Z¹;
X may be a bond, CO-, SO-, or SO₂-, with the remaining valency forming a linkage to the side chain of the amino acid component of Ψ;
V is a bivalent organic moiety linking Y and X;
and n may be 1, 2, 3, 4, 5, 6, 7, 8, 9 or 10. Where n is 2 or more, each Y, V, and X is independent of every other Y, V, and X.

Accordingly, Z² may be bound at each side by amide, sulfinamide, sulfonamide, or ester linkages or by amino, ether, or thioether linkages depending upon the nature of Y and X and the corresponding linking groups on Z¹ and the side chain. Where n is 2 or greater, each V may also be bound to each adjacent V by linkages as described. Preferably, linkages are amides, esters or sulfonamides, most preferably amides. Accordingly, in some embodiments, each Y is -NH or -NR and each X is CO- or SO₂-.

In some embodiments, Z² is a spacer of formula -S_{A}-, -S_{B}-, -S_{A}-S_{B}- or -S_{B}-S_{A}-, wherein S_{A} and S_{B} are as defined below.

In some embodiments, Z² is selected from -S_{A}-, -S_{B}-, or -S_{B}-S_{A}-, that is, [side chain]-Z²Z¹ is [side chain]-S_{A}-Z¹, [side chain]-S_{B}-Z¹ or [side chain]-S_{B}-S_{A}-Z¹.

### The group S_{A}

S_{A} may be a single amino acid residue or a residue of an amino acid derivative, especially an amino acid derivative residue having a sulfinyl or sulfonyl in place of the carboxy moiety at the C terminus. Additionally or alternatively, the single amino acid residue may have an oxygen or sulfur atom in place of the nitrogen atom at the N terminus.

S_{A} may comprise a nitrogen-containing heterocycle, said nitrogen-containing heterocycle being bound within the lipophilic group at one end via a bond, a carboxy, a sulfinyl, or a sulfonyl group, and at the other via a ring nitrogen atom. For example, S_{A} may comprise a piperazine ring.

Suitably, S_{A} is a 5-8-membered heterocycle having 1 or 2 nitrogen atoms and substituted with an -X or an -L-X group, where X is a bond, CO-, SO-, or SO₂-, and where L, if present, is C₁₋₄alkylene (- denotes a point of attachment within the lipophilic group).

Preferably, S_{A} is a 6-membered heterocycle having 1 or 2 nitrogen atoms, preferably 2, and substituted with a -CH₂CO-, -CH₂SO-, or -CH₂SO₂- group.For example, S_{A} may be:

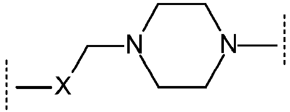

For example, S_{A} may be: 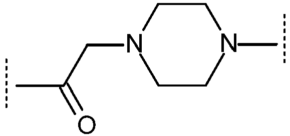 (referred to herein as piperazine-1-yl-acetyl).

Preferably, S_{A} is a single amino acid residue or piperazine-1-yl-acetyl. More preferably S_{A} is a single amino acid residue In some embodiments, the amino acid may be selected from γ-Glu, α-Glu, α-Asp, β-Asp, Ala, Dapa (2,3-diaminopropionic acid), or Dab (2,4-diaminobutanoic). It will be understood that where more than one carboxylic acid or amino moiety is present, connection may be at any moiety as appropriate. Any carboxylic acid or amino resides not bound within the residue may be free, that is, present as a free carboxylic acid or primary amine, or may be derivatised. Suitable derivatisation is known in the art. For example, carboxylic acid moieties may be present in S_{A} amino acid residues as esters, for example, as methyl esters. Amino moieties may be present as alkylated amines, for example, methylated, or may be protected as amide or carbamate moieties. Other suitable amino acids include β-Ala (3-aminopropanoic acid) and Gaba (4-aminobutanoic acid) and similar ω amino acids.

It will be understood that amino acids may be D or L, or a racemic or enantioenriched mixture. In some embodiments, the amino acid is an L-amino acid. In some embodiments, the amino acid is a D-amino acid.

In some preferred embodiments, S_{A} has a carboxylic acid substituent, with γ-Glu, α-Glu, α-Asp, and β-Asp, and sulfinyl and sulfonyl derivatives thereof, being preferred. Accordingly, in some embodiments, the amino acid residue is: 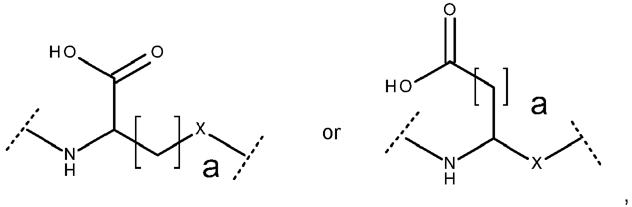 where -X- is -CO-, -SO-, -SO₂-, preferably -CO-, and a is 1 or 2, preferably 2. In some embodiments, the carboxylic acid is an ester, and the amino acid residue is: 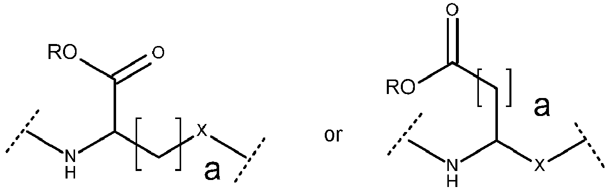 where -X- is -CO-, -SO-, -SO₂-, preferably -CO-, and a is 1 or 2, preferably 2, and R is C₁₋₄alkyl or C₆aryl. Preferably R is C₁₋₄alkyl, preferably methyl or ethyl, more preferably ethyl.

A preferred S_{A} group bearing a carboxylic acid is γ-Glu.

Preferably, S_{A} is selected from Dapa or γ-Glu. Most preferably, S_{A} is γ-Glu.

### The group S_{B}

S_{B} may be a linker of general formula: 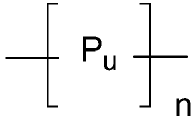 wherein P_{U} is a polymeric unit and n is 1, 2, 3, 4, 5, 6, 7, 8, 9 or 10. One terminus of the linker S_{B} is an -NH, -NR, -S or-O, wherein R may be alkyl, a protecting group or may form a linkage to another part of the polymeric unit; while the other is a bond or CO-, SO- or SO₂-. Accordingly, each polymeric unit P_{U} may be bound at each side by amide, sulfinamide, sulfonamide, or ester linkages or by amino, ether, or thioether linkages depending upon the nature of Y and X and the corresponding linking groups on Z¹, S_{A}, and Lys.

In some embodiments, each P_{U} may be independently a unit of formula: 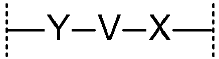 wherein:
Y may be -NH, -NR, -S or -O, wherein R may be alkyl, a protecting group or may form a linkage to another part of the spacer, with the remaining valency forming a linkage to Z¹;
X may be a bond, CO-, SO-, or SO₂-, with the remaining valency forming a linkage to Lys;
and V is a bivalent organic moiety linking Y and X.

In some embodiments, V is the α-carbon of a natural or unnatural amino acid, that is V is - CHR^{AA}-, wherein R^{AA} is an amino acid side chain; or V is an optionally substituted C₁₋₆alkylene, or V is a chain comprising one or more units of ethylene glycol in series, also known as PEG chain, for example, -CH₂CH₂-(OCH₂CH₂)ₘ-O-(CH₂)ₚ-, where m is 0, 1, 2, 3, 4, or 5, and p is 1, 2, 3, 4, or 5; when X is CO-, p is preferably 1, 3, 4, or 5. Optional alkylene substituents include fluoro, methyl, hydroxy, hydroxymethy, and amino.

Preferred P_{U} units include:
(i). Single amino acid residues: P_{U}ⁱ ;
(ii). Dipeptide residues: P_{U}ⁱⁱ; and
(iii). Amino-(PEG)ₘ-carboxylic acid residues: P_{U}ⁱⁱⁱ,
and may be present in any combination or order. For example, S_{B} may comprise one or more of each of P_{U}ⁱ, P_{U}ⁱⁱ, and P_{U}ⁱⁱⁱ in any order, or may comprise one or more units of P_{U}ⁱ, P_{U}ⁱⁱ, and P_{U}ⁱⁱⁱ only, or one of more units selected from P_{U}ⁱ and P_{U}ⁱⁱ, P_{U}ⁱ and P_{U}ⁱⁱⁱ, or P_{U}ⁱⁱ and P_{U}ⁱⁱⁱ.

### (i). P_{U}ⁱ single amino acid residues

Each P_{U}ⁱ may be independently selected from any natural or unnatural amino acid residue and, for example, may be selected from Gly, Pro, Ala, Val, Leu, Ile, Met, Cys, Phe, Tyr, Trp, His, Lys, Arg, Gln, Asn, α-Glu, γ-Glu, Asp, SerThr, Dapa, Gaba, Aib, β-Ala, 5-aminopentanoyl, 6-aminohexanoyl, 7-aminoheptanoyl, 8-aminooctanoyl, 9-aminononanoyl, and 10-aminodecanoyl. Preferably, P_{U}ⁱ amino acid residues are selected from Gly, Ser, Ala, Thr, and Cys, more preferably from Gly and Ser.

In some embodiments, S_{B} is -(P_{U}ⁱ)ₙ-, wherein n is 1 to 8, more preferably 5 to 7, most preferably 6. In some preferred embodiments, S_{B} is -(P_{U}ⁱ)ₙ-, n is 6 and each P_{U}ⁱ is independently selected from Gly or Ser, with a preferred sequence being -Gly-Ser-Gly-Ser-Gly-Gly-.

### (ii). P_{U}ⁱⁱ dipeptide residues

Each P_{U}ⁱⁱ may be independently selected from any dipeptide residue comprising two natural or unnatural amino acid residues bound by an amide linkage. Preferred P_{U}ⁱⁱ dipeptide residues include Gly-Gly, Gly-Ser, Ser-Gly, Gly-Ala, Ala-Gly, and Ala-Ala, more preferably Gly-Ser and Gly-Gly.

In some embodiments, S_{B} is -(P_{U}ⁱⁱ)ₙ-, wherein n is 2 to 4, more preferably 3, and each P_{U}ⁱⁱ is independently selected from Gly-Ser and Gly-Gly. In some preferred embodiments S_{B} is - (P_{U}ⁱⁱ)ₙ-, n is 3 and each P_{U}ⁱⁱ is independently selected from Gly-Ser and Gly-Gly, with a preferred sequence being -(Gly-Ser)-(Gly-Ser)-(Gly-Gly).

Amino acids having stereogenic centres within P_{U}ⁱ and Puⁱⁱ may be racemic, enantioenriched, or enantiopure. In some embodiments, the or each amino acid is independently an L-amino acid. In some embodiments, the or each amino acid is independently a D-amino acid.

### (iii). P_{U}ⁱⁱⁱ amino-(PEG)ₘ-carboxylic acid residues

Each P_{U}ⁱⁱⁱ may be independently a residue of general formula: 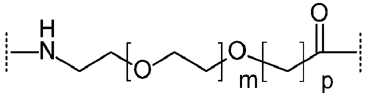 wherein m is 0, 1, 2, 3, 4, or 5, preferably 1, 2, 3, or 4, more preferably 1 or 2, and p is 1, 2, 3, 4 or 5, e.g. 1, 3, 4, or 5, preferably 1.

In some embodiments, m is 1 and p is 1, that is, P_{U}ⁱⁱⁱ is a residue of 8-amino-3,6-dioxaoctanoic acid (also known as {2-[2-aminoethoxy]ethoxy}acetic acid and H₂N-PEG₃-COOH). This residue is referred to herein as -PEG₃-.

In some embodiments, m is 5 and p is 2, that is, P_{U}ⁱⁱⁱ is a residue of 2-[2-[2-[2-[2- [2- (2-aminoethoxy)ethoxy]ethoxy]ethoxy]ethoxy]ethoxy]propanoic acid (also known as H₂N-PEG₆-COOH). This residue is referred to herein as -PEG₆-.

In some embodiments, m is 2 and p is 1, that is, P_{U}ⁱⁱⁱ is a residue of 11-amino-3,6,9-trioxaundecanoic acid (also known as H₂N-PEG₄-COOH). This residue is referred to herein as -PEG₄-

In some embodiments, S_{B} is -(P_{U}ⁱⁱⁱ)ₙ-, wherein n is 1 to 3, more preferably 2.

In some preferred embodiments, S_{B} is selected from -PEG₃-PEG₃- and -PEG₃-PEG₃-PEG3-. Most preferably, S_{B} is -PEG₃-PEG₃-.

### Preferred -Z²-Z¹

It will be understood that the above preferences may be independently combined to give preferred -Z¹ and -Z²-Z¹ combinations.

Some preferred -Z²-Z¹ combinations are shown below (in each case, --- indicates the point of attachment to the side chain of the amino acid component of Ψ:
(i) [17-Carboxy-heptadecanoyl]-isoGlu-Peg3-Peg3 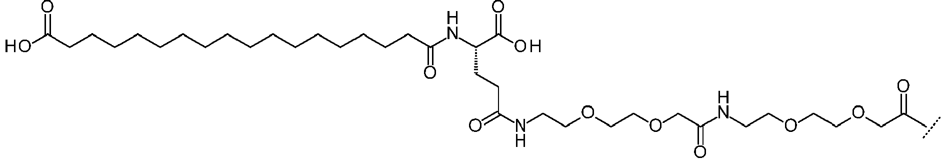
(ii) [17-Carboxy-heptadecanoyl]-isoGlu 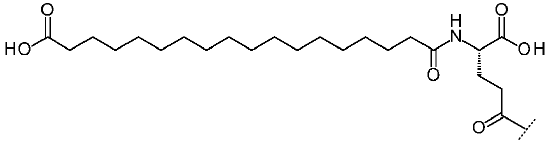
(iii) [17-Carboxy-heptadecanoyl]-Peg3-Peg3-isoGlu 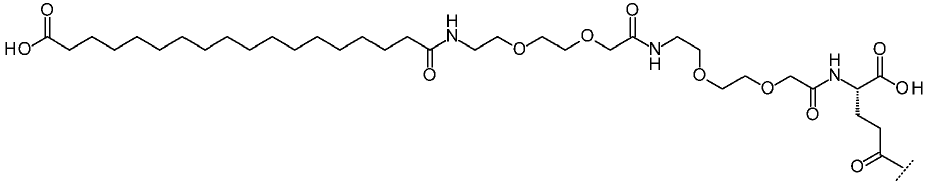
(iv) [17-Carboxy-heptadecanoyl]-isoGlu-GSGSGG 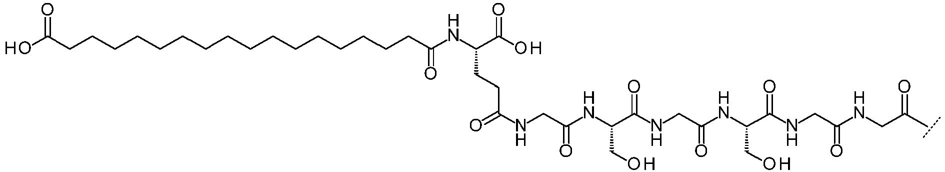
(v) [17-Carboxy-heptadecanoyl]-AA-Peg3-Peg3 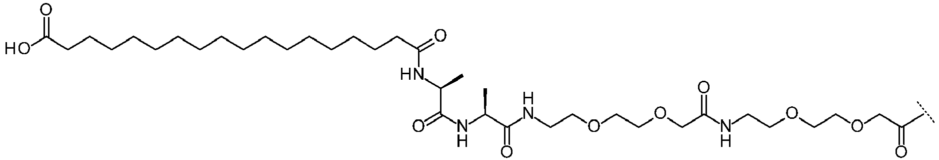
(vi) ([19-Carboxy-nonadecanoyl]-AA-Peg3-Peg3-Peg3) 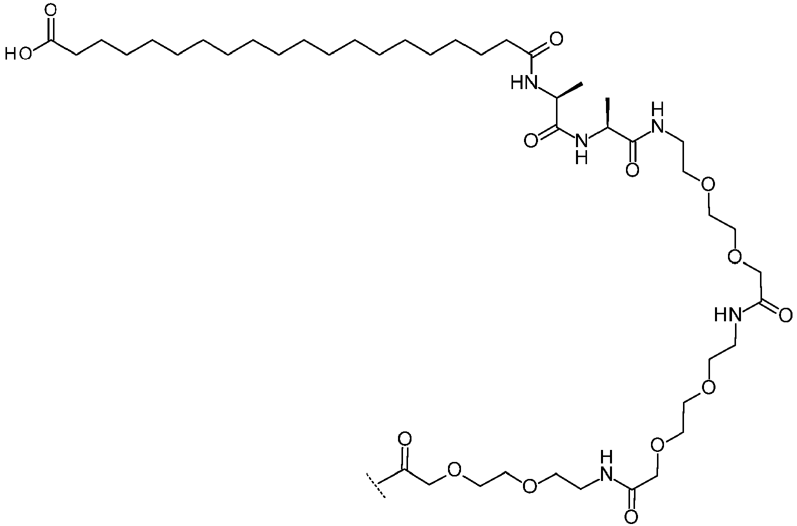
(vii) [17-Carboxy-heptadecanoyl]-Peg3-Peg3 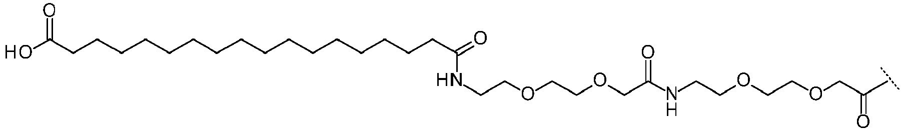
(viii) [17-Carboxy-heptadecanoyl]-isoGlu-Peg3-Peg3-Peg3 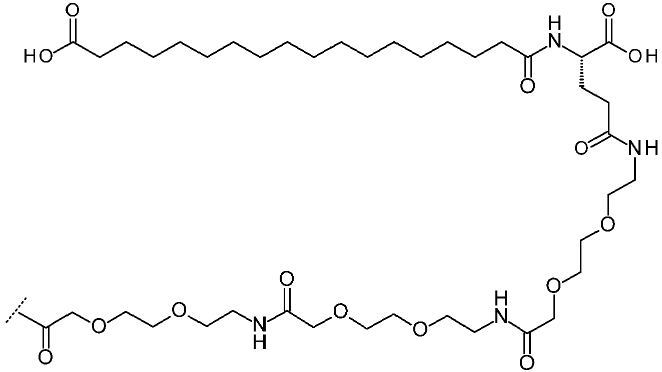
(ix) [17-Carboxy-heptadecanoyl]-Dapa-Peg3-Peg3 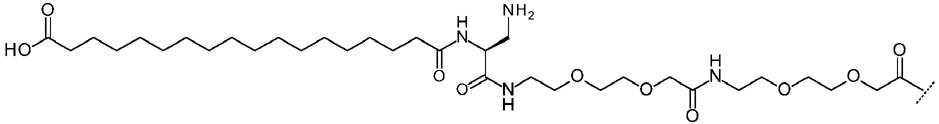
(x) [19-Carboxy-heptadecanoyl]-isoGlu-Peg3-Peg3-Peg3 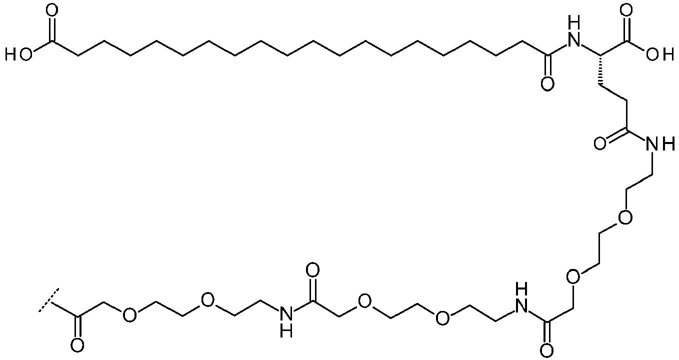
(xi) [19-Carboxy-nonadecanoyl]-isoGlu-Peg3-Peg3 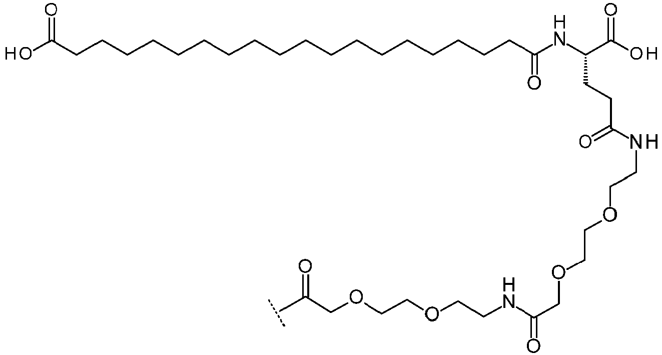
(xii) (19-Carboxy-nonadecanoyl)-[(Piperazine-1-yl)-acetyl]-Peg3-Peg3 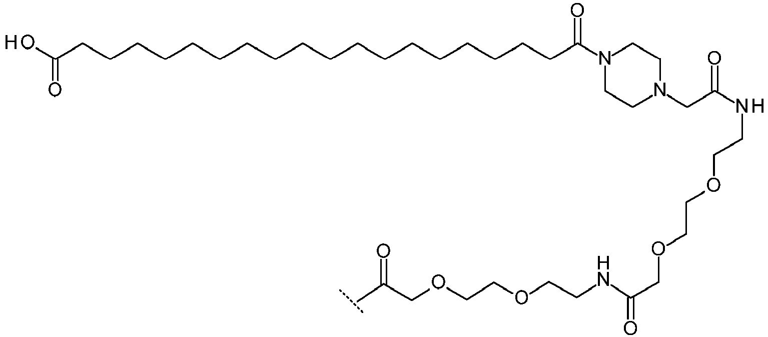
(xiii) (19-Carboxy-nonadecanoyl)-[(Piperazine-1-yl)-acetyl]-Peg6

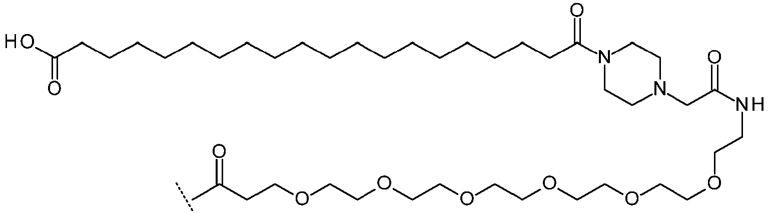

The skilled person will be well aware of suitable techniques for preparing the compounds employed in the context of the invention. For examples of suitable chemistry, see, e.g., WO98/08871, WO00/55184, WO00/55119, Madsen et al. (J. Med. Chem. 2007, 50, 6126-32), and Knudsen et al. 2000 (J. Med Chem. 43, 1664-1669).

### Clinical utility

The GIP analogue compounds employed in the context of the invention may provide an attractive treatment option for metabolic diseases including obesity, diabetes mellitus (diabetes), obesity-related disorders, and diabetes-related disorders. Diabetes comprises a group of metabolic diseases characterized by hyperglycemia resulting from defects in insulin secretion, insulin action, or both. Diabetes is classified into type 1 diabetes, type 2 diabetes and gestational diabetes on the basis on pathogenic characteristics. Type 1 diabetes accounts for 5-10% of all diabetes cases and is caused by auto-immune destruction of insulin-secreting pancreatic β-cells. Acute signs of diabetes include excessive urine production, resulting compensatory thirst and increased fluid intake, blurred vision, unexplained weight loss, lethargy, and changes in energy metabolism. However, in type 2 diabetes symptoms are often not severe or may be absent. The chronic hyperglycemia of diabetes is associated with long-term damage, dysfunction, and failure of various organs, notably the eyes, kidneys, nerves, heart and blood vessels.

Type 2 diabetes accounts for 90-95% of diabetes cases and is a result of a complex set of metabolic disorders. However, symptoms are often not severe or may be absent. Type 2 diabetes is the consequence of endogenous insulin production becoming insufficient to maintain plasma glucose levels below diagnostic thresholds.

Gestational diabetes refers to any degree of glucose intolerance identified during pregnancy.

Pre-diabetes includes impaired fasting glucose and impaired glucose tolerance and refers to those states that occur when blood glucose levels are elevated but below the levels that are established for the clinical diagnosis for diabetes.

A large proportion of people with type 2 diabetes and pre-diabetes are at increased risk of morbidity and mortality due to the high prevalence of additional metabolic risk factors, including abdominal obesity (excessive fat tissue around the abdominal internal organs), atherogenic dyslipidemia (blood fat disorders including high triglycerides, low HDL cholesterol and/or high LDL cholesterol, which foster plaque buildup in artery walls), elevated blood pressure (hypertension) a prothrombotic state (e.g. high fibrinogen or plasminogen activator inhibitor- 1 in the blood), and/or a proinflammatory state (e.g., elevated C-reactive protein in the blood).

Conversely, obesity confers an increased risk of developing pre-diabetes, type 2 diabetes as well as, e.g., certain types of cancer, obstructive sleep apnea and gall-bladder disease. Dyslipidemia is associated with increased risk of cardiovascular disease. High Density Lipoprotein (HDL) is of clinical importance since an inverse correlation exists between plasma HDL concentrations and risk of atherosclerotic disease. The majority of cholesterol stored in atherosclerotic plaques originates from LDL and hence an elevated concentration of Low Density Lipoproteins (LDL) is closely associated with atherosclerosis. The HDL/LDL ratio is a clinical risk indictor for atherosclerosis and coronary atherosclerosis in particular.

Compounds employed in the context of the invention act as GIP-GLP1 dual agonists. The dual agonist may combine the effect of GIP, *e.g.,* on fat metabolism and weight loss, and blood glucose, with the effect of GLP-1, *e.g.,* on blood glucose levels and food intake. They may therefore act to accelerate elimination of excessive adipose tissue, induce sustainable weight loss, and improve glycemic control. Dual GIP-GLP1 agonists may also act to reduce cardiovascular risk factors such as high cholesterol, such as high LDL-cholesterol.

The GIP-GLP1 dual agonist compounds of the present invention may therefore be used (alone or in combination) as pharmaceutical agents for preventing weight gain, promoting weight loss, reducing excess body weight or treating obesity (e.g., by control of appetite, feeding, food intake, calorie intake, and/or energy expenditure and lipolysis), including morbid obesity, as well as associated diseases and health conditions including but not limited to obesity linked inflammation, obesity linked gallbladder disease and obesity induced sleep apnea. The GIP-GLP1 dual agonist compounds employed in the context of the invention may also be used for treatment of insulin resistance, glucose intolerance, pre-diabetes, increased fasting glucose, type 2 diabetes, hypertension, dyslipidemia (or a combination of these metabolic risk factors), atherosclerosis, arteriosclerosis, coronary heart disease, peripheral artery disease and stroke. These are all conditions which may be associated with obesity. However, the effects of the compounds employed in the context of the invention on these conditions may be mediated in whole or in part via an effect on body weight, or may be independent thereof.

The GIP-GLP1 dual agonist compounds may thus be used (alone or in combination) for the treatment and/or prevention of any of the diseases, disorders, or conditions described herein, including insulin resistance, glucose intolerance, increased fasting glucose, pre-diabetes, type 1 diabetes, type 2 diabetes, gestational diabetes hypertension, dyslipidemia, or a combination thereof. In certain embodiments, the diabetes related disorder is selected from atherosclerosis, arteriosclerosis, coronary heart disease, peripheral artery disease and stroke; or associated with a condition selected from atherogenic dyslipidemia, blood fat disorders, elevated blood pressure, hypertension, a prothrombotic state, and proinflammatory state, or a combination thereof. In certain embodiments, the blood fat disorder is selected from high triglycerides, low HDL cholesterol, high LDL cholesterol, plaque buildup in artery walls, or a combination thereof. In certain embodiments, the prothrombotic state is selected from high fibrinogen levels in the blood and high plasminogen activator inhibitor-1 levels in the blood. In certain embodiments, the proinflammatory state is an elevated C-reactive protein level in the blood. In certain embodiments, the obesity related disorder is selected from obesity linked inflammation, obesity linked gallbladder disease and obesity induced sleep apnea.

The GIP-GLP1 dual agonist compounds may also be used for the treatment and/or prevention of any of the diseases, disorders, or conditions associated with diabetes related osteoporosis including increased risk of bone fractures.. The observed increase in fracture risk is likely to be related to impaired bone quality rather than to bone mineral density. The related mechanisms, due at least in part to hyperglycemia, neuropathy, and higher incidence of hypovitaminosis D, are not yet fully understood.

In some embodiments, the invention also provides a therapeutic kit comprising a GIP analogue of the invention, optionally in combination with a pharmaceutically acceptable carrier. In some embodiments, the invention provides a device comprising a GIP analogue of the invention for delivery of the GIP analogue to a subject.

### Pharmaceutical compositions

The GIP-GLP1 dual agonist compounds of the present invention, or salts or solvates thereof, may be formulated as pharmaceutical compositions prepared for storage or administration, which typically comprise a therapeutically effective amount of a compound employed in the context of the invention, or a salt or solvate thereof, in a pharmaceutically acceptable carrier. In some embodiments, the pharmaceutical composition is formulated as a liquid suitable for administration by injection or infusion, or which is formulated to cause slow release of the GIP-GLP1 dual agonist compound.

The therapeutically effective amount of a compound of the present invention will depend, e.g., on the route of administration, the type of mammal being treated, and the physical characteristics of the specific mammal under consideration. These factors and their relationship to determining this amount are well known to skilled practitioners in the medical arts. This amount and the method of administration can be tailored to achieve optimal efficacy, and may depend on such factors as weight, diet, concurrent medication and other factors, well known to those skilled in the medical arts. The dosage sizes and dosing regimen most appropriate for human use may be guided by the results obtained by the present invention, and may be confirmed in properly designed clinical trials.

An effective dosage and treatment protocol may be determined by conventional means, starting with a low dose in laboratory animals and then increasing the dosage while monitoring the effects, and systematically varying the dosage regimen as well. Numerous factors may be taken into consideration by a clinician when determining an optimal dosage for a given subject. Such considerations are known to the skilled person. The term "pharmaceutically acceptable carrier" includes any of the standard pharmaceutical carriers. Pharmaceutically acceptable carriers for therapeutic use are well known in the pharmaceutical art, and are described, for example, in Remington's Pharmaceutical Sciences, Mack Publishing Co. (A. R. Gennaro edit. 1985). For example, sterile saline and phosphate-buffered saline at slightly acidic or physiological pH may be used. Suitable pH buffering agents may be, e.g., phosphate, citrate, acetate, lactate, maleate, tris/hydroxymethyl)aminomethane (TRIS), *N*-Tris(hydroxymethyl)methyl-3-aminopropanesulphonic acid (TAPS), ammonium bicarbonate, diethanolamine, histidine, which in certain embodiments is a preferred buffer, arginine, lysine, or acetate or mixtures thereof. The term further encompasses any agents listed in the US Pharmacopeia for use in animals, including humans.

The term "pharmaceutically acceptable salt" refers to a salt of the compound. Salts include pharmaceutically acceptable salts, such as, e.g., acid addition salts and basic salts. Examples of acid addition salts include hydrochloride salts, citrate salts and acetate salts. Examples of basic salts include salts where the cation is selected from alkali metals, such as sodium and potassium, alkaline earth metals such as calcium, and ammonium ions ⁺N(R³)₃(R⁴), where R³ and R⁴ independently designate optionally substituted C₁₋₆-alkyl, optionally substituted C₂₋₆-alkenyl, optionally substituted aryl, or optionally substituted heteroaryl. Other examples of pharmaceutically acceptable salts are described in "Remington's Pharmaceutical Sciences" ,17th edition. Ed. Alfonso R. Gennaro (Ed.), Mark Publishing Company, Easton, PA, U.S.A., 1985 and more recent editions, and in the Encyclopaedia of Pharmaceutical Technology.

"Treatment" is an approach for obtaining beneficial or desired clinical results. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" may also mean prolonging survival as compared to expected survival if not receiving treatment. "Treatment" is an intervention performed with the intention of preventing the development or altering the pathology of a disorder. Accordingly, "treatment" refers to both therapeutic treatment and prophylactic or preventative measures in certain embodiments. Those in need of treatment include those already with the disorder as well as those in which the disorder is to be prevented. By treatment is meant inhibiting or reducing an increase in pathology or symptoms (e.g. weight gain, hyperglycemia) when compared to the absence of treatment, and is not necessarily meant to imply complete cessation of the relevant condition.

The pharmaceutical compositions of the invention may be in unit dosage form. In such form, the composition is divided into unit doses containing appropriate quantities of the active component. The unit dosage form can be a packaged preparation, the package containing discrete quantities of the preparations, for example, packeted tablets, capsules, and powders in vials or ampoules. The unit dosage form can also be a capsule, cachet, or tablet itself, or it can be the appropriate number of any of these packaged forms. It may be provided in single dose injectable form, for example in the form of an injection pen. Compositions may be formulated for any suitable route and means of administration. Pharmaceutically acceptable carriers or diluents include those used in formulations suitable for oral, rectal, nasal or parenteral (including subcutaneous, intramuscular, intravenous, intradermal, and transdermal) administration. The formulations may conveniently be presented in unit dosage form and may be prepared by any of the methods well known in the art of pharmacy. Subcutaneous or transdermal modes of administration may be particularly suitable for certain of the compounds described herein.

### Combination therapy

In certain embodiments, a GIP-GLP-1 dual agonist compound employed in the context of the invention may be administered as part of a combination therapy with at least one other agent for treatment of diabetes, obesity, dyslipidemia, or hypertension.

In such cases, the at least two active agents may be given together or separately, and as part of the same pharmaceutical formulation or as separate formulations. Thus, the GIP-GLP-1 dual agonist compound employed in the context of the invention (or the salt or solvate thereof) may be used in combination with an antidiabetic agent including but not limited to metformin, a sulfonylurea, a glinide, a DPP-IV inhibitor, a glitazone, or insulin. In certain embodiments, the compound or salt or solvate thereof is used in combination with insulin, DPP-IV inhibitor, sulfonylurea or metformin, particularly sulfonylurea or metformin, for achieving adequate glycemic control. In certain preferred embodiments, the compound or salt or solvate thereof is used in combination with insulin or an insulin analogue for achieving adequate glycemic control. Examples of insulin analogues include but are not limited to Lantus®, NovoRapid®, Humalog®, NovoMix®, Actraphane HM®, Levemir® and Apidra®.

In certain embodiments, the GIP-GLP-1 dual agonist compound or salt or solvate thereof may further be used in combination with one or more of an anti-obesity agent, including but not limited to a glucagon-like peptide receptor 1 agonist, peptide YY or analogue thereof, cannabinoid receptor 1 antagonist, lipase inhibitor, melanocortin receptor 4 agonist, or melanin concentrating hormone receptor 1 antagonist.

In certain embodiments, the GIP-GLP-1 dual agonist compound or salt or solvate thereof may be used in combination with an anti-hypertension agent, including but not limited to an angiotensin-converting enzyme inhibitor, angiotensin II receptor blocker, diuretics, beta-blocker, or calcium channel blocker.

In certain embodiments, the GIP-GLP-1 dual agonist compound or salt thereof may be used in combination with an anti-dyslipidemia agent, including but not limited to a statin, a fibrate, a niacin and/or a cholesterol absorption inhibitor.

### Synthesis of compounds of the invention

A nucleic acid molecule may encode the amino acid sequence of Formula I or a precursor thereof. The amino acid sequence encoded can be regarded as a precursor of a compound of the invention.

Typically, such nucleic acid sequences will be provided as expression constructs wherein the encoding nucleic acid is in functional linkage with appropriate control sequences to direct its expression. The expression construct may be provided in the context of a host cell capable of expressing (and optionally also secreting) the amio acid precursor, or ina cell-free expression system.

A method of producing a GIP analogue of the invention may comprise expressing an amino acid precursor of the GIP analogue and modifying the precursor to provide the GIP analogue. The modification may comprise chemical modification of a Lys, Arg or Cys residue present at position 17 to introduce the lipophilic moiety, modification of the N- or C- terminus, and/or modification of any other amino acid side chains in the molecule (e.g. to introduce a non-naturally occurring amino acid residue).

The compounds of the invention may also be manufactured by standard peptide synthetic methods, e.g. by standard solid-phase or liquid-phase methodology, either stepwise or by fragment assembly, and isolating and purifying the final peptide compound product, or by any combinations of recombinant and synthetic methods.

It may be preferable to synthesize the peptide compounds of the invention by means of solid-phase or liquid-phase peptide synthesis. In this context, reference may be made to WO 98/11125 or, *inter alia,* Fields, G.B. et al., "Principles and Practice of Solid-Phase Peptide Synthesis"; in: Synthetic Peptides, Gregory A. Grant (ed.), Oxford University Press (2nd edition, 2002) and the synthesis examples herein.

### Examples

The following examples demonstrate certain embodiments of the present invention. However, it is to be understood that these examples neither purport nor are they intended to be wholly definitive as to conditions and scope of this invention. The examples were carried out using standard techniques, which are well known and routine to those of skill in the art, except where otherwise described in detail. The following examples are presented for illustrative purposes only, and should not be construed in any way as limiting the scope of this invention.

Disclosed are GIP-GLP1 dual agonist compounds that exhibit signaling selectivity, and methods for screening these compounds. Signaling selectivity may be, for example, preferential pathway activation or preferential pathway inhibition, or both. The GIP-GLP1 dual agonist compounds may be useful for the treatment and/or prevention of diseases or conditions caused or characterized by excess body weight, including, but not limited to, obesity, morbid obesity, obesity linked inflammation, obesity linked gallbladder disease, obesity induced sleep apnea, metabolic syndrome, pre-diabetes, insulin resistance, glucose intolerance, type 2 diabetes, type I diabetes, hypertension, atherogenic dyslipidaemia, atherosclerosis, arteriosclerosis, coronary heart disease, peripheral artery disease, and stroke or microvascular disease.

While some embodiments of the invention have been described by way of illustration, it will be apparent that the invention can be put into practice with many different modifications, variations and adaptations, and with the use of numerous equivalents or alternative solutions that are within the scope of persons skilled in the art, without departing from the claims.

The methods used in the instant invention are described below, except where expressly indicated otherwise

### Example 1

### General synthesis of acylated GIP analogues

Solid phase peptide synthesis was performed on a CEM Liberty Peptide Synthesizer using standard Fmoc chemistry. TentaGel S Ram resin (1 g; 0.25 mmol/g) was swelled in NMP (10 ml) prior to use and transferred between tube and reaction vessel using DCM and NMP.

### Coupling

An Fmoc-amino acid in DMF/DCM (2:1 ; 0.2 M; 5 ml) was added to the resin in a CEM Discover microwave unit together with HATU/DMF or COMU/DMF (0.5 M; 2 ml) and DIPEA-DMF/DCM (2:1) (2.0 M; 1 ml). The coupling mixture was heated to 75°C for 5 min while nitrogen was bubbled through the mixture. The resin was then washed with DMF (4 x 10 ml).

### Deprotection

Piperidine/DMF (20%; 10 ml) was added to the resin for initial deprotection and the mixture was heated by microwaves (30 sec; 40°C). The reaction vessel was drained and a second portion of piperidine/NMP (20%; 10 ml) was added and heated (75°C; 3 min.) again. The resin was then washed with DMF (6 x 10 ml).

### Side chain acylation

Fmoc-Lys(ivDde)-OH or alternatively another amino acid with an orthogonal side chain protective group was introduced at the position of the acylation. The N-terminal of the peptide backbone was then Boc-protected using Boc2O or alternatively by using a Boc-protected amino acid in the last coupling. While the peptide was still attached to the resin, the orthogonal side chain protective group was selectively cleaved using freshly prepared hydrazine hydrate (2-4%) in NMP for 2 x 15 min. The unprotected lysine side chain was first coupled with Fmoc-Glu-OtBu or another spacer amino acid, which was deprotected with piperidine and acylated with a lipophilic moiety using the peptide coupling methodology as described above.

Abbreviations employed are as follows:
COMU: 1-[(1-(cyano-2-ethoxy-2-oxoethylideneaminooxy)-dimethylamino-morpholinomethylene)]methanaminium hexaflourophosphate
ivDde: 1-(4,4-dimethyl-2,6-dioxocyclohexylidene)3-methyl-butyl
Dde: 1-(4,4-dimethyl-2,6-dioxocyclohexylidene)-ethyl
DCM: dichloromethane
DMF: *N,N*-dimethylformamide
DIPEA: diisopropylethylamine
EtOH: ethanol
Et₂O: diethyl ether
HATU: *N*-[(dimethylamino)-1H-1,2,3-triazol[4,5-b]pyridine-1-ylmethylene]-*N-*methylmethanaminium hexafluorophosphate *N*-oxide
MeCN: acetonitrile
NMP: *N*-methylpyrrolidone
TFA: trifluoroacetic acid
TIS: triisopropylsilane

### Cleavage

The resin was washed with EtOH (3 x 10 ml) and Et₂O (3 x 10 ml) and dried to constant weight at room temperature (r.t.). The crude peptide was cleaved from the resin by treatment with TFA/TIS/water (95/2.5/2.5; 40 ml, 2 h; r.t.). Most of the TFA was removed at reduced pressure and the crude peptide was precipitated and washed three times with diethylether and dried to constant weight at room temperature.

### HPLC purification of the crude peptide

The crude peptide was purified to greater than 90% by preparative reverse phase HPLC using a PerSeptive Biosystems VISION Workstation equipped with a C-18 column (5 cm; 10 µm) and a fraction collector and run at 35 ml/min with a gradient of buffer A (0.1% TFA, aq.) and buffer B (0.1% TFA, 90% MeCN, aq.). Fractions were analyzed by analytical HPLC and MS and relevant fractions were pooled and lyophilized. The final product was characterized by HPLC and MS.

The synthesized compounds are shown in Table 1. (Not all of the following are compounds of the invention.)

**Table 1.**

| **Compound No.** | |
|---|---|
| 1 | H-Y-Aib-EGTFTSDYSIYLDK-K([17-carboxy-heptadecanoyl]-isoGlu-Peg3-Peg3)-AQRAFVEWLLAQGPSSGAPPPS-NH₂ |
| 2 | H-Y-Aib-EGTFTSDYSIYLDK-K([19-Carboxy-nonadecanoyl]-isoGlu-Peg3-Peg3)-AQRAFVEWLLAQGPSSGAPPPS-NH₂ |
| 3 | H-Y-Aib-EGTFTSDYSIYLDK-K([17-carboxy-heptadecanoyl]-isoGlu)-AQRAFVEWLLAQGPSSGAPPPS-NH₂ |
| 4 | H-Y-Aib-EGTFTSDYSIYLDK-K([17-carboxy-heptadecanoyl]-isoGlu-GSGSGG)-AQRAFVEWLLAQGPSSGAPPPS-NH₂ |
| 5 | H- Y -Aib-EGTFTSDYSIYLDK- K([17 -carboxy-heptadecanoyl]-Peg3- Peg3)-AQRAFVEWLLAQGPSSGAPPPS-NH₂ |
| 6 | H-Y-Aib-EGTFTSDYSIYLDK-K([17-carboxy-heptadecanoyl]-isoGlu-Peg3-Peg3-Peg3)-AQRAFVEWLLAQGPSSGAPPPS-NH₂ |
| 7 | H-Y-Aib-EGTFTSDYSIYLDK-K([17-carboxy-heptadecanoyl]-Dapa-Peg3-Peg3)-AQRAFVEWLLAQGPSSGAPPPS-NH₂ |
| 8 | H-Y-Aib-EGTFTSDYSIYLDK-K([19-Carboxy-nonadecanoyl]-isoGlu-Peg3-Peg3-Peg3)-AQRAFVEWLLAQGPSSGAPPPS-NH₂ |
| 9 | H-Y-DAIa-EGTFTSDYSIYLDK-K([17-carboxy-heptadecanoyl]-isoGlu-Peg3-Peg3)-AQRAFVEWLLAQGPSSGAPPPS-NH₂ |
| 10 | H-Y-Aib-EGTFTSDYSIYLEK-K([17-carboxy-heptadecanoyl]-isoGlu-Peg3-Peg3)-AAKEFVEWLLSAGPSSGAPPPS-NH₂ |
| 11 | H-Y-Aib-EGTFTSDYSI-Aib-LDK-K([17-carboxy-heptadecanoyl]-isoGlu-Peg3-Peg3)-AQRAFVEWLLAQGPSSGAPPPS-NH₂ |
| 12 | H-Y-Aib-EGTFTSDYSIYLDK-K([17-carboxy-heptadecanoyl]-isoGlu-Peg3-Peg3)-AQRAFVNWLLA-Aib-K-NH₂ |
| 13 | H-Y-Aib-EGTFTSDYSIYLDE-K([17-carboxy-heptadecanoyl]-isoGlu-Peg3-Peg3)-AAKEFIEWLESA-NH₂ |
| 14 | H-Y-Aib-EGTFTSDYSIALDK-K([17-carboxy-heptadecanoyl]-isoGlu-Peg3-Peg3)-AQRAFVNWLVA-Aib-KPSSGAPPPS-NH₂ |
| 15 | H-Y-Aib-EGTFTSDYSIALDK-K[19-carboxy-nonadecanoyl]-isoGlu-Peg3-Peg3)-AQRAFVNWLVA-Aib-KPSSGAPPPS-NH₂ |
| 16 | H-Y-Aib-EGTFTSDYSIALEK-K([17-carboxy-heptadecanoyl]-isoGlu-Peg3-Peg3)-AQRAFVEWLLAQK-NH₂ |
| 17 | H-Y-Aib-EGTFTSDLSIALEK-K([17-carboxy-heptadecanoyl]-isoGlu-Peg3-Peg3)-AQRAFVEWLLAQK-NH₂ |
| 18 | H-Y-Aib-EGTFTSDYSIYLEK-K([17-carboxy-heptadecanoyl]-isoGlu-Peg3-Peg3)-AQRAFVEWLLRA-NH₂ |
| 19 | H-Y-Aib-EGTFTSDYSIALDK-K([17-carboxy-heptadecanoyl]-isoGlu-Peg3-Peg3)-AQRAFVNWLVA-Aib-K-NH₂ |
| 20 | H-Y-Aib-EGTFTSDLSIALDK-K([17-carboxy-heptadecanoyl]-isoGlu-Peg3-Peg3)-AQRAFVNWLVA-Aib-K-NH₂ |
| 21 | H-Y-Aib-EGTFTSDYSIALDK-K([17-carboxy-heptadecanoyl]-isoGlu-Peg3-Peg3)-AQRAFVEWLVA-Aib-K-NH₂ |
| 22 | H-Y-Aib-EGTFTSDYSIALEK-K([17-carboxy-heptadecanoyl]-isoGlu-Peg3-Peg3)-AQRAFVEWLEAQGPSSGAPPPS-NH₂ |
| 23 | H-Y-Aib-EGTFTSDYSIALEK-K([17-carboxy-heptadecanoyl]-isoGlu-Peg3-Peg3)-AQRAFVEWLEAQK-NH₂ |
| 24 | H-Y-Aib-EGTFTSDLSIALEK-K([17-carboxy-heptadecanoyl]-isoGlu-Peg3-Peg3)-AQRAFVEWLEAQK-NH₂ |
| 25 | H-Y-Aib-EGTFTSDYSIALEK-K[19-carboxy-nonadecanoyl]-isoGlu-Peg3-Peg3)-AQRAFVEWLLAQK-NH₂ |
| 26 | H-Y-Aib-EGTFTSDLSIALEK-K[19-carboxy-nonadecanoyl]-isoGlu-Peg3-Peg3)-AQRAFVEWLEAQK-NH₂ |
| 27 | H-Y-Aib-EGTFTSDYSIALEK-K([17-carboxy-heptadecanoyl]-isoGlu-Peg3-Peg3)-AQRAFVEWLEAQGPSSGAPPPS-NH₂ |
| 28 | H-Y-Aib-EGTFTSDLSIALEK-K([19-carboxy-nonadecanoyl]-isoGlu-Peg3-Peg3)-AQRAFVEWLLAQK-NH₂ |
| 29 | H-Y-Aib-EGTFTSDYSIYLEK-K([19-carboxy-nonadecanoyl]-isoGlu-Peg3-Peg3)-AQRAFVEWLLRA-NH₂ |
| 30 | H-Y-DAIa-EGTFTSDYSIYLDK-K([19-carboxy-nonadecanoyl]-isoGlu-Peg3-Peg3)-AQRAFVEWLLAQGPSSGAPPPS-NH₂ |
| 31 | H-Y-Aib-EGTFTSDYSIALEK-K([19-carboxy-nonadecanoyl]-isoGlu-Peg3-Peg3)-AQRAFVEWLLAQGPSSGAPPPS-NH₂ |
| 32 | H-Y-DAIa-EGTFTSDYSIALEK-K([19-carboxy-nonadecanoyl]-isoGlu-Peg3-Peg3)-AQRAFVEWLLAQGPSSGAPPPS-NH₂ |
| 33 | H-Y-Aib-EGTFTSDLSIALEK-K([19-carboxy-nonadecanoyl]-isoGlu-Peg3-Peg3)-AQRAFVEWLLAQGPSSGAPPPS-NH₂ |
| 34 | H-Y-Aib-EGTFTSDLSIALEK-K([19-carboxy-nonadecanoyl]-isoGlu-Peg3-Peg3)-AQRAFVEWLLAQKPSSGAPPPS-NH₂ |
| 35 | H-Y-Aib-EGTFTSDYSIYLDK-K([19-carboxy-nonadecanoyl]-Peg3-Peg3)-AQRAFVEWLLAQGPSSGAPPPS-NH₂ |
| 36 | H-Y-Aib-EGTFTSDYSIYLDK-K([19-carboxy-nonadecanoyl]-lsoGlu)-AQRAFVEWLLAQGPSSGAPPPS-NH₂ |
| 37 | H-Y-Aib-EGTFTSDYSIYLDK-K([19-carboxy-nonadecanoyl]-Dapa-Peg3-Peq3)-AQRAFVEWLLAQGPSSGAPPPS-NH₂ |
| 38 | H-Y-Aib-EGTFTSDYSIALDK-K([19-carboxy-nonadecanoyl]-isoGlu-Peg3-Peg3)-AQRAFVEWLLAQKPSSGAPPPS-NH2 |
| 39 | H-Y-DAIa-EGTFTSDYSIALDK-K([19-carboxy-nonadecanoyl]-isoGlu-Peg3-Peg3)-AQRAFVEWLLAQKPSSGAPPPS-NH₂ |
| 40 | H-Y-Aib-EGTFTSDYSIALDK-K([19-carboxy-nonadecanoyl]-isoGlu-Peg3-Peg3)-AQRAFVEWLLAAPSSGAPPPS-NH₂ |
| 41 | H-Y-Aib-EGTFTSDYSIALDK-K([19-carboxy-nonadecanoyl]-isoGlu-Peg3-Peg3)-AAKEFVEWLLSAGPSSGAPPPS-NH2 |
| 42 | H-Y-Aib-EGTFTSDYSIALDK-K([19-carboxy-nonadecanoyl]-isoGlu-Peg3-Peg3)-AQRAFVEWLLAQGPSSGAPPPS-NH₂ |
| 43 | H-Y-Aib-EGTFTSDYSIALDE-K([19-carboxy-nonadecanoyl]-isoGlu-Peg3-Peg3)-AQRAFVEWLLAQGPSSGAPPPS-NH₂ |
| 44 | H-Y-Aib-EGTFTSDYSIALDE-K([19-carboxy-nonadecanoyl]-isoGlu-Peg3-Peg3)-AQRAFVEWLLAAGPSSGAPPPS-NH₂ |
| 45 | H-Y-Aib-EGTFTSDYSIALDK-K([19-carboxy-nonadecanoyl]-isoGlu-Peg3-Peg3)-AQKAFVEWLLAAGPSSGAPPPS-NH₂ |
| 46 | H-Y-Aib-EGTFTSDYSIYLDK-K([19-carboxy-nonadecanoyl]-isoGlu-Peg3-Peg3)-AQREFVEWLLAQGPSSGAPPPS-NH₂ |
| 47 | H-Y-Aib-EGTFTSDYSIALDK-K([19-carboxy-nonadecanoyl]-isoGlu-Peg3-Peg3)-AQREFVEWLLAQGPSSGAPPPS-NH₂ |
| 48 | H-Y-Aib-EGTFTSDYSIALDK-K([19-carboxy-nonadecanoyl]-isoGlu-Peg3-Peg3)-AQKEFVEWLLAQGPSSGAPPPS-NH₂ |
| 49 | H-Y-Aib-EGTFTSDYSIALDK-K([19-carboxy-nonadecanoyl]-isoGlu-Peg3-Peg3)-AQKEFVEWLLAAGPSSGAPPPS-NH2 |
| 50 | H-Y-Aib-EGTFTSDYSIALDK-K([19-carboxy-nonadecanoyl]-isoGlu-Peg3-Peg3)-AQRAFIEWLLAQGPSSGAPPPS-NH₂ |
| 51 | H-Y-Aib-EGTFTSDYSIALDK-K([17-carboxy-heptadecanoyl]-isoGlu-Peg3-Peg3)-AQRAFVEWLLAQGPSSGAPPPS-NH₂ |
| 52 | H-Y-Aib-EGTFTSDYSIALDK-K((19-Carboxy-nonadecanoyl)-[(Piperazine-1-yl)-acetyl]-Peg3-Peg3)-AQRAFVEWLLAQGPSSGAPPPS-NH₂ |
| 53 | H-Y-Aib-EGTFTSDYSIALDK-K((19-Carboxy-nonadecanoyl)-[(Piperazine-1-yl)-acetyl]-Peg3-Peg3)-AQKEFVEWLLAAGPSSGAPPPS-N H₂ |
| 54 | H-Y-Aib-EGTFTSDYSIYLDK-K([19-Carboxy-nonadecanoyl]-isoGlu-Peg3-Peg3)-AQRAFVEWLLAEGPSSGAPPPS-NH₂ |
| 55 | H-Y-Aib-EGTFTSDYSIALDK-K([19-carboxy-nonadecanoyl]-isoGlu-Peg3-Peg3)-AQRAFVEWLLAEGPSSGAPPPS-NH₂ |
| 56 | H-Y-Aib-EGTFTSDYSIALDK-K([19-carboxy-nonadecanoyl]-isoGlu-Peg3-Peg3)-AQRAFVEWLLAEPSSGAPPPS-NH₂ |

### Synthesis of compound no. 10

Solid phase peptide synthesis was performed on a CEM Liberty Peptide Synthesizer using standard Fmoc chemistry. TentaGel S Ram S resin (1,05 g; 0.25 mmol/g) was swelled in DMF (10 ml) prior to use and transferred between tube and reaction vessel using DCM and DMF.

### Coupling

An Fmoc-amino acid in DMF/DCM (2:1 ; 0.2 M; 5 ml) was added to the resin in a CEM Discover microwave unit together with COMU/DMF (0.5 M; 2 ml) and DIPEA-DMF/DCM (2:1) (2.0 M; 1 ml). The coupling mixture was heated to 75°C for 5 min while nitrogen was bubbled through the mixture. The resin was then washed with DMF (4 x 10 ml). Fmoc-Tyr(OtBu)-Ser(Psi Me,Me)-OH pseudoproline was used for amino acid number 29 and 30 counting from the C-terminal. Lys17 was incorporated as Fmoc-Lys(Dde)-OH for orthogonal coupling. The first 9 amino acids and amino acid number 24 (counting from the C-terminal) was double couple meaning the building block was coupled twice before deprotection. Boc-Tyr(tBu)-OH was incorporated as the final building block in the N-terminal.

### Deprotection

Piperidine/DMF (20%; 10 ml) was added to the resin for initial deprotection and the mixture was heated by microwaves (30 sec; 40°C). The reaction vessel was drained and a second portion of piperidine/DMF (20%; 10 ml) was added and heated (75°C; 3 min.) again. The resin was then washed with DMF (6 x 10 ml).

### Side chain acylation

While the peptide was still attached to the resin, the orthogonal side chain protective group was selectively cleaved using freshly prepared hydrazine hydrate (2-4%) in NMP for 2 x 15 min. The unprotected lysine side chain was first coupled with Fmoc-Glu-OtBu and the two Peg3 buildingblocks using standard coupling and deprotection conditions as explained aboved. Lastly the lipophilic moiety was incporated as a 17-carboxy-heptadecanoic acid mono tert butyl ester again using standard coupling conditions.

### Cleavage

The resin was washed with EtOH (3 x 10 ml) and Et2O (3 x 10 ml) and dried to constant weight at room temperature (r.t.). The crude peptide was cleaved from the resin by treatment with TFA/TIS/H₂O (95/2.5/2.5; 60 ml, 2 h; r.t.). Most of the TFA was removed at reduced pressure and the crude peptide was precipitated and washed three times with diethylether and dried to constant weight at room temperature.

### HPLC purification of the crude peptide

The crude peptide was first purified from 45% by preparative reverse phase HPLC using a PerSeptive Biosystems VISION Workstation equipped with a Gemini NX 5µ C-18 110A, 10x250 mm column and a fraction collector and run at 35 ml/min with a gradient of buffer A (0.1% TFA, aq.) and buffer B (0.1% TFA, 90% MeCN, aq.). Fractions were analyzed by analytical HPLC and MS and relevant fractions were pooled and lyophilized. The product (96 mg) was analysed to give a purity of 91% as characterized by HPLC and MS. Calculated monoisotopic mass = 4921,51, found 4921,45.

### Example 2

### Human GIP receptor (GIP R) and GLP-1 receptor (GLP-1 R) activity assay

*In vitro* effects of peptide conjugates were assessed by measuring the induction of cAMP following stimulation of the respective receptor by GIP, GLP1 or analogues of these, as outlined herein, using the AlphaSceen® cAMP kit from Perkin-Elmer according to instructions. Briefly, HEK293 cells expressing the human GIP R or GLP-1 R (stable cell lines generated through transfection of the cDNA for human GIP R or GLP-1 and selection of stable clones) were seeded at 30,000 cells/well in 96-well microtiter plates coated with 0.01 % poly-L-lysine, and grown for 1 day in culture in 200 µl growth medium (DMEM, 10% FCS, Penicillin (100 IU/ml), Streptomycin (100 µg/ml)). On the day of analysis, growth medium was removed and the cells were washed once with 150 ml Tyrode's buffer (Tyrode's Salts (9.6 g/l), 10 mM HEPES, pH 7.4). Cells were then incubated in 100 ml Assay buffer (0.1% W/V Alkali-treated Casein and 100 µM IBMX in Tyrode's Buffer) containing increasing concentrations of control and test compounds for 15 min at 37° C. The Assay buffer was removed and cells are lysed in 80 µl Lysis buffer (0.1 % w/v BSA, 5 mM HEPES, 0.3 % v/v Tween-20) per well. From each well 10 µl lysed cells was transferred to a 384-well plate and mixed with 15 µl bead-mix (1 Unit/15 µl anti-cAMP Acceptor Beads, 1 Unit/15 µl Donor Beads, and 1 Unit/15 µl Biotinylated cAMP in Assay Buffer). The plates were mixed and incubated in the dark for an hour at room temperature before measuring using an Envision™ plate reader (Perkin-Elmer).

Results were converted into cAMP concentrations using a cAMP standard curve prepared in KRBH buffer containing 0.1% (v/v) DMSO. The resulting cAMP curves were plotted as absolute cAMP concentrations (nM) over log (test compound concentration) and analyzed using the curve fitting program XLfit.

Parameters calculated to describe both the potency as well as the agonistic activity of each test compound on the receptors were:
EC50, a concentration resulting in a half-maximal elevation of cAMP levels, reflecting the potency of the test compound. The results are summarized in Table 2, 2a and 3. The most comprehensive data are summarized in Table 3.

**Table 2: EC₅₀ average values of the compounds on the GIP-R and GLP1-R compared to control peptides.**

| **Compound** | **hGIP-R cAMP (nM)** | **hGLP1- R cAMP (nM)** |
|---|---|---|
| hGIP | 0.003 | NA |
| hGLP-1 | >10 | >10 |
| 1 | 0.0055 | 0.012 |
| 2 | 0.0049 | 0.0083 |
| 3 | 0.0086 | 0.011 |
| 4 | 0.0087 | 0.010 |
| 5 | 0.012 | 0.014 |
| 6 | 0.0049 | 0.016 |
| 7 | 0.011 | 0.012 |
| 8 | 0.0053 | 0.012 |
| 9 | 0.0088 | 0.038 |
| 10 | 0.016 | 0.028 |
| 11 | 0.0047 | 0.013 |
| 12 | 0.011 | 0.016 |
| 14 | 0.0064* | 0.0080* |
| 15 | 0.0080 | 0.0088 |
| 16 | 0.0081 | 0.015 |
| 17 | 0.011 | 0.0062 |
| 18 | 0.014 | 0.022 |
| 19 | 0.0073 | 0.014 |
| 20 | 0.0097 | 0.011 |
| 21 | 0.0068 | 0.011 |
| 22 | 0.06 | 0.061 |
| 23 | 0.2 | 0.044 |
| 24 | 0.26 | 0.011 |
| 25 | 0.009 | 0.011 |
| 26 | NT | 0.014 |
| 27 | 0.06 | 0.061 |
| 28 | 0.024 | 0.017 |
| 29 | 0.025 | 0.033 |
| 30 | 0.015 | 0.028 |
| 31 | 0.007 | 0.016 |
| 32 | 0.016 | 0.040 |
| 33 | 0.009 | 0.014 |
| 34 | 0.012 | 0.015 |

| | | |
|---|---|---|
| NT: Not Tested * These EC50 values (hGIP-R and GLP1-R) for compound 14 are believed to be in error. | | |

**Table 2a: A secondary assay study with EC₅₀ average values of the compounds 1 to 21 on the GIP-R and GLP1-R.**

| **Compound** | **hGIP-R cAMP (nM)** | **hGLP1- R cAMP (nM)** |
|---|---|---|
| 1 | 0.006 | 0.015 |
| 2 | 0.008 | 0.011 |
| 3 | 0.009 | 0.011 |
| 4 | 0.009 | 0.010 |
| 5 | 0.011 | 0.014 |
| 6 | 0.005 | 0.016 |
| 7 | 0.011 | 0.012 |
| 8 | 0.005 | 0.012 |
| 9 | 0.009 | 0.039 |
| 10 | 0.016 | 0.029 |
| 11 | 0.005 | 0.013 |
| 12 | 0.012 | 0.017 |
| 14 | 0.147 | 0.056 |
| 15 | 0.006 | 0.008 |
| 16 | 0.008 | 0.009 |
| 17 | 0.008 | 0.015 |
| 18 | 0.011 | 0.006 |
| 19 | 0.014 | 0.022 |
| 20 | 0.007 | 0.014 |
| 21 | 0.010 | 0.010 |

**Table 3: EC₅₀ average values of all the compounds on the GIP-R and GLP1-R.**

| Compound | hGIP-R cAMP (nM) | hGLP-1-R cAMP (nM) |
|---|---|---|
| 1 | 0.006 | 0.015 |
| 2 | 0.008 | 0.011 |
| 3 | 0.009 | 0.011 |
| 4 | 0.009 | 0.010 |
| 5 | 0.012 | 0.014 |
| 6 | 0.005 | 0.016 |
| 7 | 0.011 | 0.012 |
| 8 | 0.005 | 0.012 |
| 9 | 0.009 | 0.038 |
| 10 | 0.016 | 0.028 |
| 11 | 0.005 | 0.013 |
| 12 | 0.011 | 0.016 |
| 13 | 0.150 | 0.057 |
| 14 | 0.006 | 0.008 |
| 15 | 0.008 | 0.009 |
| 16 | 0.008 | 0.015 |
| 17 | 0.011 | 0.006 |
| 18 | 0.014 | 0.022 |
| 19 | 0.007 | 0.014 |
| 20 | 0.010 | 0.011 |
| 21 | 0.007 | 0.011 |
| 22 | 0.060 | 0.061 |
| 23 | 0.200 | 0.044 |
| 24 | 0.260 | 0.011 |
| 25 | 0.009 | 0.011 |
| 26 | 0.580 | 0.014 |
| 27 | 0.060 | 0.061 |
| 28 | 0.024 | 0.015 |
| 29 | 0.025 | 0.033 |
| 30 | 0.017 | 0.035 |
| 31 | 0.008 | 0.017 |
| 32 | 0.018 | 0.056 |
| 33 | 0.009 | 0.014 |
| 34 | 0.012 | 0.015 |
| 35 | 0.016 | 0.012 |
| 36 | 0.010 | 0.015 |
| 37 | 0.017 | 0.014 |
| 38 | 0.005 | 0.012 |
| 39 | 0.011 | 0.006 |
| 40 | 0.023 | 0.021 |
| 41 | 0.015 | 0.038 |
| 42 | 0.009 | 0.017 |
| 43 | 0.005 | 0.016 |
| 44 | 0.007 | 0.015 |
| 45 | 0.005 | 0.024 |
| 46 | 0.006 | 0.010 |
| 47 | 0.005 | 0.016 |
| 48 | 0.006 | 0.052 |
| 49 | 0.009 | 0.053 |
| 50 | 0.007 | 0.015 |
| 51 | 0.005 | 0.017 |
| 52 | 0.010 | 0.012 |
| 53 | 0.006 | 0.014 |
| 54 | 0.016 | 0.012 |
| 55 | 0.016 | 0.013 |
| 56 | 0.025 | 0.033 |

### Example 3

### Pharmacokinetics of selected compounds in mice

### Method

C57BL/6J mice (males with a body weight of approximately 25 g) were given either a single subcutaneous (s.c.) bolus or a single intravenous (*i.v.*) bolus of each peptide to be tested.

Following s.c. administration of the selected compounds (50, 100 or 200 nmol/kg), blood samples were drawn at 8 (eight) timepoints up to 72 hours post-dose. Following *i.v.* administration of the selected compounds (50, 100 or 200 nmol/kg), blood samples were drawn at 8 (eight) timepoints up to 48 hours post-dose. Blood samples were drawn by sublingual bleeding. The dosing vehicle was a phosphate buffer containing mannitol (pH 7.5).

At each sampling time point, samples from two mice were drawn, i.e. 16 mice were included for each compound and each administration route. The mice were euthanized immediately after blood sampling by cervical dislocation. Plasma samples were analyzed after solid phase extraction (SPE) or protein precipitation followed by liquid chromatography mass spectrometry (LC-MS/MS). Mean plasma concentrations were used for calculation of the pharmacokinetic parameters using the non-compartmental approach in Phoenix WinNonlin 6.3. Plasma terminal elimination half-life (T½) was determined as ln(2)/λz where λz is the magnitude of the slope of the log linear regression of the log concentration versus time profile during the terminal phase. Bioavailability was determined as AUC_{inf} (s.c.) / AUC_{inf} (*i.v*.) × 100, where AUC_{inf} is the area under the plasma concentration - time curve extrapolated to infinity (AUC_{inf} = AUCₗₐₛₜ + Clast/λZ, where Cₗₐₛₜ is the last observed plasma concentration). Tₘₐₓ is the post-dose time where the maximal plasma concentration was observed. The results are summarized in Table 4.

**Table 4. Terminal elimination half-life (h) and bioavailability in mice following s.c. and i.v. administration of selected compounds.**

| **Compound** | **T½ (h.)** | | **Tmax (h.)** | **Bioavailability** |
|---|---|---|---|---|
| | *i.v.* | *s.c*. | *s.c*. | *s.c.* |
| Semaglutide | 7.8 | 7.5 | 4 | 100% |
| 1 | 10.8 | 9.1 | 6 | 90% |
| 2 | 16.6 | 16.5 | 6 | 83% |
| 3 | 9.1 | - | 8 | 89% |
| 4 | 8.7 | 10.8 | 6 | 100%* |
| 15 | 17.3 | - | 8 | 95% |
| 16 | 7.4 | - | 6 | 50% |
| 8 | 17.2 | - | 8 | 54% |
| 28 | 13.6 | 14.1 | 2 | 93% |
| 30 | 17.1 | 15.9 | 8 | 83% |
| 31 | 11.7 | 15.1 | 8 | 88% |
| 32 | 17.4 | 11.8 | 8 | 100%* |
| 34 | 14.9 | 17.1 | 8 | 79% |

| | | | | |
|---|---|---|---|---|
| -: Parameter not calculated *: The bioavailability was capped to 100% | | | | |

### Example 4

### IPGTT (Intraperitoneal Glucose Tolerance Test) in diabetic db/db mice.

Male diabetic *db*/*db* (BKS.Cg-Dock7^{m}+/+Lepr^{db}J) mice (Charles River, France), were maintained on normal chow (Altromin 1324, Brogaarden A/S, Gentofte, Denmark) and domestic quality water with added citric acid to pH ∼ 3.6. The animals were housed in groups of n = 4 in a light-, temperature-, and humidity-controlled room (12:12 h light-dark cycle, with lights on at 06.00-18.00 hr; 23 ± 0.5°C; 50-80% relative humidity). Mice, 11-12 weeks old, were fasted for 5 hr before the IPGTT. GIP-GLP-1 dual acting receptor agonists (0.5 and 5 nmol/kg) and vehicle were administered subcutaneously (s.c.) 22 hours before the intraperitoneal (i.p.) injection of glucose (t = 0 min; 1 g/kg; 5 ml/kg). The GLP-1 analogue liraglutide (10 nmol/kg) was administered subcutaneously 4 hours before the i.p. injection of glucose. Tail vein blood was sampled at time t = 0 (before glucose administration), 15, 30, 60, 120, and 180 min for measurements of blood glucose. Results are shown in Figure 1.

### Example 5

### Sub-chronic effects of GIP-GLP-1 receptor dual acting agonists on body weight, food intake, glucose tolerance and fasted blood glucose in diet-induced obese (DIO) C57BL/6J mice

Male C57BL/6J mice (Taconic A/S, Denmark) fed high-fat diet (60% of total energy from fat, D12492, Research Diet Inc.) for approximately 6 months were used. The animals were housed in groups of n = 3-4 in a light-, temperature-, and humidity-controlled room (12:12 h light-dark cycle, with lights on at 06.00-18.00 hr; 20-22°C; 50-80% relative humidity). Mice from cages of 4 were split into two cages (2 mice per cage) two weeks prior to start of the mock phase. All mice were mock-treated (once daily s.c. injection of vehicle) for a week to acclimatize the animals to handling and injections. Subsequently, the mice were stratified according to body weight into 6 groups (n = 7-9). The average starting body weight was 45-46 grams. Animals were thereafter treated once every third day with s.c. injections (5 ml/kg) of vehicle (25 mM phosphate, 125 mM sodium chloride buffer, pH 7.4), or GIP-GLP-1 dual acting receptor agonists (3 nmol/kg). First day of dosing was on day 0 and last day of dosing on day 18. The daily injections were given in the morning (at 8.00-9.00). Body weight was determined daily throughout the study. Food and water intake per cage were measured every third day of the study (in conjunction with dosing). On day 12, animals were fasted for 5 hours and an oral glucose tolerance test (OGTT) was performed. The animals were dosed in the morning 5 hour before the oral gavage of glucose (t = 0 min; 2 g/kg; 5 ml/kg). Tail vein blood was sampled at time t = 0 (before glucose administration), 15, 30, 60, 120, and 180 min for measurements of blood glucose. On day 18, animals were fasted for 5 hours, and blood samples were taken for measurements of blood glucose. The animals were dosed in the morning 5 hour before the blood sampling. After the final blood sampling, the mice were euthanized. Results are shown in Figure 2 - 5.

Statistical analyses were performed using Graph Pad Prism version 5. The measured parameters were compared using one-way or two-way ANOVAs followed by Dunnett's Multiple Comparison Tests vs. vehicle group or by Bonferroni post tests vs. vehicle group, respectively. Differences were considered statistically significant at p < 0.05. Statistical differences vs vehicle: *p<0.05, **p<0.01, ***p<0.001.

## Claims

1. A GIP analogue having the general Formula I: 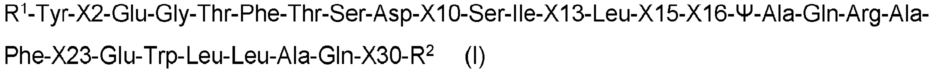 wherein
R¹ is H, C₁₋₄ alkyl, acetyl, formyl, benzoyl, trifluoroacetyl or pGlu;
X2 is selected from Aib and D-Ala;
X10 is selected from Tyr and Leu;
X13 is selected from Ala, Tyr and Aib;
X15 is selected from Asp and Glu;
X16 is selected from Glu and Lys;
X23 is selected from Val and Ile;
X30 is selected from Lys, Gly and Y1, or is absent;
and positions 1 to 29 of the GIP analogue have a maximum of 4 changes compared to the sequence Y-Aib-EGTFTSDYSIYLDKKAQRAFVEWLLAQ;
Y1 (when present) is selected from Gly-Pro-Ser-Ser-Gly-Ala-Pro-Pro-Pro-Ser, Lys-Pro-Ser-Ser-Gly-Ala-Pro-Pro-Pro-Ser, Gly-Pro-Ser-Ser-Gly-Ala-Pro-Pro-Ser, Pro-Ser-Ser-Gly-Ala-Pro-Pro-Pro-Ser and Pro-Ser-Ser-Gly-Ala-Pro-Pro-Ser;
Ψ is a residue of Lys, Arg, Orn or Cys in which the side chain is conjugated to a substituent having the formula -Z¹ or-Z²-Z¹; where:
-Z¹ is a fatty chain having a polar group at one end of the chain and a connection to Ψ or Z²,
-X- at the end of the chain distal from the polar group,
wherein the polar group comprises a carboxylic acid or a carboxylic acid bioisostere, a phosphonic acid, or a sulfonic acid group;
and -X- is a bond, -CO-, -SO-, or -SO₂-;
-Z²- (if present) is a spacer of formula: 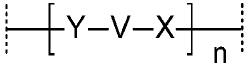 connecting Z¹ to Ψ;
wherein:
each Y is independently -NH, -NR, -S or -O, where R is alkyl, a protecting group or forms a linkage to another part of the spacer Z²;
each X is independently a bond, CO-, SO-, or SO₂-;
with the proviso that when Y is -S, the X to which it is bound is a bond;
each V is independently a bivalent organic moiety linking Y and X;
and n is 1-10;
and
R² is -NH₂ or-OH;
or a pharmaceutically acceptable salt or solvate thereof;
wherein said GIP analogue has agonist activity at the GIP and GLP-1 receptors.

2. A GIP analogue according to claim 1 further comprising the residue Glu15.

3. A GIP analogue according to claim 1 or claim 2 possessing one of the following combinations of residues at the positions between 1 and 29:
Aib2, Tyr10, Tyr13, Asp15, Lys16, Val23;
D-Ala2, Tyr10, Tyr13, Asp15, Lys16, Val23;
Aib2, Tyr10, Aib13, Asp15, Lys16, Val23;
Aib2, Tyr10, Ala13, Glu15, Lys16, Val23;
Aib2, Leu10, Ala13, Glu15, Lys16, Val23;
Aib2, Tyr10, Tyr13, Glu15, Lys16, Val23;
D-Ala2, Tyr10, Ala13, Asp15, Lys16, Val23,;
Aib2, Tyr10, Ala13, Asp15, Lys16, Val23;
Aib2, Tyr10, Tyr13, Asp15, Lys16, Ile23;
D-Ala2, Tyr10, Tyr13, Asp15, Lys16, Ile23;
Aib2, Tyr10, Aib13, Asp15, Lys16, Ile23;
Aib2, Tyr10, Ala13, Glu15, Lys16, Ile23;
Aib2, Leu10, Ala13, Glu15, Lys16, Ile23.

4. A GIP analogue according to claim 1 wherein:
(a) residues 1-29 of Formula I have the sequence:
Y-Aib-EGTFTSDYSIYLDKΨAQRAFVEWLLAQ;
Y-DAIa-EGTFTSDYSIYLDKΨAQRAFVEWLLAQ;
Y-Aib-EGTFTSDYSI-Aib-LDKΨAQRAFVEWLLAQ;
Y-Aib-EGTFTSDYSIALEKΨAQRAFVEWLLAQ;
Y-Aib-EGTFTSDLSIALEKΨAQRAFVEWLLAQ;
Y-Aib-EGTFTSDYSIALEKΨAQRAFVEWLLAQ;
Y-DAla-EGTFTSDYSIALEKΨAQRAFVEWLLAQ;
Y-Aib-EGTFTSDLSIALEKΨAQRAFVEWLLAQ;
Y-Aib-EGTFTSDYSIALDKΨAQRAFVEWLLAQ;
Y-DAla-EGTFTSDYSIALDKΨAQRAFVEWLLAQ;
Y-Aib-EGTFTSDYSIALDEΨAQRAFVEWLLAQ;
Y-Aib-EGTFTSDYSIALDKΨAQRAFIEWLLAQ-; or
Y-Aib-EGTFTSDYSIALDKΨAQRAFVEWLLAQ; or
(b) the peptide backbone (Formula I) has the sequence:
Y-Aib-EGTFTSDYSIYLDKΨAQRAFVEWLLAQGPSSGAPPPS;
Y-DAIa-EGTFTSDYSIYLDKΨAQRAFVEWLLAQGPSSGAPPPS;
Y-Aib-EGTFTSDYSI-Aib-LDKΨAQRAFVEWLLAQGPSSGAPPPS;
Y-Aib-EGTFTSDYSIALEKΨAQRAFVEWLLAQK;
Y-Aib-EGTFTSDLSIALEKΨAQRAFVEWLLAQK;
Y-Aib-EGTFTSDYSIALEKΨAQRAFVEWLLAQGPSSGAPPPS;
Y-DAIa-EGTFTSDYSIALEKΨAQRAFVEWLLAQGPSSGAPPPS;
Y-Aib-EGTFTSDLSIALEKΨAQRAFVEWLLAQKPSSGAPPPS;
Y-Aib-EGTFTSDYSIALDKΨAQRAFVEWLLAQKPSSGAPPPS;
Y-DAIa-EGTFTSDYSIALDKΨAQRAFVEWLLAQKPSSGAPPPS;
Y-Aib-EGTFTSDYSIALDKΨAQRAFVEWLLAQGPSSGAPPPS;
Y-Aib-EGTFTSDYSIALDEΨAQRAFVEWLLAQGPSSGAPPPS;
Y-Aib-EGTFTSDYSIALDKΨAQRAFIEWLLAQGPSSGAPPPS-;
Y-Aib-EGTFTSDYSIALDKΨAQRAFVEWLLAQGPSSGAPPP;
Y-Aib-EGTFTSDYSIALDKΨAQRAFVEWLLAQGPSSGAPPPS; or
Y-Aib-EGTFTSDLSIALEKΨAQRAFVEWLLAQGPSSGAPPPS.

5. A GIP analogue according to any one of the preceding claims wherein Ψ is a residue of Lys in which the side chain is conjugated to a substituent having the formula -Z¹ or -Z²-Z¹.

6. A GIP analogue according to claim 5 wherein Z¹ is HOOC-B-C₁₆₋₂₂alkylene-(CO)-, wherein B is a bond.

7. A GIP analogue according to claim 5 or claim 6 wherein Z¹ is:
17 carboxyheptadecanoyl [HOOC-(CH₂)₁₆-(CO)-];
19-carboxynonadecanoyl [HOOC-(CH₂)₁₈-(CO)-]; or
21-carboxyhenicosanoyl [HOOC-(CH₂)₂₀-(CO)-].

8. A GIP analogue according to any one of claims 5 to 7 wherein Z² comprises a residue of isoGlu, Dapa, or Peg3.

9. A GIP analogue according to any one of claims 5 to 8 wherein-Z²-Z¹ is:
(i) [17-Carboxy-heptadecanoyl]-isoGlu-Peg3-Peg3 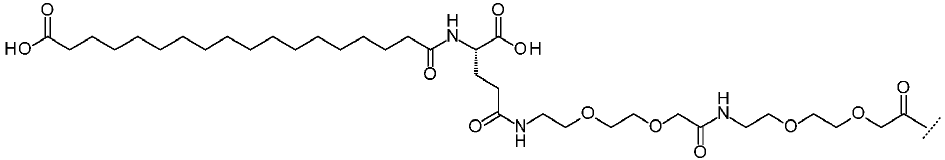
(ii) [17-Carboxy-heptadecanoyl]-isoGlu 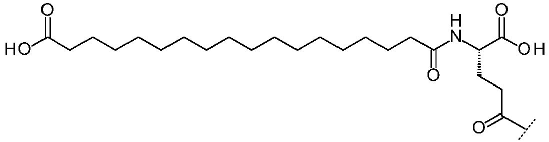
(iii) [17-Carboxy-heptadecanoyl]-Peg3-Peg3-isoGlu 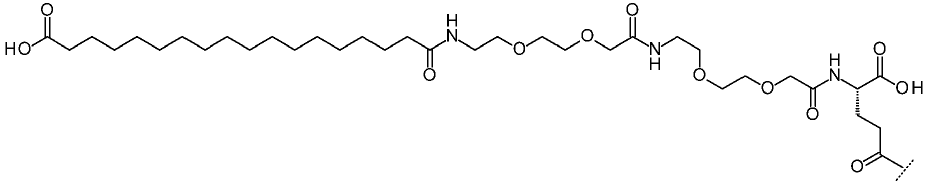
(iv) [17-Carboxy-heptadecanoyl]-isoGlu-GSGSGG 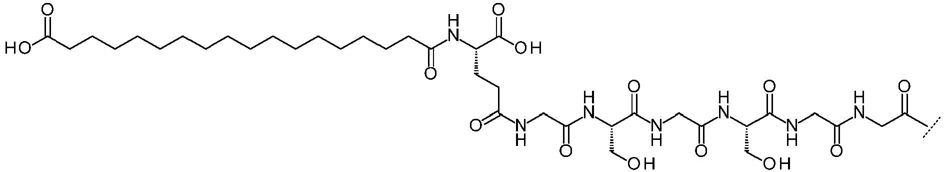
(v) [17-Carboxy-heptadecanoyl]-AA-Peg3-Peg3 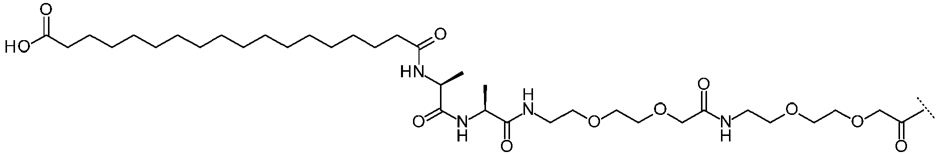
(vi) ([19-Carboxy-nonadecanoyl]-AA-Peg3-Peg3-Peg3) 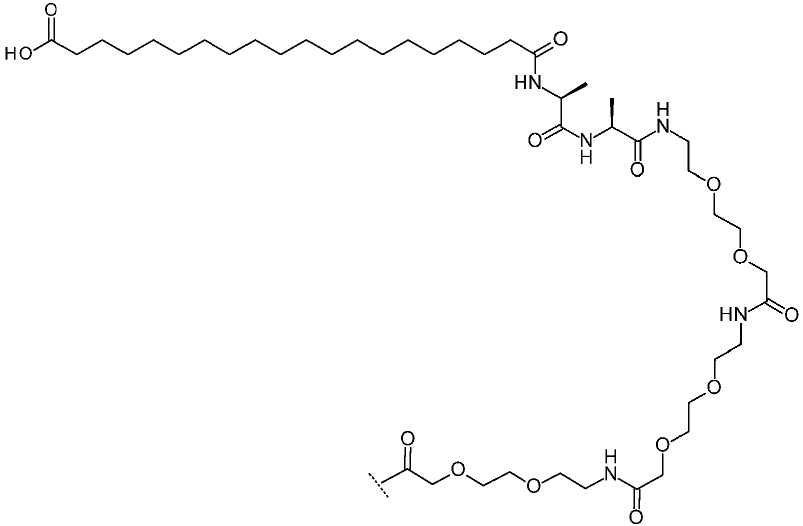
(vii) [17-Carboxy-heptadecanoyl]-Peg3-Peg3 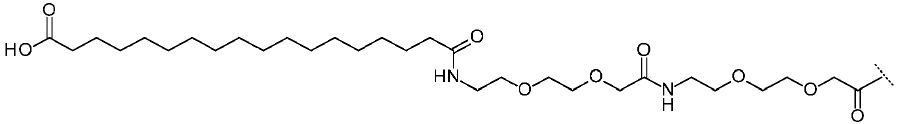
(viii) [17-Carboxy-heptadecanoyl]-isoGlu-Peg3-Peg3-Peg3 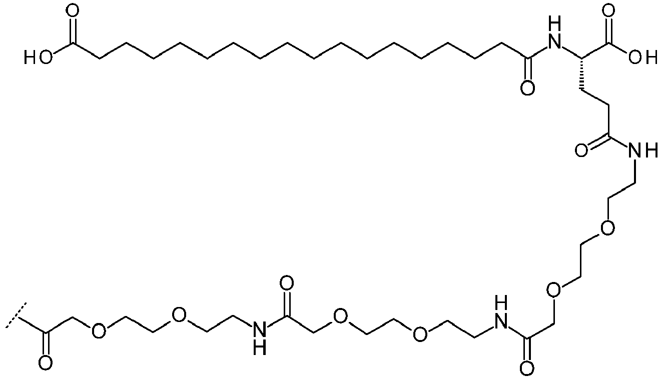
(ix) [17-Carboxy-heptadecanoyl]-Dapa-Peg3-Peg3 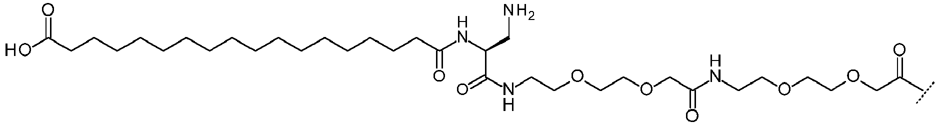
(x) [19-Carboxy-heptadecanoyl]-isoGlu-Peg3-Peg3-Peg3 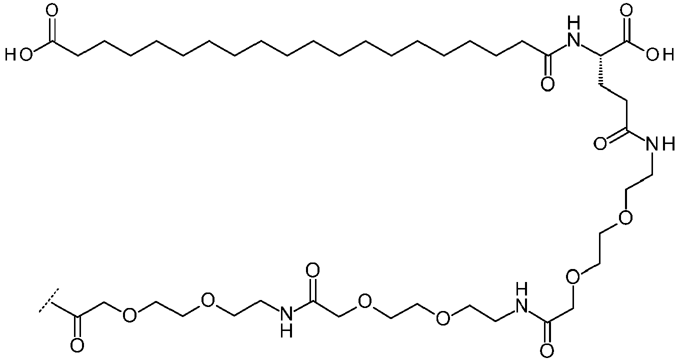
(xi) [19-Carboxy-nonadecanoyl]-isoGlu-Peg3-Peg3 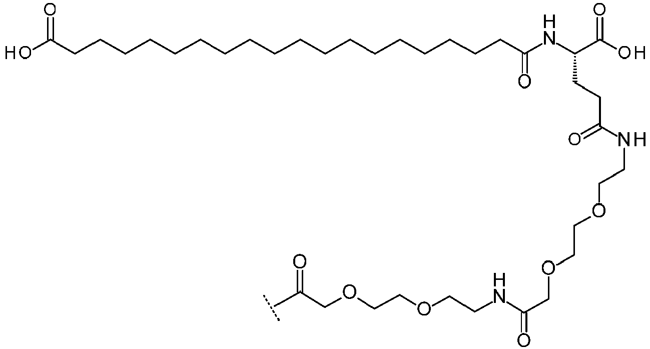
(xii) (19-Carboxy-nonadecanoyl)-[(Piperazine-1-yl)-acetyl]-Peg3-Peg3 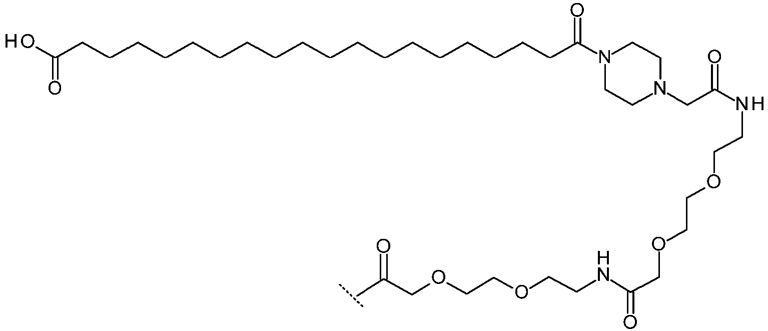 or
(xiii) (19-Carboxy-nonadecanoyl)-[(Piperazine-1-yl)-acetyl]-Peg6

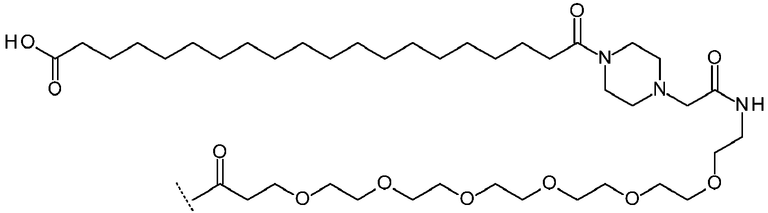

10. A GIP analogue according to claim 1 wherein:
(a) residues 1-29 of Formula I have the sequence:
Y-Aib-EGTFTSDYSIYLDK-K([17-carboxy-heptadecanoyl]-isoGlu-Peg3-Peg3)-AQRAFVEWLLAQ;
Y-Aib-EGTFTSDYSIYLDK-K([19-Carboxy-nonadecanoyl]-isoGlu-Peg3-Peg3)-AQRAFVEWLLAQ;
Y-Aib-EGTFTSDYSIYLDK-K([17-carboxy-heptadecanoyl]-isoGlu)-AQRAFVEWLLAQ;
H-Y-Aib-EGTFTSDYSIYLDK-K([17-carboxy-heptadecanoyl]-isoGlu-GSGSGG)-AQRAFVEWLLAQ;
Y-Aib-EGTFTSDYSIYLDK-K([17-carboxy-heptadecanoyl]-Peg3-Peg3)-AQRAFVEWLLAQ;
Y-Aib-EGTFTSDYSIYLDK-K([17-carboxy-heptadecanoyl]-isoGlu-Peg3-Peg3-Peg3)-AQRAFVEWLLAQ;
Y-Aib-EGTFTSDYSIYLDK-K([17-carboxy-heptadecanoyl]-Dapa-Peg3-Peg3)-AQRAFVEWLLAQ;
Y-Aib-EGTFTSDYSIYLDK-K([19-Carboxy-nonadecanoyl]-isoGlu-Peg3-Peg3-Peg3)-AQRAFVEWLLAQ;
Y-DAla-EGTFTSDYSIYLDK-K([17-carboxy-heptadecanoyl]-isoGlu-Peg3-Peg3)-AQRAFVEWLLAQ;
Y-Aib-EGTFTSDYSI-Aib-LDK-K([17-carboxy-heptadecanoyl]-isoGlu-Peg3-Peg3)-AQRAFVEWLLAQ;
Y-Aib-EGTFTSDYSIALEK-K([17-carboxy-heptadecanoyl]-isoGlu-Peg3-Peg3)-AQRAFVEWLLAQ;
Y-Aib-EGTFTSDLSIALEK-K([17-carboxy-heptadecanoyl]-isoGlu-Peg3-Peg3)-AQRAFVEWLLAQ;
Y-Aib-EGTFTSDYSIALEK-K[19-carboxy-nonadecanoyl]-isoGlu-Peg3-Peg3)-AQRAFVEWLLAQ;
Y-Aib-EGTFTSDLSIALEK-K([19-carboxy-nonadecanoyl]-isoGlu-Peg3-Peg3)-AQRAFVEWLLAQ;
Y-DAla-EGTFTSDYSIYLDK-K([19-carboxy-nonadecanoyl]-isoGlu-Peg3-Peg3)-AQRAFVEWLLAQ;
Y-Aib-EGTFTSDYSIALEK-K([19-carboxy-nonadecanoyl]-isoGlu-Peg3-Peg3)-AQRAFVEWLLAQ;
Y-DAla-EGTFTSDYSIALEK-K([19-carboxy-nonadecanoyl]-isoGlu-Peg3-Peg3)-AQRAFVEWLLAQ;
Y-Aib-EGTFTSDLSIALEK-K([19-carboxy-nonadecanoyl]-isoGlu-Peg3-Peg3)-AQRAFVEWLLAQ;
Y-Aib-EGTFTSDYSIYLDK-K([19-carboxy-nonadecanoyl]-Peg3-Peg3)-AQRAFVEWLLAQ;
Y-Aib-EGTFTSDYSIYLDK-K([19-carboxy-nonadecanoyl]-IsoGlu)-AQRAFVEWLLAQ;
Y-Aib-EGTFTSDYSIYLDK-K([19-carboxy-nonadecanoyl]-Dapa-Peg3-Peg3)-AQRAFVEWLLAQ;
Y-Aib-EGTFTSDYSIALDK-K([19-carboxy-nonadecanoyl]-isoGlu-Peg3-Peg3)-AQRAFVEWLLAQ;
Y-DAla-EGTFTSDYSIALDK-K([19-carboxy-nonadecanoyl]-isoGlu-Peg3-Peg3)-AQRAFVEWLLAQ;
Y-Aib-EGTFTSDYSIALDE-K([19-carboxy-nonadecanoyl]-isoGlu-Peg3-Peg3)-AQRAFVEWLLAQ;
Y-Aib-EGTFTSDYSIALDK-K([19-carboxy-nonadecanoyl]-isoGlu-Peg3-Peg3)-AQRAFIEWLLAQ;
Y-Aib-EGTFTSDYSIALDK-K([17-carboxy-heptadecanoyl]-isoGlu-Peg3-Peg3)-AQRAFVEWLLAQ; or
Y-Aib-EGTFTSDYSIALDK-K((19-Carboxy-nonadecanoyl)-[(Piperazine-1-yl)-acetyl]-Peg3-Peg3)-AQRAFVEWLLAQ; or
(b) the peptide backbone (Formula I) has the sequence:
Y-Aib-EGTFTSDYSIYLDK-K([17-carboxy-heptadecanoyl]-isoGlu-Peg3-Peg3)-AQRAFVEWLLAQGPSSGAPPPS;
Y-Aib-EGTFTSDYSIYLDK-K([19-Carboxy-nonadecanoyl]-isoGlu-Peg3-Peg3)-AQRAFVEWLLAQGPSSGAPPPS;
Y-Aib-EGTFTSDYSIYLDK-K([17-carboxy-heptadecanoyl]-isoGlu)-AQRAFVEWLLAQGPSSGAPPPS;
H-Y-Aib-EGTFTSDYSIYLDK-K([17-carboxy-heptadecanoyl]-isoGlu-GSGSGG)-AQRAFVEWLLAQGPSSGAPPPS;
Y-Aib-EGTFTSDYSIYLDK-K([17-carboxy-heptadecanoyl]-Peg3-Peg3)-AQRAFVEWLLAQGPSSGAPPPS;
Y-Aib-EGTFTSDYSIYLDK-K([17-carboxy-heptadecanoyl]-isoGlu-Peg3-Peg3-Peg3)-AQRAFVEWLLAQGPSSGAPPPS;
Y-Aib-EGTFTSDYSIYLDK-K([17-carboxy-heptadecanoyl]-Dapa-Peg3-Peg3)-AQRAFVEWLLAQGPSSGAPPPS;
Y-Aib-EGTFTSDYSIYLDK-K([19-Carboxy-nonadecanoyl]-isoGlu-Peg3-Peg3-Peg3)-AQRAFVEWLLAQGPSSGAPPPS;
Y-DAla-EGTFTSDYSIYLDK-K([17-carboxy-heptadecanoyl]-isoGlu-Peg3-Peg3)-AQRAFVEWLLAQGPSSGAPPPS;
Y-Aib-EGTFTSDYSI-Aib-LDK-K([17-carboxy-heptadecanoyl]-isoGlu-Peg3-Peg3)-AQRAFVEWLLAQGPSSGAPPPS;
Y-Aib-EGTFTSDYSIALEK-K([17-carboxy-heptadecanoyl]-isoGlu-Peg3-Peg3)-AQRAFVEWLLAQK;
Y-Aib-EGTFTSDLSIALEK-K([17-carboxy-heptadecanoyl]-isoGlu-Peg3-Peg3)-AQRAFVEWLLAQK;
Y-Aib-EGTFTSDYSIALEK-K[19-carboxy-nonadecanoyl]-isoGlu-Peg3-Peg3)-AQRAFVEWLLAQK;
Y-Aib-EGTFTSDLSIALEK-K([19-carboxy-nonadecanoyl]-isoGlu-Peg3-Peg3)-AQRAFVEWLLAQK;
Y-DAIa-EGTFTSDYSIYLDK-K([19-carboxy-nonadecanoyl]-isoGlu-Peg3-Peg3)-AQRAFVEWLLAQGPSSGAPPPS;
Y-Aib-EGTFTSDYSIALEK-K([19-carboxy-nonadecanoyl]-isoGlu-Peg3-Peg3)-AQRAFVEWLLAQGPSSGAPPPS;
Y-DAla-EGTFTSDYSIALEK-K([19-carboxy-nonadecanoyl]-isoGlu-Peg3-Peg3)-AQRAFVEWLLAQGPSSGAPPPS;
Y-Aib-EGTFTSDLSIALEK-K([19-carboxy-nonadecanoyl]-isoGlu-Peg3-Peg3)-AQRAFVEWLLAQKPSSGAPPPS;
Y-Aib-EGTFTSDYSIYLDK-K([19-carboxy-nonadecanoyl]-Peg3-Peg3)-AQRAFVEWLLAQGPSSGAPPPS;
Y-Aib-EGTFTSDYSIYLDK-K([19-carboxy-nonadecanoyl]-IsoGlu)-AQRAFVEWLLAQGPSSGAPPPS;
Y-Aib-EGTFTSDYSIYLDK-K([19-carboxy-nonadecanoyl]-Dapa-Peg3-Peg3)-AQRAFVEWLLAQGPSSGAPPPS;
Y-Aib-EGTFTSDLSIALEK-K([19-carboxy-nonadecanoyl]-isoGlu-Peg3-Peg3)-AQRAFVEWLLAQGPSSGAPPPS
Y-Aib-EGTFTSDYSIALDK-K([19-carboxy-nonadecanoyl]-isoGlu-Peg3-Peg3)-AQRAFVEWLLAQKPSSGAPPPS;
Y-DAla-EGTFTSDYSIALDK-K([19-carboxy-nonadecanoyl]-isoGlu-Peg3-Peg3)-AQRAFVEWLLAQKPSSGAPPPS;
Y-Aib-EGTFTSDYSIALDK-K([19-carboxy-nonadecanoyl]-isoGlu-Peg3-Peg3)-AQRAFVEWLLAQGPSSGAPPPS;
Y-Aib-EGTFTSDYSIALDE-K([19-carboxy-nonadecanoyl]-isoGlu-Peg3-Peg3)-AQRAFVEWLLAQGPSSGAPPPS;
Y-Aib-EGTFTSDYSIALDK-K([19-carboxy-nonadecanoyl]-isoGlu-Peg3-Peg3)-AQRAFIEWLLAQGPSSGAPPPS;
Y-Aib-EGTFTSDYSIALDK-K([17-carboxy-heptadecanoyl]-isoGlu-Peg3-Peg3)-AQRAFVEWLLAQGPSSGAPPPS; or
Y-Aib-EGTFTSDYSIALDK-K((19-Carboxy-nonadecanoyl)-[(Piperazine-1-yl)-acetyl]-Peg3-Peg3)-AQRAFVEWLLAQGPSSGAPPPS.

11. A pharmaceutical composition comprising a GIP analogue according to any one of claims 1 to 10, or a pharmaceutically acceptable salt or solvate thereof, in admixture with a carrier, e.g. wherein said composition is formulated as a liquid suitable for administration by injection or infusion, or formulated to cause slow release of said GIP analogue.

12. A GIP analogue according to any one of claims 1 to 10, or a pharmaceutically acceptable salt or solvate thereof, for use in a method of medical treatment

13. A GIP analogue according to any one of claims 1 to 10, or a pharmaceutically acceptable salt or solvate thereof, for use in a method of treatment and/or prevention of diabetes or a diabetes related disorder, or obesity or an obesity related disorder.

14. A GIP analogue for use according to claim 13 wherein:
(a) the diabetes related disorder is insulin resistance, glucose intolerance, increased fasting glucose, hypoglycemia (for example induced by insulin treatment), pre-diabetes, type 1 diabetes, type 2 diabetes, gestational diabetes hypertension, dyslipidemia, bone related disorder such as osteoporosis, or a combination thereof;
(b) the diabetes related disorder is atherosclerosis, arteriosclerosis, coronary heart disease, peripheral artery disease, stroke; or is a condition associated with atherogenic dyslipidemia, a blood fat disorder (such as high triglycerides, low HDL cholesterol, high LDL cholesterol, plaque buildup in artery walls, or a combination thereof), elevated blood pressure, hypertension, a prothrombotic state, or a proinflammatory state; or
(c) the obesity related disorder is obesity linked inflammation, obesity linked gallbladder disease or obesity induced sleep apnea, or may be associated with a condition selected from atherogenic dyslipidemia, blood fat disorders, elevated blood pressure, hypertension, a prothrombotic state, and a proinflammatory state, or a combination thereof.

## Patentansprüche

1. GIP-Analogon, das die folgende allgemeine Formel I aufweist: 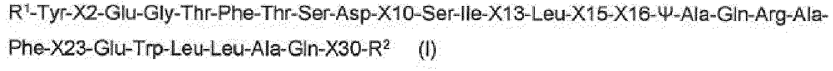 worin:
R¹ H, C₁₋₄-Alkyl, Acetyl, Formyl, Benzoyl, Trifluoracetyl oder pGlu ist;
X2 aus Aib und D-Ala ausgewählt ist;
X10 aus Tyr und Leu ausgewählt ist;
X13 aus Ala, Tyr und Aib ausgewählt ist;
X15 aus Asp und Glu ausgewählt ist;
X16 aus Glu und Lys ausgewählt ist;
X23 aus Val und Ile ausgewählt ist;
X30 aus Lys, Gly und Y1 ausgewählt ist oder fehlt;
und die Positionen 1 bis 29 des GIP-Analogons verglichen mit der Sequenz Y-Aib-EGTFTSDYSIYLDKKAQRAFVEWLLAQ maximal 4 Veränderungen aufweisen,
Y1 (falls vorhanden) aus Gly-Pro-Ser-Ser-Gly-Ala-Pro-Pro-Pro-Ser, Lys-Pro-Ser-Ser-Gly-Ala-Pro-Pro-Pro-Ser, Gly-Pro-Ser-Ser-Gly-Ala-Pro-Pro-Ser, Pro-Ser-Ser-Gly-Ala-Pro-Pro-Pro-Ser und Pro-Ser-Ser-Gly-Ala-Pro-Pro-Ser ausgewählt ist;
ψ ein Rest von Lys, Arg, Orn oder Cys ist, in dem die Seitenkette an einen Substituenten der Formel -Z¹ oder -Z²-Z¹ konjugiert ist; worin:
-Z¹ eine Fettsäurekette mit einer polaren Gruppe an einem Ende der Kette und einer Verbindung mit ψ oder Z² ist, -X- am Ende der Kette distal zur polaren Gruppe ist,
wobei die polare Gruppe eine Carbonsäure oder ein Carbonsäurebioisoster, eine Phosphonsäure oder eine Sulfonsäuregruppe umfasst;
und -X- eine Bindung, -CO-, -SO- oder -SO₂- ist;
-Z²- (falls vorhanden) ein Spacer der folgenden Formel ist: 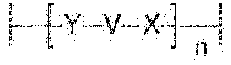
der Z¹ mit ψ verbindet;
wobei:
die Y jeweils unabhängig -NH, -NR, -S oder -O sind, wobei R Alkyl, eine Schutzgruppe ist oder eine Bindung an einen anderen Teil des Spacers Z² bildet;
die X jeweils unabhängig eine Bindung, CO-, SO- oder SO₂- sind;
mit der Maßgabe, dass, wenn Y -S ist, das X, an das es gebunden ist, eine Bindung ist;
die V jeweils unabhängig eine zweiwertige organische Gruppierung sind, die Y und X verbindet;
und
n = 1 bis 10 ist;
und
R² -NH₂ oder -OH ist;
oder ein pharmazeutisch annehmbares Salz oder Solvat davon;
wobei das GIP-Analogon an den GIP- und GLP-1-Rezeptoren eine Agonistenaktivität aufweist.

2. GIP-Analogon nach Anspruch 1, das weiters den Rest Glu15 umfasst.

3. GIP-Analogon nach Anspruch 1 oder 2, das eine der folgenden Kombinationen von Resten an den Positionen zwischen 1 und 29 besitzt:
Aib2, Tyr10, Tyr13, Asp15, Lys16, Val23;
D-Ala2, Tyr10, Tyr13, Asp15, Lys16, Val23;
Aib2, Tyr10, Aib13, Asp15, Lys16, Val23;
Aib2, Tyr10, Ala13, Glu15. Lys16, Val23;
Aib2, Leu10, Ala13, Glu15, Lys16, Val23;
Aib2, Tyr10, Tyr13, Glu15, Lys16, Val23;
D-Ala2, Tyr10, Ala13, Asp15, Lys16, Val23,;
Aib2, Tyr10, Ala13, Asp15, Lys16, Val23;
Aib2, Tyr10, Tyr13, Asp15, Lys16, Ile23;
D-Ala2, Tyr10, Tyr13. Asp15, Lys16, Ile23;
Aib2, Tyr10, Aib13, Asp15, Lys16, Ile23;
Aib2, Tyr10, Ala13, Glu15, Lys16, Ile23;
Aib2, Leu10, Ala13, Glu15, Lys16, Ile23.

4. GIP-Analogon nach Anspruch 1, wobei:
(a) die Reste 1 bis 29 der Formel I die folgende Sequenz aufweisen:
Y-Aib-EGTFTSDYStYLDKVAQRAFVEWLLAQ;
Y-DAla-EGTFTSDYSIYLDKΨAQRAFVEWLLAQ;
Y-Aib-EGTFTSDYSI-Aib-LDKΨAQRAFVEWLLAQ;
Y-Aib-EGTFTSDYSIALEKΨAQRAFVEWLLAQ;
Y-Aib-EGTFTSDLSIALEKΨAQRAFVEWLLAQ;
Y-Aib-EGTFTSDYSIALEKΨAQRAFVEWLLAQ;
Y-DAla-EGTFTSDYSIALEKΨAGRAFVEWLLAQ;
Y-Aib-EGTFTSDLSIALEKΨAQRAFVEWLLAQ;
Y-Aib-EGTFTSDYSIALDKΨAQRAFVEWLLAQ;
Y-DAla-EGTFTSDYSIALDKΨAQRAFVEWLLAQ;
Y-Aib-EGTFTSDYSIALDEΨAQRAFVEWLLAQ;
Y-Aib-EGTFTSDYSIALDKΨAQRAFIEWLLAQ-; oder
Y-Aib-EGTFTSDYSIALDKΨAQRAFVEWLLAQ; oder
(b) die Peptidhauptkette (Formel I) die folgende Sequenz aufweist:
Y-Aib-EGTFTSDYSIYLDKΨAQRAFVEWLLAQGPSSGAPPPS;
Y-DAla-EGTFTSDYSlYLDKVAQRAFVEWLLAQGPSSGAPPPS;
Y-Aib-EGTFTSDYSI-Aib-LDKΨAQRAFVEWLLAQGPSSGAPPPS;
Y-Aib-EGTFTSDYSIALEKΨAQRAFVEWLAQK;
Y-Aib-EGTFTSDLSIALEKΨAQRAFVEWLLAQK;
Y-Aib-EGTFTSDYSIALEKΨAQRAFVEWLLAQGPSSGAPPPS;
Y-DAla-EGTFTSDYSIALEKΨAQRAFVEWLLAQGPSSGAPPPS;
Y-Aib-EGTFTSDLSIALEKΨAQRAFVEWLLAQKPSSGAPPPS;
Y-Aib-EGTFTSDYSIALDKΨAQRAFVEWLLAQKPSSGAPPPS;
Y-DAla-EGTFTSDYSIALDKΨAQRAFVEWLLAQKPSSGAPPPS;
Y-Aib-EGTFTSDVSIALDKΨAQRAFVEWLLAQGPSSGAPPPS;
Y-Aib-EGTFTSDYSIALDEΨAQRAFVEWLLAQGPSSGAPPPS;
Y-Aib-EGTFTSDYSIALDKΨAQRAFIEWLLAQGPSSGAPPPS-;
Y-Aib-EGTFTSDYSIALDKΨAQRAFVEWLLAQGPSSGAPPP;
Y-Aib-EGTFTSDYSIALDKΨAQRAFVEWLLAQGPSSGAPPPS; oder
Y-Aib-EGTFTSDLSIALEKΨAQRAFVEWLLAQGPSSGAPPPS.

5. GIP-Analogon nach einem der vorangegangenen Ansprüche, wobei ψ ein Rest von Lys ist, in dem die Seitenkette an einen Substituenten der Formel -Z¹ oder -Z²-Z¹ konjugiert ist.

6. GIP-Analogon nach Anspruch 5, wobei Z¹ HOOC-B-C₁₆₋₂₂-Alkylen-(CO)- ist, worin B eine Bindung ist.

7. GIP-Analogon nach Anspruch 5 oder 6, wobei Z¹ Folgendes ist:
| | |
|---|---|
| 17-Carboxyheptadecanoyl | [HOOC-(CH₂)₁₆-(CO)-]; |
| 19-Carboxynonadecanoyl | [HOOC-(CH₂)₁₈-(CO)-]; oder |
| 21-Carboxyhenicosanoyl | [HOOC-(CH₂)₂₀-(CO)-]. |

8. GIP-Analogon nach einem der Ansprüche 5 bis 7, wobei Z² einen Rest von isoGlu, Dapa oder Peg 3 umfasst.

9. GIP-Analogon nach einem der Ansprüche 5 bis 8, wobei -Z²-Z¹ Folgendes ist:
(i) [17-Carboxyheptadecanoyl]-isoGlu-Peg3-Peg3 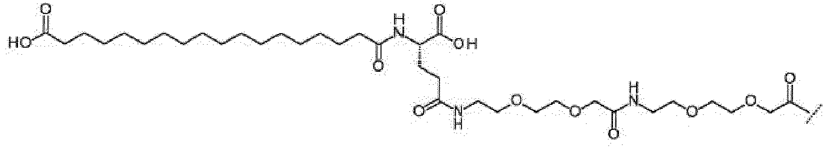
(ii) [17-Carboxyheptadecanoyl]-isoGlu 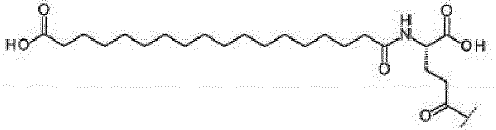
(iii) [17-Carboxyheptadecanoyl]-Peg3-Peg3-isoGlu 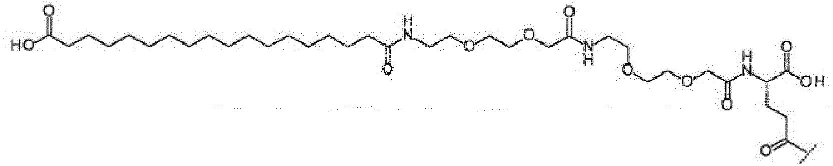
(iv) [17-Carboxyheptadecanoyl]-isoGlu-GSGSGG 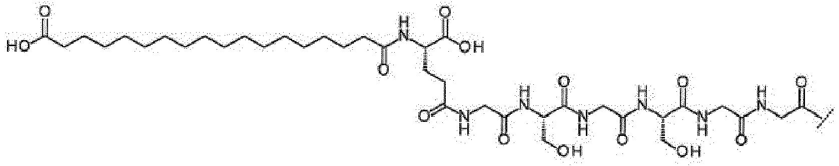
(v) [17-Carboxyheptadecanoyl]-AA-Peg3-Peg3 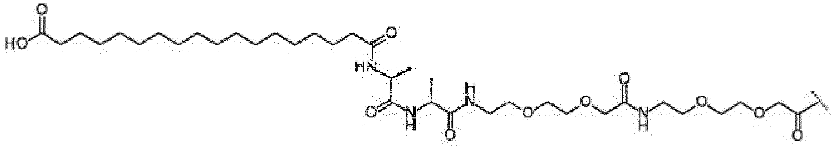
(vi) ([19-Carboxynonadecanoyl]-AA-Peg3-Peg3-Peg3) 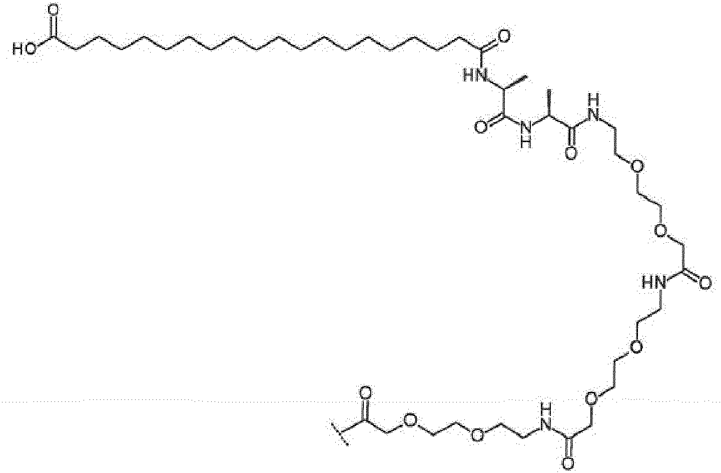
(vii) [17-Carboxyheptadecanoyl]-Peg3-Peg3 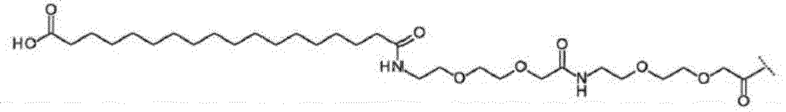
(viii) [17-Carboxyheptadecanoyl]-isoGlu-Peg3-Peg3-Peg3 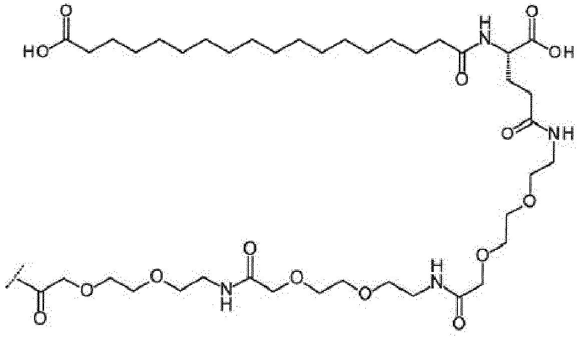
(ix) [17-Carboxyheptadecanoyl]-Dapa-Peg3-Peg3 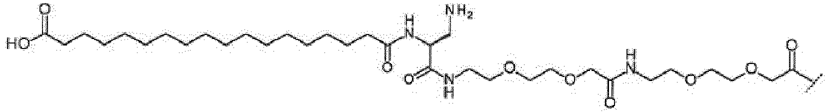
(x) [19-Carboxyheptadecanoyl]-isoGlu-Peg3-Peg3-Peg3 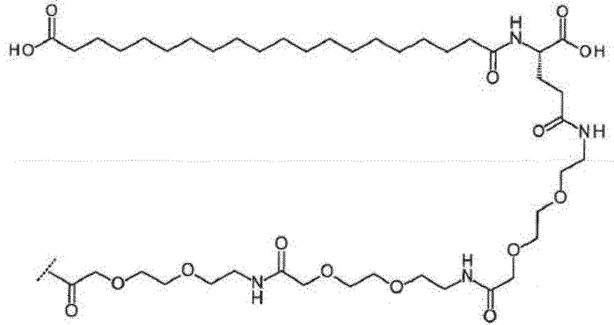
(xi) [19-Carboxynonadecanoyl]-isoGlu-Peg3-Peg3 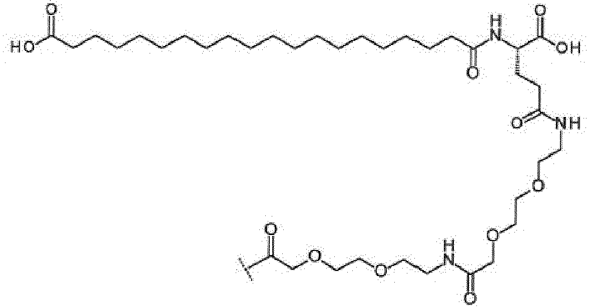
(xii) (19-Carboxynonadecanoyl)[(piperazin-1-yl)acetyl)]-Peg3-Peg3 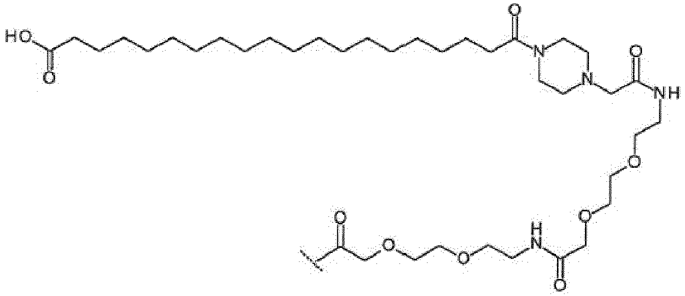 oder
(xiii) (19-Carboxynonadecanoyl)[(piperazin-1-yl)acetyl]-Peg6

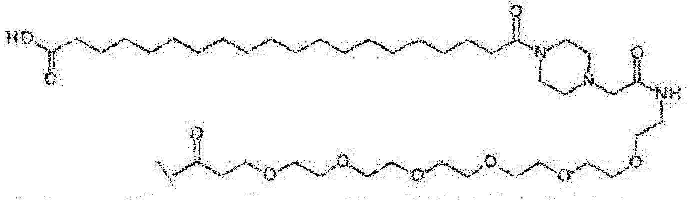

10. GIP-Analogon nach Anspruch 1, wobei:
(a) die Reste 1 bis 29 der Formel I die folgende Sequenz aufweisen:
Y-Aib-EGTFTSDYSIYLDK-K([17-Carboxyheptadecanyol]-isoGlu-Peg3-Peg3)-AQRAFVEWLLAQ;
Y-Aib-EGTFTSDYSIYLDK-K([19-Carboxynonadecanoyl]-isoGlu-Peg3-Peg3).
AQRAFVEWLLAQ;
Y-Aib-EGTFTSDYSIYLDK-K([17-Carboxyheptadecanoyl]-isoGlu)-AQRAFVEWLLAQ;
H-Y-Aib-EGTFTSDYSIYLDK-K{[17-Carboxyheptadecanyol]-isoGlu-GSGSGG)-AQRAFVEWLLAQ;
Y-Aib-EGTFTSDYSIYLDK-K([17-Carboxyheptadecanoyl]-Peg3-Peg3)-AQRAFVEWLLAQ;
Y-Aib-EGTFTSDYSIYLDK-K([17-Carboxyheptadecanoyl]-isoGlu-Peg3-Peg3-Peg3)-AQRAFVEWLLAQ;
Y-Aib-EGTFTSDYSIYLDK-K([17-Carboxyheptadecanoyl]-Dapa-Peg3-Peg3)-AQRAFVEWLLAQ;
Y-Aib-EGTFTSDYSIYLDK-K([19-Carboxynonadecanoyl]-isoGlu-Peg3-Peg3-Peg3)-AQRAFVEWLLAQ;
Y-DAla-EGTFTSDYSIYLDK-K([17-Carboxyheptadecanoyl]-isoGlu-Peg3-Peg3)-AQRAFVEWLLAQ;
Y-Aib-EGTFTSDYSI-Aib-LDK-K([17-Carboxyheptadecanoyl]-isoGlu-Peg3-Peg3)-AQRAFVEWLLAQ;
Y-Aib-EGTFTSDYSIALEK-K([17-Carboxyheptadecanyol]-isoGlu-Peg3-Peg3)-AQRAFVEWLLAQ;
Y-Aib-EGTFTSDLSIALEK-K([17-Carboxyheptadecanoyl]-isoGlu-Peg3-Peg3)-AQRAFVEWLLAQ;
Y-Aib-EGTFTSDYSIALEK-K[19-Carboxynonadecanoyl]-isoGlu-Peg3-Peg3)-AQRAFVEWLLAQ;
Y-Aib-EGTFTSDLSIALEK-K([19-Carboxynonadecanoyl]-isoGlu-Peg3-Peg3)-AQRAFVEWLLAQ;
Y-DAla-EGTFTSDYSIYLDK-K([19-Carboxynonadecanoyl]-isoGlu-Peg3-Peg3)-AQRAFVEWLLAQ;
Y-Aib-EGTFTSDYSIALEK-K([19-Carboxynonadecanoyl]-isoGlu-Peg3-Peg3)-AQRAFVEWLLAQ;
Y-DAla-EGTFTSDYSIALEK-K([19-Carboxynonadecanoyl]-isoGlu-Peg3-Peg3)-AQRAFVEWLLAQ;
Y-Aib-EGTFTSDLSIALEK-K([19-Carboxynonadecanoyl]-isoGlu-Peg3-Peg3)-AQRAFVEWLLAQ;
Y-Aib-EGTFTSDYSIYLDK-K([19-Carboxynonadecanoyl]-Peg3-Peg3)-AQRAFVEWLLAQ;
Y-Aib-EGTFTSDYSIYLDK-K([19-Carboxynonadecanoyl]-IsoGlu)-AQRAFVEWLLAQ;
Y-Aib-EGTFTSDYSIYLDK-K([19-Carboxynonadecanoyl]-Dapa-Peg3-Peg3)-AQRAFVEWLLAQ;
Y-Aib-EGTFTSDYSIALDK-K([19-Carboxynonadecanoyl]-isoGlu-Peg3-Peg3)-AQRAFVEWLLAQ;
Y-DAla-EGTFTSDYSIALDK-K([19-Carboxynonadecanoyl]-isoGlu-Peg3-Peg3)-AQRAFVEWLLAQ;
Y-Aib-EGTFTSDYSIALDE-K([19-Carboxynonadecanoyl]-isoGlu-Peg3-Peg3)-AQRAFVEWLLAQ;
Y-Aib-EGTFTSDYSIALDK-K([19-Carboxynonadecanoyl]-isoGlu-Peg3-Peg3)-AQRAFIEWLLAQ;
Y-Aib-EGTFTSDYSIALDK-K([17-Carboxyheptadecanoyl]-isoGlu-Peg3-Peg3)-AQRAFVEWLLAQ; oder
Y-Aib-EGTFTSDYSIALDK-K((19-Carboxynonadecanoyl)-[(piperazin-1-yl)-acetyl]-Peg3-Peg3)-AQRAFVEWLLAQ; oder
(b) die Peptidhauptkette (Formel I) die folgende Sequenz aufweist:
Y-Aib-EGTFTSIDYSIYLDK-K([17-Carboxyheptadecanoyl]-isoGlu-Peg3-Peg3)-AQRAFVEWLLAQGPSSGAPPPS;
Y-Aib-EGTFTSDYSIYLDK-K([19-Carboxynonadecanoyl]-isoGlu-Pag3-Peg3)-AQRAFVEWLLAQGPSSGAPPPS;
Y-Aib-EGTFTSDYSIYLDK-K([17-Carboxyheptadecanoyl]-isoGlu)-AQRAFVEWLLAQGPSSGAPPPS;
H-Y-Aib-EGTFTSDYSIYLDK-K([17-Carboxyheptadecanoyl]-isoGlu-GSGSGG)-AQRAFVEWLLAQGPSSGAPPPS;
Y-Aib-EGTFTSDYSIYLDK-K([17-Carboxyheptadecanoyl]-Peg3-Peg3)-AQRAFVEWLLAQGPSSGAPPPS;
Y-Aib-EGTFTSDYSIYLDK-K([17-Carboxyheptadecanoyl]-isoGlu-Peg3-Peg3-Peg3)-AQRAFVEWLLAQGPSSGAPPPS;
Y-Aib-EeTFTSDYSIYLDK-K([17-Carboxyheptadecanoyl]-Dapa-Peg3-Peg3)-AQRAFVEWLLAQGPSSGAPPPS;
Y-Aib-EGTFTSDYSIYLDK-K([19-Carboxynonadecartoyl]-isoGlu-Peg3-Peg3-Peg3)-AQRAFVEWLLAQGPSSGAPPPS;
Y-DAla-EGTFTSDYSIYLDK-K([17-Carboxyheptadecanoyl]-isoGlu-Peg3-Peg3)-AQRAFVEWLLAQGPSSGAPPPS;
Y-Aib-EGTFTSDYSI-Aib-LDK-K([17-Carboxyheptadecanoyl]-isoGlu-Peg3-Peg3)-AQRAFVEWLLAQGPSSGAPPPS;
Y-Alb-F-GTFTSDYSIALEK-K([17-Carboxyheptadecanoyl]-isoGlu-Peg3-Peg3)-AQRAFVEWLLAQK,
Y-Aib-EGTPTSDLSIALEK-K([17-Carboxyheptadecanoyl]-isoGlu-Peg3-Peg3)-AQRAFVEWLLAQK;
Y-Aib-EGTFTSDYSIALEK-K[19-Carboxynonadecanoyl]-isoGlu-Peg3-Peg3)-AQRAFVEWLLAQK;
Y-Aib-EGTFTSDLSIALEK-K([19-Carboxynonadecanoyl]-isoGlu-Peg3-Peg3)-AQRAFVEWLLAQK;
Y-DAla-EGTFTSDYSIYLDK-K([19-Carboxynonadecanoyl]-isoGlu-Peg3-Peg3)-AQRAFVEWLLAQGPSSGAPPPS;
Y-Aib-EGTFTSDYSIALEK-K([19-Carboxynonadecanoyl]-isoGlu-Peg3-Peg3)-AQRAFVEWLLAQGPSSGAPPPS;
Y-DAla-EGTFTSDYSIALEK-K([19-Carboxynonadecanoyl]-isoGlu-Peg3-Peg3)-AQRAFVEWLLAQGPSSGAPPPS;
Y-Aib-EGTFTSDLSIALEK-K([19- Carboxynonadecanoyl1-isoGlu-Peg3-Reg3)-AQRAFVEWLLAQKPSSGAPPPS;
Y-Aib-EGTFTSDYSIYLDK-K([19-Carboxynonadecanoyl]-Peg3-Peg3)-AQRAFVEWLLAQGPSSGAPPPS;
Y-Aib-EGTPTSDYSIYLDK-K([19-Carboxynonadecanoyl]-IsoGlu)-AQRAFVEWLLAQGPSSGAPPPS;
Y-Aib-EGTFTSDYSIYLDK-K([19-Carboxynonadecanoyl]-Dapa-Peg3-Peg3)-AQRAFVEWLLAQGPSSGAPPPS;
Y-Aib-EGTFTSDLSIALEK-K([19-Carboxynonadecanoyl]-isoGlu-Peg3-Peg3)-AGRAFVEWLLAQGPSSGAPPPS
Y-Aib-EGTFTSDYSIALDK-K([19-Carboxynonadecanoyl]-isoGlu-Peg3-Peg3)-AQRAFVEWLLAQKPSSGAPPPS;
Y-DAla-EGTFTSDYSIALDK-K([19-Carboxynonadecanoyl]-isoGlu-Peg3-Peg3)-AQRAFVEWLLAQKPSSGAPPPS;
Y-Aib-ESTFTSDYSIALDK-K([19-Carboxynonadecanoyl]-isoGlu-Peg3-Peg3)-AQRAFVEWLLAQGPSSGAPPPS;
Y-Aib-EGTFTSDYSIALDE-K([19-Carboxynonadecanoyl]-isoGlu-Peg3-Peg3)-AQRAFVEWLLAQGPSSGAPPPS;
Y-Aib-EGTFTSDYSIALDK-K([19-Carboxynonadecanoyl]-isoGlu-Peg3-Peg3)-AQRAFIEWLLAQGPSSGAPPPS;
Y-Aib-EGTPTSDYSIALDK-K5[17-Carboxyheptadecanoyl]-isoGlu-Peg3-Peg3)-AQRAFVEWLLAQGPSSGAPPPS; oder
Y-Aib-EGTFTSDYSIALDK-K((19-Carboxynonadecanoyl)-[(piperazin-1-yl)-acetyl]-Peg3-Peg3)-AQRAFVEWLL4QGPSSGAPPPS.

11. Pharmazeutische Zusammensetzung, umfassend ein GIP-Analogon nach einem der Ansprüche 1 bis 10 oder ein pharmazeutisch annehmbares Salz oder Solvat davon, in Beimengung mit einem Träger, z.B. wobei die Zusammensetzung als eine Flüssigkeit formuliert ist, die zur Verabreichung durch Injektion oder Infusion geeignet ist, oder wobei sie formuliert ist, um eine langsame Freisetzung des GIP-Analogons zu bewirken.

12. GIP-Analogon nach einem der Ansprüche 1 bis 10 oder ein pharmazeutisch annehmbares Salz oder Solvat davon zur Verwendung in einem Verfahren zur medizinischen Behandlung.

13. GIP-Analogon nach einem der Ansprüche 1 bis 10 oder ein pharmazeutisch annehmbares Salz oder Solvat davon zur Verwendung in einem Verfahren zur Behandlung und/oder Prävention von Diabetes oder einer diabetesbedingten Störung oder von Adipositas oder einer adipositasbedingten Störung.

14. GIP-Analogon zur Verwendung nach Anspruch 13, wobei:
(a) die diabetesbedingte Störung eine Insulinresistenz, Glucoseintoleranz, erhöhte Glucose im nüchternen Zustand, Hypoglykämie (z.B. ausgelöst durch Insulinbehandlung), Prädiabetes, Typ-1-Diabetes, Typ-2-Diabetes, Bluthochdruck bei Schwangerschaftsdiabetes, Dyslipidämie, Knochenerkrankungen wie Osteoporose oder eine Kombination daraus ist;
(b) die diabetesbedingte Störung Atherosklerose, Arteriosklerose, koronare Herzkrankheit, periphere Arterienkrankheit, Schlaganfall oder ein Leiden ist, das mit atherogener Dyslipidämie, einer Blutfettstörung (wie hohen Triglyzeriden, niedrigem HDL-Cholesterin, hohem LDL-Cholesterin, Plaqueablagerungen an Arterienwänden oder einer Kombination daraus), erhöhtem Blutdruck, Bluthochdruck, einem Prothrombosezustand oder einem entzündungsfördernden Zustand in Zusammenhang steht; oder
(c) die adipositasbedingte Störung adipositasbedingte Entzündung, adipositasbedingte Gallenblasenkrankheit oder adipositasbedingte Schlafapnoe ist oder mit einem Leiden in Zusammenhang stehen kann, das aus atherogener Dyslipidämie, Blutfettstörungen, erhöhtem Blutdruck, Bluthochdruck, einem Prothrombosezustand und einem entzündungsfördernden Zustand oder einer Kombination daraus ausgewählt ist.

## Revendications

1. Analogue du GIP possédant la Formule générale I : 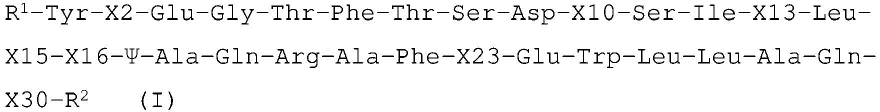 dans laquelle
R¹ est H, alkyle en C₁₋₄, acétyle, formyle, benzoyle, trifluoroacétyle ou pGlu ;
X2 est sélectionné parmi Aib et D-Ala ;
X10 est sélectionné parmi Tyr et Leu ;
X13 est sélectionné parmi Ala, Tyr et Aib ;
X15 est sélectionné parmi Asp et Glu ;
X16 est sélectionné parmi Glu et Lys ;
X23 est sélectionné parmi Val et Ile ;
X30 est sélectionné parmi Lys, Gly et Y1, ou est absent ;
et les positions 1 à 29 de l'analogue du GIP comportent au maximum 4 modifications par comparaison à la séquence Y-Aib-EGTFTSDYSIYLDKKAQRAFVEWLLAQ ;
Y1 (lorsque présent) est sélectionné parmi Gly-Pro-Ser-Ser-Gly-Ala-Pro-Pro-Pro-Ser, Lys-Pro-Ser-Ser-Gly-Ala-Pro-Pro-Pro-Ser, Gly-Pro-Ser-Ser-Gly-Ala-Pro-Pro-Ser, Pro-Ser-Ser-Gly-Ala-Pro-Pro-Pro-Ser et Pro-Ser-Ser-Gly-Ala-Pro-Pro-Ser ;
Ψ est un résidu de Lys, Arg, Orn ou Cys dans lequel la chaîne latérale est conjuguée à un substituant possédant la formule -Z¹ ou -Z²-Z¹ ; où :
-Z¹ est une chaîne lipidique comportant un groupe polaire à une extrémité de la chaîne et une connexion à Ψ ou Z², -X- à la fin de la chaîne en position distale du groupe polaire,
où le groupe polaire comprend un acide carboxylique ou un bioisostère d'acide carboxylique, un acide phosphonique, ou un groupe acide sulfonique ;
et -X- est une liaison, -CO-, -SO-, ou -SO₂- ;
-Z² (si présent) est un espaceur ayant la formule : 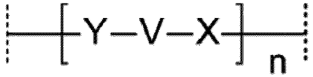 connectant Z¹ à Ψ ;
dans laquelle :
chaque Y est indépendamment -NH, -NR, -S ou -O, où R est un alkyle, un groupe protecteur ou forme une liaison avec une autre partie de l'espaceur Z² ;
chaque X est indépendamment une liaison, CO-, SO-, ou SO₂- ;
à condition que lorsque Y est -S, le X auquel il est lié soit une liaison ;
chaque V est indépendamment un fragment organique bivalent liant Y et X ;
et n est 1-10 ;
et
R² est -NH₂ ou -OH ;
ou un sel ou solvate de celui-ci pharmaceutiquement acceptable ;
où ledit analogue du GIP possède une activité agoniste au niveau des récepteurs du GIP et du GLP-1.

2. Analogue du GIP selon la revendication 1 comprenant en outre le résidu Glu15.

3. Analogue du GIP selon la revendication 1 ou la revendication 2 possédant l'une des combinaisons de résidus suivantes aux positions entre 1 et 29 :
Aib2, Tyr10, Tyr13, Asp15, Lys16, Va123 ;
D-Ala2, Tyr10, Tyr13, Asp15, Lys16, Va123 ;
Aib2, Tyr10, Aib13, Asp15, Lys16, Va123 ;
Aib2, Tyr10, Ala13, Glu15, Lys16, Va123 ;
Aib2, Leu10, Ala13, Glu15, Lys16, Va123 ;
Aib2, Tyr10, Tyr13, Glu15, Lys16, Va123 ;
D-Ala2, Tyr10, Ala13, Asp15, Lys16, Va123 ;
Aib2, Tyr10, Ala13, Asp15, Lys16, Va123 ;
Aib2, Tyr10, Tyr13, Asp15, Lys16, Ile23 ;
D-Ala2, Tyr10, Tyr13, Asp15, Lys16, Ile23 ;
Aib2, Tyr10, Aib13, Asp15, Lys16, Ile23 ;
Aib2, Tyr10, Ala13, Glu15, Lys16, Ile23 ;
Aib2, Leu10, ALa13, Glu15, Lys16, Ile23.

4. Analogue du GIP selon la revendication 1 dans lequel :
(a) les résidus 1 à 29 de la Formule I possèdent la séquence :
Y-Aib-EGTFTSDYSIYLDKΨAQRAFVEWLLAQ ;
Y-DAla-EGTFTSDYSIYLDKΨAQRAFVEWLLAQ ;
Y-Aib-EGTFTSDYSI-Aib-LDKΨAQRAFVEWLLAQ ;
Y-Aib-EGTFTSDYSIALEKΨAQRAFVEWLLAQ ;
Y-Aib-EGTFTSDLSIALEKΨAQRAFVEWLLAQ ;
Y-Aib-EGTFTSDYSIALEKΨAQRAFVEWLLAQ ;
Y-DAla-EGTFTSDYSIALEKΨAQRAFVEWLLAQ ;
Y-Aib-EGTFTSDLSIALEKΨAQRAFVEWLLAQ ;
Y-Aib-EGTFTSDYSIALDKΨAQRAFVEWLLAQ ;
Y-DAla-EGTFTSDYSIALDKΨAQRAFVEWLLAQ ;
Y-Aib-EGTFTSDYSIALDEΨAQRAFVEWLLAQ ;
Y-Aib-EGTFTSDYSIALDKΨAQRAFIEWLLAQ- ; ou
Y-Aib-EGTFTSDYSIALDKΨAQRAFVEWLLAQ ; ou
(b) le squelette peptidique (Formule I) possède la séquence :
Y-Aib-EGTFTSDYSIYLDKΨAQRAFVEWLLAQGPSSGAPPPS ;
Y-DAla-EGTFTSDYSIYLDKΨAQRAFVEWLLAQGPSSGAPPPS ;
Y-Aib-EGTFTSDYSI-Aib-LDKΨAQRAFVEWLLAQGPSSGAPPPS ;
Y-Aib-EGTFTSDYSIALEKΨAQRAFVEWLLAQK ;
Y-Aib-EGTFTSDLSIALEKΨAQRAFVEWLLAQK ;
Y-Aib-EGTFTSDYSIALEKΨAQRAFVEWLLAQGPSSGAPPPS ;
Y-DAla-EGTFTSDYSIALEKΨAQRAFVEWLLAQGPSSGAPPPS ;
Y-Aib-EGTFTSDLSIALEKΨAQRAFVEWLLAQKPSSGAPPPS ;
Y-Aib-EGTFTSDYSIALDKΨAQRAFVEWLLAQKPSSGAPPPS ;
Y-DAla-EGTFTSDYSIALDKΨAQRAFVEWLLAQKPSSGAPPPS ;
Y-Aib-EGTFTSDYSIALDKΨAQRAFVEWLLAQGPSSGAPPPS ;
Y-Aib-EGTFTSDYSIALDEΨAQRAFVEWLLAQGPSSGAPPPS ;
Y-Aib-EGTFTSDYSIALDKΨAQRAFIEWLLAQGPSSGAPPPS- ;
Y-Aib-EGTFTSDYSIALDKΨAQRAFVEWLLAQGPSSGAPPP ;
Y-Aib-EGTFTSDYSIALDKΨAQRAFVEWLLAQGPSSGAPPPS ; ou
Y-Aib-EGTFTSDLSIALEKΨAQRAFVEWLLAQGPSSGAPPPS.

5. Analogue du GIP selon l'une quelconque des revendications précédentes, dans lequel Ψ est un résidu de Lys dans lequel la chaîne latérale est conjuguée à un substituant possédant la formule -Z¹ ou -Z²-Z¹.

6. Analogue du GIP selon la revendication 5, dans lequel Z¹ est HOOC-B-alkylène en C₁₆₋₂₂-(CO)-, dans laquelle B est une liaison.

7. Analogue du GIP selon la revendication 5 ou la revendication 6, dans lequel Z¹ est :
17 carboxyheptadécanoyle [HOOC-(CH₂)₁₆-(CO)-];
19-carboxynonadécanoyle [HOOC-(CH₂)₁₈-(CO)-] ; ou
21-carboxyhénicosanoyle [HOOC-(CH₂)₂₀-(CO)-].

8. Analogue du GIP selon l'une quelconque des revendications 5 à 7, dans lequel Z² comprend un résidu de isoGlu, Dapa, ou Peg3.

9. Analogue du GIP selon l'une quelconque des revendications 5 à 8, dans lequel -Z²-Z¹ est :
(i) [17-carboxy-heptadécanoyl]-isoGlu-Peg3-Peg3 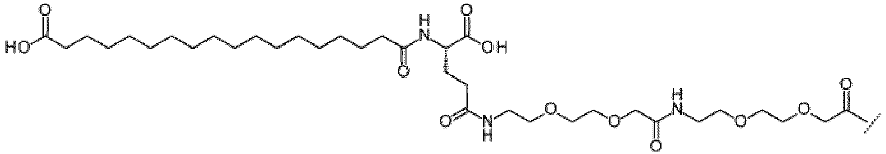
(ii) [17-carboxy-heptadécanoyl]-isoGlu 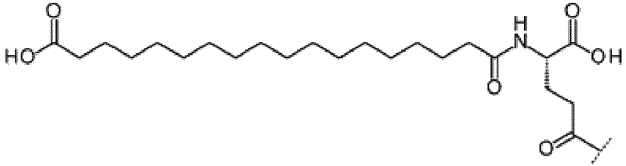
(iii) [17-carboxy-heptadécanoyl]-Peg3-Peg3-isoGlu 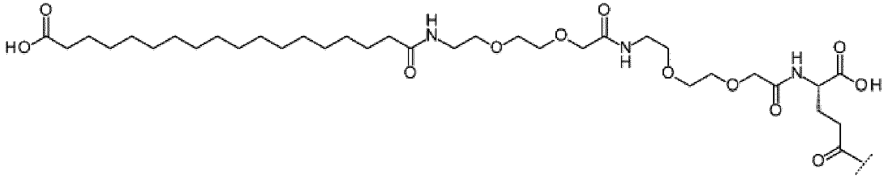
(iv) [17-carboxy-heptadécanoyl]-isoGlu-GSGSGG 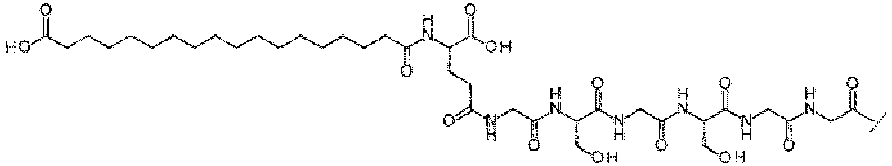
(v) [17-carboxy-heptadécanoyl]-AA-Peg3-Peg3 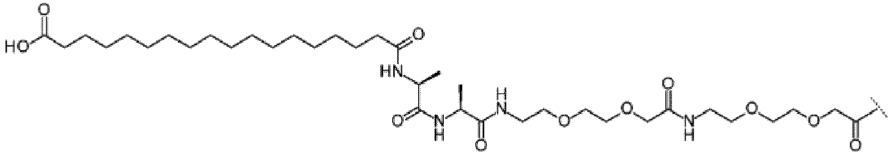
(vi) ([19-carboxy-nonadécanoyl]-AA-Peg3-Peg3-Peg3) 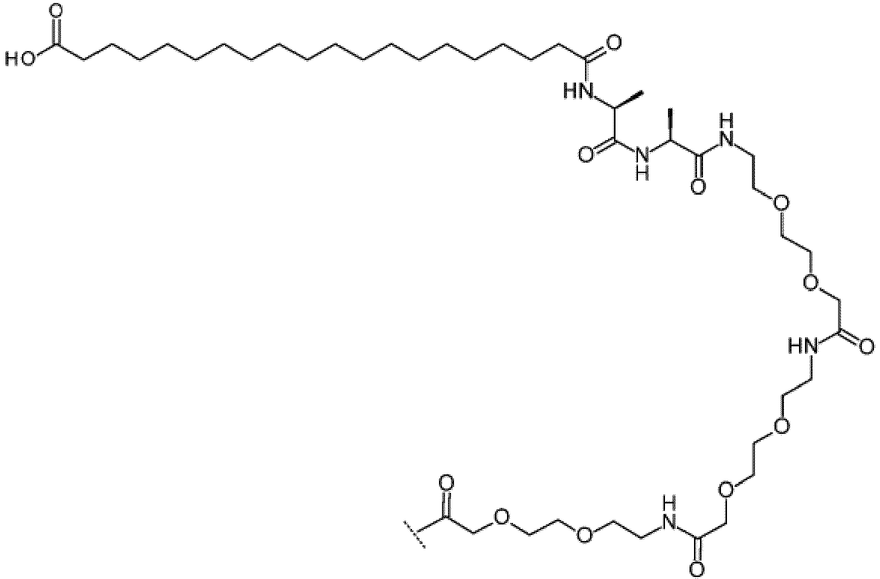
(vii) [17-carboxy-heptadécanoyl]-Peg3-Peg3 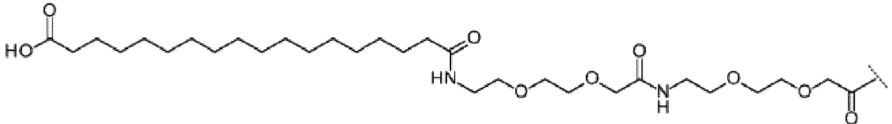
(viii) [17-carboxy-heptadécanoyl]-isoGlu-Peg3-Peg3-Peg3 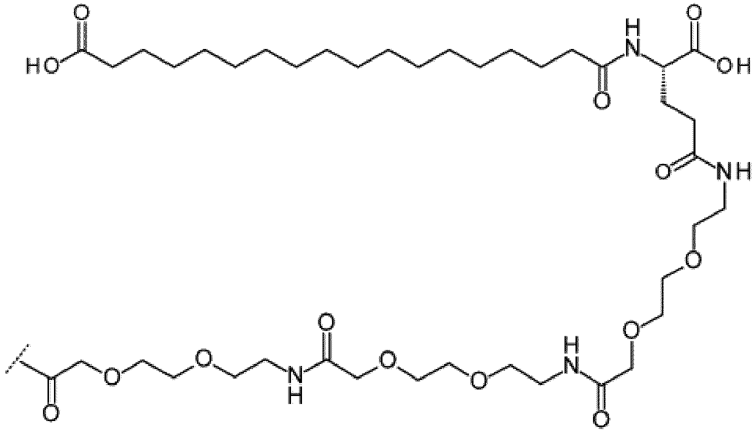
(ix) [17-carboxy-heptadécanoyl]-Dapa-Peg3-Peg3 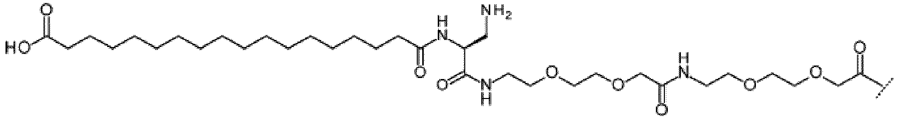
(x) [19-carboxy-heptadécanoyl]-isoGlu-Peg3-Peg3-Peg3 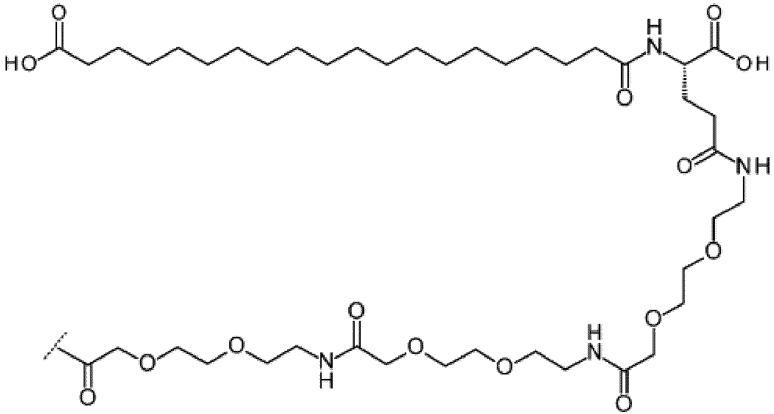
(xi) [19-carboxy-nonadécanoyl]-isoGlu-Peg3-Peg3 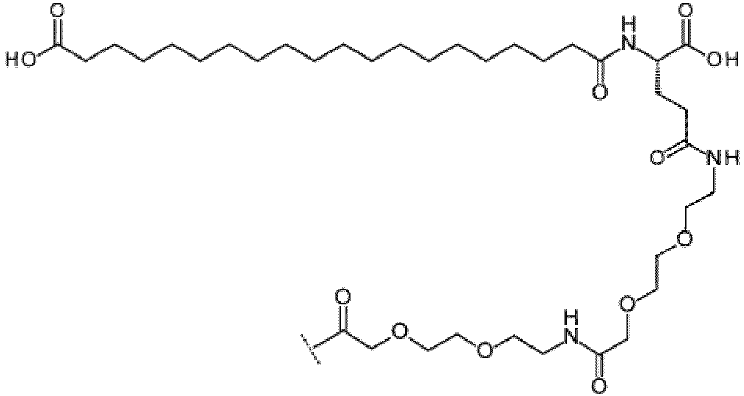
(xii) [19-carboxy-nonadécanoyl]-[(pipérazine-1-yl)-acétyl]-Peg3-Peg3 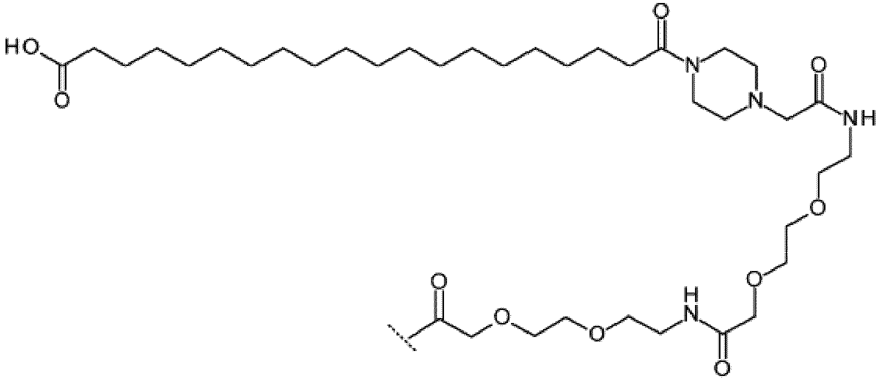 ou
(xiii) [19-carboxy-nonadécanoyl]-[(pipérazine-1-yl)-acétyl]-Peg6

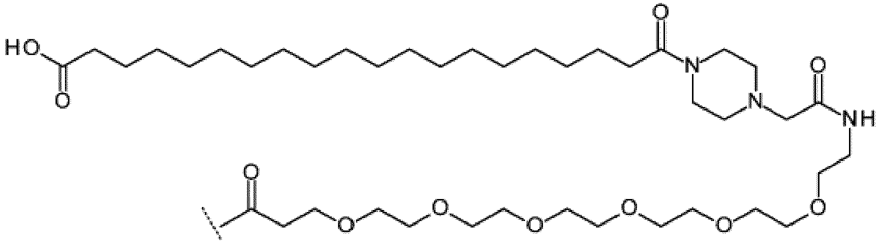

10. Analogue du GIP selon la revendication 1 dans lequel :
(a) les résidus 1-29 de la Formule I possèdent la séquence :
Y-Aib-EGTFTSDYSIYLDK-K([17-carboxy-heptadécanoyl]-isoGlu-Peg3-Peg3)-AQRAFVEWLLAQ ;
Y-Aib-EGTFTSDYSIYLDK-K([19-carboxy-nonadécanoyl]-isoGlu-Peg3-Peg3)-AQRAFVEWLLAQ ;
Y-Aib-EGTFTSDYSIYLDK-K([17-carboxy-heptadécanoyl]-isoGlu)-AQRAFVEWLLAQ ;
H-Y-Aib-EGTFTSDYSIYLDK-K([17-carboxy-heptadécanoyl]-isoGlu-GSGSGG)-AQRAFVEWLLAQ ;
Y-Aib-EGTFTSDYSIYLDK-K([17-carboxy-heptadécanoyl]-Peg3-Peg3)-AQRAFVEWLLAQ ;
Y-Aib-EGTFTSDYSIYLDK-K([17-carboxy-heptadécanoyl]-isoGlu-Peg3-Peg3-Peg3)-AQRAFVEWLLAQ ;
Y-Aib-EGTFTSDYSIYLDK-K([17-carboxy-heptadécanoyl]-Dapa-Peg3-Peg3)-AQRAFVEWLLAQ ;
Y-Aib-EGTFTSDYSIYLDK-K([19-carboxy-nonadécanoyl]-isoGlu-Peg3-Peg3-Peg3)-AQRAFVEWLLAQ ;
Y-DAla-EGTFTSDYSIYLDK-K([17-carboxy-heptadécanoyl]-isoGlu-Peg3-Peg3)-AQRAFVEWLLAQ ;
Y-Aib-EGTFTSDYSI-Aib-LDK-K([17-carboxy-heptadécanoyl]-isoGlu-Peg3-Peg3)-AQRAFVEWLLAQ ;
Y-Aib-EGTFTSDYSIALEK-K([17-carboxy-heptadécanoyl]-isoGlu-Peg3-Peg3)-AQRAFVEWLLAQ ;
Y-Aib-EGTFTSDLSIALEK-K([17-carboxy-heptadécanoyl]-isoGlu-Peg3-Peg3)-AQRAFVEWLLAQ ;
Y-Aib-EGTFTSDYSIALEK-K([19-carboxy-nonadécanoyl]-isoGlu-Peg3-Peg3)-AQRAFVEWLLAQ ;
Y-Aib-EGTFTSDLSIALEK-K([19-carboxy-nonadécanoyl]-isoGlu-Peg3-Peg3)-AQRAFVEWLLAQ ;
Y-DAla-EGTFTSDYSIYLDK-K([19-carboxy-nonadécanoyl]-isoGlu-Peg3-Peg3)-AQRAFVEWLLAQ ;
Y-Aib-EGTFTSDYSIALEK-K([19-carboxy-nonadécanoyl]-isoGlu-Peg3-Peg3)-AQRAFVEWLLAQ ;
Y-DAla-EGTFTSDYSIALEK-K([19-carboxy-nonadécanoyl]-isoGlu-Peg3-Peg3)-AQRAFVEWLLAQ ;
Y-Aib-EGTFTSDLSIALEK-K([19-carboxy-nonadécanoyl]-isoGlu-Peg3-Peg3)-AQRAFVEWLLAQ ;
Y-Aib-EGTFTSDYSIYLDK-K([19-carboxy-nonadécanoyl]-Peg3-Peg3)-AQRAFVEWLLAQ ;
Y-Aib-EGTFTSDYSIYLDK-K([19-carboxy-nonadécanoyl]-isoGlu)-AQRAFVEWLLAQ ;
Y-Aib-EGTFTSDYSIYLDK-K([19-carboxy-nonadécanoyl]-Dapa-Peg3-Peg3)-AQRAFVEWLLAQ ;
Y-Aib-EGTFTSDYSIALDK-K([19-carboxy-nonadécanoyl]-isoGlu-Peg3-Peg3)-AQRAFVEWLLAQ ;
Y-DAla-EGTFTSDYSIALDK-K([19-carboxy-nonadécanoyl]-isoGlu-Peg3-Peg3)-AQRAFVEWLLAQ ;
Y-Aib-EGTFTSDYSIALDE-K([19-carboxy-nonadécanoyl]-isoGlu-Peg3-Peg3)-AQRAFVEWLLAQ ;
Y-Aib-EGTFTSDYSIALDK-K([19-carboxy-nonadécanoyl]-isoGlu-Peg3-Peg3)-AQRAFIEWLLAQ ;
Y-Aib-EGTFTSDYSIALDK-K([17-carboxy-heptadécanoyl]-isoGlu-Peg3-Peg3)-AQRAFVEWLLAQ ; ou
Y-Aib-EGTFTSDYSIALDK-K((19-carboxy-nonadécanoyl]-[(pipérazine-1-yl)-acétyl]-Peg3-Peg3)-AQRAFVEWLLAQ ; ou
(b) le squelette peptidique (Formule I) possède la séquence :
Y-Aib-EGTFTSDYSIYLDK-K([17-carboxy-heptadécanoyl]-isoGlu-Peg3-Peg3)-AQRAFVEWLLAQGPSSGAPPPS ;
Y-Aib-EGTFTSDYSIYLDK-K([19-carboxy-nonadécanoyl]-isoGlu-Peg3-Peg3)-AQRAFVEWLLAQGPSSGAPPPS ;
Y-Aib-EGTFTSDYSIYLDK-K([17-carboxy-heptadécanoyl]-isoGlu)-AQRAFVEWLLAQGPSSGAPPPS ;
H-Y-Aib-EGTFTSDYSIYLDK-K([17-carboxy-heptadécanoyl]-isoGlu-GSGSGG)-AQRAFVEWLLAQGPSSGAPPPS ;
Y-Aib-EGTFTSDYSIYLDK-K([17-carboxy-heptadécanoyl]-Peg3-Peg3)-AQRAFVEWLLAQGPSSGAPPPS ;
Y-Aib-EGTFTSDYSIYLDK-K([17-carboxy-heptadécanoyl]-isoGlu-Peg3-Peg3-Peg3)-AQRAFVEWLLAQGPSSGAPPPS ;
Y-Aib-EGTFTSDYSIYLDK-K([17-carboxy-heptadécanoyl]-Dapa-Peg3-Peg3)-AQRAFVEWLLAQGPSSGAPPPS ;
Y-Aib-EGTFTSDYSIYLDK-K([19-carboxy-nonadécanoyl]-isoGlu-Peg3-Peg3-Peg3)-AQRAFVEWLLAQGPSSGAPPPS ;
Y-DAla-EGTFTSDYSIYLDK-K([17-carboxy-heptadécanoyl]-isoGlu-Peg3-Peg3)-AQRAFVEWLLAQGPSSGAPPPS ;
Y-Aib-EGTFTSDYSI-Aib-LDK-K([17-carboxy-heptadécanoyl]-isoGlu-Peg3-Peg3)-AQRAFVEWLLAQGPSSGAPPPS ;
Y-Aib-EGTFTSDYSIALEK-K([17-carboxy-heptadécanoyl]-isoGlu-Peg3-Peg3)-AQRAFVEWLLAQK ;
Y-Aib-EGTFTSDLSIALEK-K([17-carboxy-heptadécanoyl]-isoGlu-Peg3-Peg3)-AQRAFVEWLLAQK ;
Y-Aib-EGTFTSDYSIALEK-K([19-carboxy-nonadécanoyl]-isoGlu-Peg3-Peg3)-AQRAFVEWLLAQK ;
Y-Aib-EGTFTSDLSIALEK-K([19-carboxy-nonadécanoyl]-isoGlu-Peg3-Peg3)-AQRAFVEWLLAQK ;
Y-DAla-EGTFTSDYSIYLDK-K([19-carboxy-nonadécanoyl]-isoGlu-Peg3-Peg3)-AQRAFVEWLLAQGPSSGAPPPS ;
Y-Aib-EGTFTSDYSIALEK-K([19-carboxy-nonadécanoyl]-isoGlu-Peg3-Peg3)-AQRAFVEWLLAQGPSSGAPPPS ;
Y-DAla-EGTFTSDYSIALEK-K([19-carboxy-nonadécanoyl]-isoGlu-Peg3-Peg3)-AQRAFVEWLLAQGPSSGAPPPS ;
Y-Aib-EGTFTSDLSIALEK-K([19-carboxy-nonadécanoyl]-isoGlu-Peg3-Peg3)-AQRAFVEWLLAQKPSSGAPPPS ;
Y-Aib-EGTFTSDYSIYLDK-K([19-carboxy-nonadécanoyl]-Peg3-Peg3)-AQRAFVEWLLAQGPSSGAPPPS ;
Y-Aib-EGTFTSDYSIYLDK-K([19-carboxy-nonadécanoyl]-isoGlu)-AQRAFVEWLLAQGPSSGAPPPS ;
Y-Aib-EGTFTSDYSIYLDK-K([19-carboxy-nonadécanoyl]-Dapa-Peg3-Peg3)-AQRAFVEWLLAQGPSSGAPPPS ;
Y-Aib-EGTFTSDLSIALEK-K([19-carboxy-nonadécanoyl]-isoGlu-Peg3-Peg3)-AQRAFVEWLLAQGPSSGAPPPS ;
Y-Aib-EGTFTSDYSIALDK-K([19-carboxy-nonadécanoyl]-isoGlu-Peg3-Peg3)-AQRAFVEWLLAQKPSSGAPPPS ;
Y-DAla-EGTFTSDYSIALDK-K([19-carboxy-nonadécanoyl]-isoGlu-Peg3-Peg3)-AQRAFVEWLLAQKPSSGAPPPS ;
Y-Aib-EGTFTSDYSIALDK-K([19-carboxy-nonadécanoyl]-isoGlu-Peg3-Peg3)-AQRAFVEWLLAQGPSSGAPPPS ;
Y-Aib-EGTFTSDYSIALDE-K([19-carboxy-nonadécanoyl]-isoGlu-Peg3-Peg3)-AQRAFVEWLLAQGPSSGAPPPS ;
Y-Aib-EGTFTSDYSIALDK-K([19-carboxy-nonadécanoyl]-isoGlu-Peg3-Peg3)-AQRAFIEWLLAQGPSSGAPPPS ;
Y-Aib-EGTFTSDYSIALDK-K([17-carboxy-heptadécanoyl]-isoGlu-Peg3-Peg3)-AQRAFVEWLLAQGPSSGAPPPS ; ou
Y-Aib-EGTFTSDYSIALDK-K((19-carboxy-nonadécanoyl]-[(pipérazine-1-yl)-acétyl]-Peg3-Peg3)-AQRAFVEWLLAQGPSSGAPPPS.

11. Composition pharmaceutique comprenant un analogue du GIP selon l'une quelconque des revendications 1 à 10, ou un sel ou solvate de celui-ci pharmaceutiquement acceptable, dans un mélange avec un véhicule, par exemple où ladite composition est formulée sous forme d'un liquide approprié pour une administration par injection ou perfusion, ou formulée pour entraîner une libération lente dudit analogue du GIP.

12. Analogue du GIP selon l'une quelconque des revendications 1 à 10, ou sel ou solvate de celui-ci pharmaceutiquement acceptable, destiné à être utilisé dans un procédé de traitement médical.

13. Analogue du GIP selon l'une quelconque des revendications 1 à 10, ou sel ou solvate de celui-ci pharmaceutiquement acceptable, destiné à être utilisé dans un procédé de traitement et/ou de prévention du diabète ou d'un trouble lié au diabète, ou de l'obésité ou d'un trouble lié à l'obésité.

14. Analogue du GIP destiné à être utilisé selon la revendication 13, dans lequel :
(a) le trouble lié au diabète est l'insulinorésistance, l'intolérance au glucose, l'hyperglycémie à jeun, l'hypoglycémie (par exemple induite par un traitement par l'insuline), le pré-diabète, le diabète de type 1, le diabète de type 2, l'hypertension liée au diabète gestationnel, la dyslipidémie, un trouble osseux tel que l'ostéoporose, ou une combinaison de ceux-ci ;
(b) le trouble lié au diabète est l'athérosclérose, l'artériosclérose, une coronaropathie, une maladie artérielle périphérique, un accident vasculaire cérébral ; ou est une affection associée à la dyslipidémie athérogène, un trouble des lipides sanguins (tel qu'un taux de triglycérides élevé, un faible taux de cholestérol HDL, un taux de cholestérol LDL élevé, une accumulation de plaque dans les parois artérielles, ou une combinaison de ceux-ci), l'hypertension artérielle, l'hypertension, un état prothrombotique, ou un état pro-inflammatoire ; ou
(c) le trouble lié à l'obésité est une inflammation liée à l'obésité, une maladie de la vésicule biliaire liée à l'obésité ou l'apnée du sommeil induite par l'obésité, ou peut être associé à une affection sélectionnée parmi la dyslipidémie athérogène, des troubles des lipides sanguins, l'hypertension artérielle, l'hypertension, un état prothrombotique, et un état pro-inflammatoire, ou une combinaison de ceux-ci.
